# EUROPEAN PATENT APPLICATION

(11) **EP 4 653 466 A1**
(43) Date of publication of application: **26.11.2025**
(21) Application number: 24744290.8
(22) Date of filing: 17.01.2024
(51) Int. Cl.: C07K 16/46, C12N 15/13, A61K 39/395, A61P 35/00

(54) **ANTI-ALK-1 ANTIBODY AND USE THEREOF**

(30) Priority: 18.01.2023 CN 202310083522; 25.09.2023 CN 202311249366
(71) Applicant: KINTOR PHARMACEUTICAL (GUANGDONG) CO., LTD, Zhuhai, Guangdong 519040 (CN)
(72) Inventor: TONG, Youzhi, Zhuhai, Guangdong 519040 (CN); ZHOU, Qingqing, Zhuhai, Guangdong 519040 (CN); ZHUANG, Lanfang, Zhuhai, Guangdong 519040 (CN); YANG, Jianfei, Zhuhai, Guangdong 519040 (CN)
(74) Representative: Hoffmann Eitle
(86) International application number: PCT/CN2024/072728
(87) International publication number: WO 2024/153115

(57) **Abstract**

The present invention provides an anti-ALK-1 monoclonal antibody having high affinity and excellent druggability, further provides an anti-ALK-1 and anti-VEGF bispecific antibody and an anti-ALK-1 and anti-PD-1 bispecific antibody on the basis of the anti-ALK-1 monoclonal antibody, and further provides a pharmaceutical composition containing the antibody, and a use thereof in the preparation of a drug for inhibiting angiogenesis and/or treating tumors.

## Description

The present application claims priority of Chinese patent application CN 202310083522.1 filed on January 18, 2023 and Chinese patent application CN202311249366.8 filed on September 25, 2023. All of the specification, drawings and claims of the priority documents are incorporated into the specification of the present application and are regarded as part of the original description of the specification of the present application. The applicant further declares that the applicant has the right to amend the specification and claims of the present application based on the priority document.

### Technical field

The present application relates to a monoclonal antibody that specifically binds ALK-1, a bispecific antibody that simultaneously binds ALK-1 and VEGF, a bispecific antibody that specifically binds to ALK-1 and PD-1, and a pharmaceutical composition of antibodies, and the uses thereof.

### Background

ALK-1 is a type I cell surface receptor for transforming growth factor beta receptor type 1 (TGF-β-1). Human ALK-1 is a 503 amino acid polypeptide, which includes a signal sequence (amino acids: 1-21), a N-terminal extracellular TGF-β-1 ligand binding domain or ECD (amino acids: 22-118), and a single transmembrane domain (amino acids: 119-141), a regulatory glycine/serine-rich (GS) domain (amino acids: 142-202), and a C-terminal serine-threonine kinase domain (amino acids: 202- 492). Although ALK-1 shares 60-80% overall homology with other type I receptors (ALK-2 to ALK-7), the ECD of ALK-1 is significantly different from that of other ALK family members. For example, in humans, only the ECD of ALK-2 is significantly related to that of ALK-1 (sharing approximately 25% amino acid identity).

Typically, TGF-β superfamily ligands exert their biological activities by binding to heteromeric receptor complexes of two types (I and II) of serine/threonine kinases. Type II receptors are constitutively active kinases that Phosphorylate type I receptors upon ligand binding. In turn, activated type I kinases phosphorylate downstream signaling molecules, including various Smads, which translocate to the nucleus and lead to transcriptional responses. (Heldin et al. Nature, 1997, vol. 390, pp. 465-471) Under the expression of ALK-1, it has been demonstrated that Smad1 is specifically phosphorylated and translocate to the nucleus where it directly regulates the expression of the Smad1 responsive genes Id1 and EphB2.

ALK-1 is expressed highly and selectively in endothelial cells and other highly vascularized tissues such as the placenta or brain. The expression of ALK-1 in endothelial cells is significantly higher than that of other types of receptors (ALK-5) and endoglin. Mutations in ALK-1 are associated with hereditary hemorrhagic telangiectasia (HHT), suggesting that ALK-1 plays a key role in controlling blood vessel development or repair (Abdalla et al. J. Med. Genet., 2003, vol. 40, pp. 494-502; Sadick et al. Hematologica/The Hematology J., 2005, vol. 90, 818-828.). In addition, two independent studies of ALK-1 knockout mice provide critical in vivo evidence for the function of ALK-1 during angiogenesis (Oh et al. Proc Natl Acad Sci USA, 2000, vol. 97, pp. 2626-2631; Urness et al. Nature Genetics, 2000, vol. 26, pp. 328-331.).

Anti-angiogenic therapies are expected to be chronic in nature. Therefore, targets with highly selective endothelial function, such as ALK-1, are preferred to reduce attrition caused by side effects. Furthermore, given that ALK-1 ECD differs significantly from the ECD of other ALK family members, mAbs against human ALK-1 ECD are expected to selectively target ALK-1. Based on these considerations, there is a strong need for a monoclonal antibody directed against the ALK-1 extracellular domain that can inhibit dimerization with the type II receptor, thereby preventing Smad1 phosphorylation and downstream transcriptional reactions.

The development of the vascular network is a multi-step process in which vascular endothelial growth factor (VEGF) plays a key role. VEGF is an initiating factor for angiogenesis and co-exists with fibroblast growth factor (FGF) to stimulate endothelial cell proliferation and migration. Subsequently, VEGF and Notch pathway proteins signal to initiate and support endothelial blood vessel sprouting. Then remodeling of the basement membrane by extracellular matrix proteases then continues to support vessel sprouting and branching. The maturation and stabilization of early branch vessels and the subsequent formation of functional vascular beds is a complex process that includes multiple factors such as angiopoietin, platelet-derived growth factors, sphingosine phosphate receptors, and the ALK1/ENG pathway. Much of the known information about vascular maturation comes from genetic studies and some in vivo angiogenesis models. The exact functions of these molecules in tumors have not been fully understood due to the lack of adequate models of tumor angiogenesis. In vitro studies using cultured endothelial cell lines and similar in vivo studies using the chicken chorioallantoic membrane (CAM) assay often fail to recapitulate tumor-host interactions. Therefore, optimal sustained inhibition of tumor angiogenesis may require coordinated inhibition of multiple components of the angiogenic program.

Cancer is a leading cause of death and is characterized by the uncontrolled growth and spread of abnormal cells. Many types of tumors rely on the growth of new blood vessels (angiogenesis) to provide adequate nutrients and oxygen, allowing cancer cells to grow, invade nearby tissue, and spread to other parts of the body. Angiogenesis inhibition is a widely used cancer treatment. Several angiogenesis inhibitors that act by blocking the vascular endothelial growth factor (VEGF) pathway are approved or in development. These therapies, given alone or in combination with chemotherapy and/or radiation therapy, can significantly improve survival rates.

Studies in endothelial cells and mice have shown that down-regulation of ALK1 expression blocks the migration ability of endothelial cells, while blocking VEGF-A can reduce the proliferation ability of endothelial cells. ALK1 and VEGF have different mechanisms of action in angiogenesis, but they can synergistically promote the migration and proliferation of endothelial cells. Existing VEGF/VEGFR blocking drugs will develop a certain degree of drug resistance after being used for a period of time. Therefore, the combination of target drugs with different anti-angiogenesis mechanisms is a new direction of development.

Although VEGF inhibitors play an important role in the treatment of some tumors; for some tumors, there is no response at all or only a temporary response to treatment with a VEGF inhibitor. Therefore, there is a need for additional compositions and methods to inhibit angiogenesis in the context of cancer treatment.

Bispecific antibodies (BsAb) are artificial antibodies prepared through cell fusion or recombinant DNA technology. They can specifically bind to two distinct antigens or two different epitopes of the same antigen. BsAbs can work through bridging cells, bridging receptors, bridging factors, etc. Compared with monoclonal antibodies or combinations, bispecific antibodies are more effective and safer, and have differentiated advantages. BsAbs can recognize and bind to two different antigenic epitopes, thereby bridging tumor cells and effector cells, mediating their effect on tumors, targeted killing; or bridging two different receptors, which may activate new biological signals. Compared with monoclonal antibody therapy alone, BsAbs can block multiple signaling pathways at the same time, preventing drug resistance and improving efficacy; and in contrast to monoclonal antibody combination therapies, BsAbs have greater specificity and targeting and safer. By bridging two different receptors that control the signaling pathways of new blood vessel proliferation in the tumor microenvironment, ALK1/VEGF BsAb can simultaneously inhibit two different signaling pathways for blood vessel proliferation, thereby strengthening the inhibition of the proliferation of new blood vessels in the tumor microenvironment to achieve the effect of inhibiting tumor growth. Compared with a co-formulation of ALK1 and VEGF-targeted antibodies, the present application confirms lower dose, better targeting and higher drug-related safety of ALK1/VEGF BsAb.

As the understanding of targeting checkpoints including the immune checkpoint programmed death 1 (PD-1), its ligand PD-L1 and cytotoxic T lymphocyte-associated antigen (CTLA-4) used in the field of tumor treatment has grown, their clinical studies in hepatocellular carcinoma have also been widely carried out, and some of them have achieve good outcomes. In September 2017, the U.S. Food and Drug Administration approved Nivolumab for the treatment of patients with hepatocellular carcinoma who have failed by sorafenib (Nivolumab (Opdivo^{®}) Injection Instructions). Nivolumab can block PD-1 of T lymphocytes from binding to PD-L1 on the surface of tumor cells, thereby alleviating the immunosuppression by tumor cells and enable immune cells to re-exercise their anti-tumor cellular immunity and kill tumor cells. However, the effective rate of using PD-1 inhibitor alone is only about 20% in the vast majority of unselected solid tumors. Patients with long-term use will develop drug resistance and have too many side effects. In recent years, drug combination has become a trend in tumor treatment. It can not only enrich the diversity of anti-tumor treatments, achieve short-term and efficient control of tumor growth and alleviate the course of the disease, but also achieve the purpose of using a combination of existing drugs to cure the disease and ensure prognosis. Therefore, in order to improve the tumor inhibition rate and reduce side effects, more and more anti-tumor drugs are combined with PD-1/PD-L1 drugs to treat various advanced solid tumors.

Nivolumab blocks the combination of PD-1 and PD-L1, thereby reducing the immune evasion of tumors and improving the immune attack of T lymphocytes on tumor cells. Therefore, the development of bispecific antibodies simultaneously targeting both ALK-1 and PD-1 opens up valuable prospects in improving anti-tumor efficacy and prolonging the overall survival and safety of patients.

### Summary

The present application provides an anti-ALK-1 monoclonal antibody and an anti-ALK-1-anti-VEGF BsAb with high affinity and excellent pharmaceutical properties. Compared with the ALK-1 monoclonal antibody or the VEGF monoclonal antibody, the BsAb antibody has higher binding affinity to CHO-K1-hALK1 cells, stronger blocking effect on the formation of microtubules in HUVEC cells, more significant inhibitory effect on tumor growth, and good safety.
According to the first aspect of the present application, the present application provides a monoclonal antibody or antigen-binding portion thereof that binds to ALK-1, wherein the antibody or an antigen-binding portion thereof has a heavy chain and a light chain;
the CDR1H, CDR2H and CDR3H of the heavy chain variable domain VH of the antibody or antigen-binding portion thereof are CDR1H, CDR2H and CDR3H of the heavy chain variable domain of SEQ ID NO: 1;or the CDR1H, CDR2H and CDR3H of the heavy chain variable domain VH of the antibody or antigen-binding part thereof are CDR1H, CDR2H and CDR3H of the sequence which is obtained by adding, deleting or replacing one or more amino acids in SEQ ID NO: 1 and has the same function as SEQ ID NO: 1;
the CDR1L, CDR2L and CDR3L of the light chain variable domain VL of the antibody or antigen-binding portion thereof are obtained by amino acid mutation based on the CDR1L, CDR2L and CDR3L of the light chain variable domain of SEQ ID NO: 5, the mutation refers to mutation of amino acids at position 91 and position 95 of SEQ ID NO: 5. Preferably, the amino acids No. 91 and No. 95 are not a combination of tryptophan W and valine V, proline P and tryptophan W, leucine L and phenylalanine F.

In some embodiments of the present application, the CDR1L, CDR2L and CDR3L of the light chain variable domain VL of the antibody or antigen-binding portion thereof are CDR1L, CDR2L and CDR3L of the light chain variable domain of SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53 or SEQ ID NO: 55. Preferably, the CDR1L, CDR2L and CDR3L of the light chain variable domain VL of the antibody or antigen-binding portion thereof are CDR1L, CDR2L and CDR3L of the light chain variable domain of SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51 and SEQ ID NO: 55; more preferably, the CDR1L, CDR2L and CDR3L of the light chain variable domain VL of the antibody or antigen-binding portion thereof are CDR1L, CDR2L and CDR3L of the light chain variable domain of SEQ ID NO: 11, SEQ ID NO: 13, SEQ ID NO: 15, SEQ ID NO: 25, SEQ ID NO: 31 or SEQ ID NO: 51; most preferably, the CDR1L, CDR2L and CDR3L of the light chain variable domain VL of the antibody or antigen-binding portion thereof are the CDR1L, CDR2L and CDR3L of the light chain variable domain of SEQ ID NO: 11.

In some embodiments of the present application, the monoclonal antibody or antigen-binding portion thereof that binds to ALK-1, wherein the antibody or antigen-binding portion thereof has a heavy chain and a light chain, as defined according to the IMGT antibody numbering scheme for CDR regions, the light chain CDR is selected from:
(a) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 12; or
(b) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 14; or
(c) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 16; or
(d) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 18; or
(e) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 20; or
(f) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 22; or
(g) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:24; or
(h) the light chain complementarity determining region 1 (CDR1L) described in the amino acid sequence of SEQ ID: 6, the light chain complementarity determining region 2(CDR2L) described in the amino acid sequence of SEQ ID: 7, and the light chain complementarity determining region 3 (CDR3L) described in the amino acid sequence of SEQ ID: 26; or
(i) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:28; or
(j) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 30; or
(k) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 32; or
(l) the light chain complementarity determining region 1 (CDR1L) described in the amino acid sequence of SEQ ID: 6,
   the light chain complementarity determining region (CDR2L) described in the amino acid sequence of SEQ ID: 7, and the light chain complementarity determining region 3 (CDR3L) described in the amino acid sequence of SEQ ID: 34; or
(m) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 36; or
(n) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 38; or
(o) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 40; or
(p) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 42; or
(q) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 44; or
(r) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 46; or
(s) the light chain complementarity determining region 1 (CDR1L) described in the amino acid sequence of SEQ ID: 6, the light chain complementarity determining 2 (CDR2L) described in the amino acid sequence of SEQ ID: 7, and the light chain complementarity determining region 3 (CDR3L) described in the amino acid of SEQ ID: 48; or
(t) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 50; or
(u) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 52; or
(v) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 54; or
(w) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 56;

the heavy chain includes the heavy chain complementarity determining region 1 (CDR1H) described in the amino acid sequence of SEQ ID: 2, the heavy chain complementarity determining region 2 (CDR2H) described in the amino acid sequence of SEQ ID: 3, and the heavy chain complementarity determining region 3 (CDR3H) described in the amino acid sequence of SEQ ID: 4.

Preferably, the light chain CDR region is selected from the CDR of (a)-(k), (m)-(u) or (w) above; further preferably, selected from the CDR of (a)-(c), (h), (k) or (u); most preferably selected from the CDR in (a).

In some embodiments of the present application, the CDR1H, CDR2H and CDR3H of the heavy chain variable domain VH of the antibody or antigen-binding portion thereof are the CDR1H, CDR2H and CDR3H of the heavy chain variable domain of SEQ ID NO: 1; the CDR1L, CDR2L and CDR3L of the light chain variable domain VL of the antibody or antigen-binding portion thereof are the CDR1L, CDR2L and CDR3L of the light chain variable domains of SEQ ID NO: 11, SEQ ID NO: 13, SEQ ID NO: 15, SEQ ID NO: 25, SEQ ID NO: 31 or SEQ ID NO: 51; most preferably, the CDR1L, CDR2L and CDR3L of the light chain variable domain VL of the antibody or antigen-binding portion thereof are the CDR1L, CDR2L and CDR3L of the light chain variable domains of SEQ ID NO: 11.

In some embodiments of the present application, the heavy chain variable domain VH of the antibody or antigen-binding portion thereof comprises the amino acid sequence of SEQ ID NO: 1, SEQ ID NO: 67, SEQ ID NO: 70, SEQ ID NO: 73, SEQ ID NO: 75, SEQ ID NO: 78, SEQ ID NO: 80 or SEQ ID NO: 81; the light chain variable domain VL of the antibody or antigen-binding portion thereof comprises the amino acid sequence of SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45. SEQ ID NO: 47, SEQ ID NO: 49, SEQ ID NO: 51, SEQ ID NO: 53, SEQ ID NO: 55, SEQ ID NO: 66, SEQ ID NO: 71, SEQ ID NO: 72, SEQ ID NO:74, SEQ ID NO:76, SEQ ID NO:77 or SEQ ID NO:79. Preferably, the light chain variable domain VL of the antibody or antigen-binding portion thereof comprises the amino acid sequence of SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 55, SEQ ID NO: 66, SEQ ID NO: 71, SEQ ID NO: 72, SEQ ID NO: 74, SEQ ID NO: 76, SEQ ID NO: 77 or SEQ ID NO: 79; more preferably, the light chain variable domain VL of the antibody or antigen-binding portion thereof comprises the amino acid sequence of SEQ ID NO: 11, SEQ ID NO: 13, SEQ ID NO: 15, SEQ ID NO: 25, SEQ ID NO: 31, SEQ ID NO: 51, SEQ ID NO: 72, SEQ ID NO: 79 or SEQ ID NO: 76; most preferably, the light chain variable domain VL of the antibody or antigen-binding portion thereof comprises the amino acid sequence of SEQ ID NO: 11.

In some embodiments of the present application, the heavy chain variable domain VH of the antibody or antigen-binding portion thereof comprises the amino acid sequence of SEQ ID NO: 1; the light chain variable domain of the antibody or antigen-binding portion thereof comprises the amino acid sequence of SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO:43, SEQ ID NO:45, SEQ ID NO:47, SEQ ID NO:49, SEQ ID NO:51, SEQ ID NO:53 or SEQ ID NO:55. Preferably, the light chain variable domain VL of the antibody or antigen-binding portion thereof comprises the amino acid sequence of SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51,or SEQ ID NO: 55; more preferably, the light chain variable domain VL of the antibody or antigen-binding fragment thereof comprises the amino acid sequence of SEQ ID NO: 11, SEQ ID NO: 13, SEQ ID NO: 15, SEQ ID NO: 25, SEQ ID NO: 31 or SEQ ID NO: 51; most preferably, the light chain variable domain VL of the antibody or antigen-binding portion thereof comprises the amino acid sequence of SEQ ID NO: 11.

In some embodiments of the present application, the antibody or antigen-binding fragment thereof binds to the extracellular domain of human ALK-1.

In some embodiments of the present application, the antibody or antigen-binding fragment is a human antibody or antigen-binding fragment.

In some embodiments of the present application, the antigen-binding fragment is a Fab fragment, an F(ab')₂ fragment or a single-chain antibody.

In some embodiments of the present application, the antibody or antigen-binding fragment is IgG, IgM, IgE, IgA or IgD, further preferably the antibody or antigen-binding fragment is IgG1, IgG2, IgG3 or IgG4, more preferably the antibody or antigen-binding fragment is human IgG1-LALA.

According to a second aspect of the present application, it provides a BsAb, which includes a first antigen-binding region (ALK-1 binding region) that specifically binds to ALK-1 and a second antigen-binding region that specifically binds to VEGF(VEGF binding region). The first antigen-binding region that specifically binds to ALK-1 includes a heavy chain variable region (VH) and a light chain variable region (VL), and the second antigen-binding region that specifically binds to VEGF includes a heavy chain variable region (VH) and a light chain variable region (VL) or a VEGF receptor fragment that specifically binds to VEGF;
the CDR1H, CDR2H and CDR3H of the heavy chain variable domain VH of the first antigen binding region are CDR1H, CDR2H and CDR3H of the heavy chain variable domain of SEQ ID NO: 1; or the CDR1H, CDR2H and CDR3H of the heavy chain variable domain VH of the first antigen binding region are CDR1H, CDR2H and CDR3H of the sequence which is obtained by adding, deleting or replacing one or more amino acids in SEQ ID NO: 1 and has the same function as SEQ ID NO: 1.

The CDR1L, CDR2L and CDR3L of the light chain variable domain VL of the first antigen binding region are obtained by amino acid mutation based on the CDR1L, CDR2L and CDR3L of the light chain variable domain of SEQ ID NO: 5; the mutation refers to the mutation of amino acids at position 91 and position 95 of SEQ ID NO: 5. Preferably, the amino acids No. 91 and No. 95 are not a combination of tryptophan W and valine V, proline P and tryptophan W, leucine L and phenylalanine F.

In some embodiments of the present application, the CDR1L, CDR2L and CDR3L of the light chain variable domain VL of the first antigen binding region are CDR1L, CDR2L and CDR3L of the light chain variable domain of SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO : 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49 , SEQ ID NO: 51, SEQ ID NO: 53 or SEQ ID NO: 55. Preferably, the CDR1L, CDR2L and CDR3L of the light chain variable domain VL of the first antigen binding region are CDR1L, CDR2L and CDR3L of the light chain variable domain of SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 35, SEQ ID NO : 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 55; more preferably, the CDR1L, CDR2L and CDR3L of the light chain variable domain VL of the first antigen binding region are CDR1L, CDR2L and CDR3L of the light chain variable domain of SEQ ID NO: 11, SEQ ID NO: 13, SEQ ID NO: 15, SEQ ID NO: 25, SEQ ID NO: 31 or SEQ ID NO: 51; most preferably, the first CDR1L, CDR2L and CDR3L of the light chain variable domain VL of an antigen-binding region are CDR1L, CDR2L and CDR3L of the light chain variable domain of SEQ ID NO: 11.

In some embodiments of the present application, the CDR1H, CDR2H and CDR3H of the heavy chain variable domain VH of the first antigen binding region are the CDR1H, CDR2H and CDR3H of the heavy chain variable domain of SEQ ID NO: 1.

In some embodiments of the present application, the CDR region is defined according to the IMGT antibody numbering scheme, and the light chain CDR of the first antigen-binding region is selected from the following combination:
(a) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 12; or
(b) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 14; or
(c) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 16; or
(d) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 18; or
(e) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 20; or
(f) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 22; or
(g) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 24; or
(h) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 26; or
(i) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 28; or
(j) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 30; or
(k) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 32; or
(l) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 34; or
(m) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 36; or
(n) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 38; or
(o) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 40; or
(p) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 42; or
(q) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 44; or
(r) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 46; or
(s) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 48; or
(t) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 50; or
(u) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 52; or
(v) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 54; or
(w) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 56;
the heavy chain variable domain of the first antigen binding region includes the heavy chain complementarity determining region 1 (CDR1H) described in the amino acid sequence of SEQ ID: 2, and the heavy chain complementarity determining region 2 (CDR2H) described in the amino acid sequence of SEQ ID: 3, the heavy chain complementarity determining region 3 (CDR3H) described in the amino acid sequence of SEQ ID: 4.

Preferably, the light chain CDR region is selected from the CDR in (a)-(k), (m)-(u) or (w) described above; further preferably selected from CDR in (a)-(c), (h), (k) or (u), most preferably selected from in (a).

In some embodiments of the present application, the heavy chain variable region of the first antigen binding region comprises the amino acid sequence of SEQ ID NO: 1, SEQ ID NO: 67, SEQ ID NO: 70, SEQ ID NO: 73, SEQ ID NO: 75, SEQ ID NO: 78, SEQ ID NO: 80 or SEQ ID NO: 81; or comprises the heavy chain variable region of the first antigen binding region which is obtained by adding, deleting or replacing one or more amino acids in SEQ ID NO: 1 and has the same function as SEQ ID NO: 1, SEQ ID NO: 67 or SEQ ID NO: 81. Preferably, the heavy chain variable region of the first antigen-binding region comprises the amino acid sequence of SEQ ID NO: 1, SEQ ID NO: 67 or SEQ ID NO: 81.

In some embodiments of the present application, the heavy chain variable region of the first antigen-binding region includes the amino acid sequence of SEQ ID NO: 1; or comprises the heavy chain variable regions of the first antigen-binding regions which is obtained by adding, deleting or replacing one or more amino acids in SEQ ID NO: 1, and has the same function as SEQ ID NO: 1.

In some embodiments of the present application, the light chain variable region of the first antigen binding region comprises the amino acid sequence selected from those shown in SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO : 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51 , SEQ ID NO: 53, SEQ ID NO: 55, SEQ ID NO: 66, SEQ ID NO: 71, SEQ ID NO: 72, SEQ ID NO: 74, SEQ ID NO: 76, SEQ ID NO: 77 or SEQ ID NO:79. Preferably, the light chain variable domain VL of the first antigen binding region comprises the amino acid sequence selected from those shown in SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 55, SEQ ID NO: 66, SEQ ID NO: 71, SEQ ID NO: 72, SEQ ID NO: 74, SEQ ID NO: 76, SEQ ID NO: 77 or SEQ ID NO: 79; more preferably, the light chain variable domain VL of the first antigen binding region comprises the amino acid sequence selected from those shown in SEQ ID NO: 11, SEQ ID NO: 13, SEQ ID NO : 15, SEQ ID NO: 25, SEQ ID NO: 31, SEQ ID NO: 51, SEQ ID NO: 66, SEQ ID NO: 72, SEQ ID NO: 79 , or SEQ ID NO: 76; most preferably, the light chain variable domain VL of the first antigen binding region is the amino acid sequence shown in SEQ ID NO: 11, SEQ ID NO: 66, SEQ ID NO: 72, SEQ ID NO: 79 or SEQ ID NO: 76.

In some embodiments of the present application, the light chain variable region of the first antigen binding region comprises the amino acid sequence selected from those shown in SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO : 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51 , SEQ ID NO: 53 or SEQ ID NO: 55. Preferably, the light chain variable domain VL of the first antigen binding region comprises the amino acid sequence selected from those shown in SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 55; more preferably, the light chain variable domain VL of the first antigen-binding region comprises the amino acid sequence selected from those shown in SEQ ID NO: 11, SEQ ID NO: 13, SEQ ID NO: 15, SEQ ID NO: 25, SEQ ID NO: 31 or SEQ ID NO: 51; most preferably, the light chain variable domain VL of the first antigen binding region is the amino acid sequence shown in SEQ ID NO: 11.

In some embodiments of the present application, the BsAb comprises a heavy chain constant region of an IgG, preferably comprises a heavy chain constant region of an IgG1, IgG4 or IgG2, more preferably comprises a heavy chain constant region of an IgG1, most preferably, comprises a heavy chain constant region of SEQ ID NO:9.

In some embodiments of the present application, the second antigen-binding region that specifically binds VEGF includes a heavy chain variable region and a light chain variable region, and CDR1H, CDR2H and CDR3H of the heavy chain variable region VH are the CDR1H, CDR2H and CDR3H of the heavy chain variable domain of SEQ ID NO: 57, or the CDR1H, CDR2H and CDR3H of the heavy chain variable region VH are the CDR1H, CDR2H and CDR3H of the sequence which is obtained by adding, deleting or replacing one or more amino acids in SEQ ID NO: 57 and has the same function as SEQ ID NO: 57. The CDR1L, CDR2L and CDR3L of the light chain variable region VL are the CDR1L, CDR2L and CDR3L of the light chain variable domain of SEQ ID NO: 61, or the CDR1L, CDR2L and CDR3L of the heavy chain variable region VL are the CDR1L, CDR2L and CDR3L of sequence which is obtained by adding, deleting or replacing one or more amino acids in SEQ ID NO: 61 and has the same function as SEQ ID NO: 61.

In some embodiments of the present application, the second antigen-binding region that specifically binds to VEGF includes a heavy chain variable region and a light chain variable region,
the heavy chain variable region includes CDR1H as shown in SEQ ID NO: 58, CDR2H as shown in SEQ ID NO: 59 and CDR3H as shown in SEQ ID NO: 60; and
the light chain variable region includes CDR1L as shown in SEQ ID NO:62, CDR2L as shown in SEQ ID NO:63 and CDR3L as shown in SEQ ID NO:64.

In some embodiments of the present application, the heavy chain variable region comprises the sequence of SEQ ID NO: 57 and the light chain variable region comprises the sequence of SEQ ID NO: 61.

In some embodiments of the present application, the second antigen-binding region that specifically binds to VEGF comprises a VEGF receptor fragment that specifically binds to VEGF, the VEGF receptor fragment comprises the extracellular domain 2 of VEGF receptor 1 and the extracellular domain 3 of VEGF receptor 2.

In some embodiments of the present application, the first antigen binding region or the second antigen binding region is in scFv form, preferably, the first antigen binding region is in scFv form.

In some embodiments of the present application, the first antigen-binding region and the second antigen-binding region are connected through a linker,
preferably, the linker includes (G4S)n, n is an integer greater than 1;
more preferably, the linker is composed of (G4S)n, n is an integer from 2 to 10;
most preferably, the linker is composed of (G4S)n, and n is 2, 3 or 4; for example, the Linker is GGGGSGGGGSGGGGSGGGGS (SEQ ID NO: 65).

In some embodiments of the present application, the scFv includes a heavy chain variable region and a light chain variable region, and the heavy chain variable region and the light chain variable region are connected by a linker,
preferably, the linker includes (G4S)n, n is an integer greater than 1;
more preferably, the linker is composed of (G4S)n, n is an integer from 2 to 10;
most preferably, the linker consists of (G4S)n, where n is 2, 3 or 4.

In some embodiments of the present application, the BsAb consists of 4 chains, including two identical first chains and two identical second chains,
the first chain sequentially includes the VH of the VEGF binding region, the CH of the VEGF binding region, the Linker, the VH of the ALK-1 binding region, the Linker and the VL of the ALK-1 binding region from the N terminal to the C terminal, and
the second chain sequentially includes VL and CL of the VEGF binding region from N-terminal to C-terminal.

In some embodiments of the present application, the specific sequence contained in the bispecific antibody is as follows:
the VH sequence of the VEGF binding region is shown in SEQ ID NO: 57,
the CH sequence of the VEGF binding region is shown in SEQ ID NO: 9,
the VL sequence of the VEGF binding region is shown in SEQ ID NO: 61,
the CL sequence of the VEGF binding region is shown in SEQ ID NO: 10,
the Linker sequence is shown in SEQ ID NO: 65;
the VL sequence of the ALK-1 binding region is shown in SEQ ID NO: 66, SEQ ID NO: 71, SEQ ID NO: 72, SEQ ID NO: 74, SEQ ID NO: 76, SEQ ID NO: 77 or SEQ ID NO :79, and
the VH sequence of the ALK-1 binding region is shown in SEQ ID NO: 67, SEQ ID NO: 1, SEQ ID NO: 70, SEQ ID NO: 73, SEQ ID NO: 75, SEQ ID NO: 78, SEQ ID NO :80 or SEQ ID NO:81.

Preferably, the sequence of the first chain is shown as SEQ ID NO: 68 or SEQ ID NO: 82; the sequence of the second strand is shown in SEQ ID NO: 69.

In some embodiments of the present application, the specific sequence of the BsAb is as follows:
the VH sequence of the VEGF binding region is shown in SEQ ID NO: 57,
the CH sequence of the VEGF binding region is shown in SEQ ID NO: 9,
the VL sequence of the VEGF binding region is shown in SEQ ID NO: 61,
the CL sequence of the VEGF binding region is shown in SEQ ID NO: 10,
the Linker sequence is shown in SEQ ID NO: 65,
the VL sequence of the ALK-1 binding region is shown in SEQ ID NO: 66, and
the VH sequence of the ALK-1 binding region is shown in SEQ ID NO: 67.

Preferably, the sequence of the first strand is shown in SEQ ID NO: 68; the sequence of the second strand is shown in SEQ ID NO: 69.

In some embodiments of the present application, the BsAb consists of 4 chains, including 2 identical first chains and 2 identical second chains,
The first chain sequentially includes the VH of the ALK-1 binding region, the CH of the ALK-1 binding region, the Linker and the VEGF binding region in sequence from N-terminal to C-terminal, and
the second chain contains the VL and CL of the ALK-1 binding region in sequence from the N-terminal to the C-terminal.

In some embodiments of the present application, the BsAb consists of 4 peptide chains, 2 identical first chains and 2 identical second chains,
the first chain sequentially includes the VH and CH of the ALK-1 binding region from the N- terminal to the C- terminal, and
the second chain includes VEGF binding region, Linker, and the VL and CL of ALK-1 binding region in sequence from the N- terminal to the C- terminal.

In some embodiments of the present application, the BsAb is composed of 2 identical peptide chains, which sequentially include VEGF binding region, IgG1-Fc, Linker, the VH of ALK-1 binding region, linker, the VL of ALK -1 binding region in sequence from N-terminal to C-terminal. Herein, IgG1-Fc means the Fc region of IgG1, preferably means the Fc region of human IgG1.

In some embodiments of the present application, the BsAb consists of 4 peptide chains, 2 identical first chains and 2 identical second chains,
the first chain sequentially includes the VH of the ALK-1 binding region, the CH of the ALK-1 binding region, the Linker, the VH of the VEGF binding region, the Linker, the VL of the VEGF binding region, and
the second chain contains the VL and CL of the ALK-1 binding region in sequence from the N-terminus to the C-terminus.

According to a third aspect of the present application, the present application provides a BsAb, which comprises a first antigen-binding region (ALK-1 binding region) that specifically binds to ALK-1 and a second antigen-binding region that specifically binds to PD-1 (PD-1 binding region). The first antigen-binding region that specifically binds ALK-1 comprises a heavy chain variable region (VH) and a light chain variable region (VL), and the second antigen-binding region that specifically binds to PD-1 comprises the heavy chain variable region (VH) and the light chain variable region (VL);
the CDR1H, CDR2H and CDR3H of the heavy chain variable domain VH of the first antigen-binding region are CDR1H, CDR2H and CDR3H of the heavy chain variable domain of SEQ ID NO: 1; or the CDR1H, CDR2H and CDR3H of the heavy chain variable domain VH of the first antigen-binding region are the CDR1H, CDR2H and CDR3H of the sequence which is obtained by adding, deleting or replacing one or more amino acids in SEQ ID NO: 1 and has the same function as SEQ ID NO: 1;
the CDR1L, CDR2L and CDR3L of the light chain variable domain VL of the first antigen binding region are obtained by amino acid mutation based on the CDR1L, CDR2L and CDR3L of the light chain variable domain of SEQ ID NO: 5; the mutation refers to mutation of the amino acids at No. 91 and No. 95 of SEQ ID NO: 5; or CDR1L, CDR2L and CDR3L of the light chain variable domain VL of the first antigen binding region are CDR1L, CDR2L and CDR3L of the light chain variable domain of SEQ ID NO. NO:5. Preferably, the amino acids at No. 91 and No. 95 are combination of tryptophan W and valine V, proline P and tryptophan W, leucine L and phenylalanine F.

In some embodiments of the present application, the CDR1L, CDR2L and CDR3L of the light chain variable domain VL of the first antigen binding region are the CDR1L, CDR2L and CDR3L of the variable domain of SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO : 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47 , SEQ ID NO: 49, SEQ ID NO: 51, SEQ ID NO: 53 or SEQ ID NO: 55. Preferably, the CDR1L, CDR2L and CDR3L of the light chain variable domain VL of the first antigen binding region are the CDR1L, CDR2L and CDR3L of the variable domain of SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 35, SEQ ID NO : 37, SEQ ID NO: 39, SEQ ID NO: 41, SEQ ID NO: 43, SEQ ID NO: 45, SEQ ID NO: 47, SEQ ID NO: 49, SEQ ID NO: 51, or SEQ ID NO: 55; more preferably, the CDR1L, CDR2L and CDR3L of the light chain variable domain VL of the first antigen binding region are the CDR1L, CDR2L and CDR3L of the variable domain of SEQ ID NO: 11, SEQ ID NO: 13, SEQ ID NO: 15, SEQ ID NO: 25, SEQ ID NO: 31 or SEQ ID NO: 51; most preferably, the CDR1L, CDR2L and CDR3L of the light chain variable domain VL of the first antigen binding region are the CDR1L, CDR2L and CDR3L of the light chain variable domain of SEQ ID NO: 11.

In some embodiments of the present application, the CDR1H, CDR2H and CDR3H of the heavy chain variable domain VH of the first antigen binding region are the CDR1H, CDR2H and CDR3H of the heavy chain variable domain of SEQ ID NO: 1.

In some embodiments of the present application, the CDR region is defined according to the IMGT antibody numbering scheme, and the light chain CDR of the first antigen-binding region is selected from the following combination:
(a) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 12; or
(b) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 14; or
(c) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 16; or
(d) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 18; or
(e) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 20; or
(f) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 22; or
(g) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 24; or
(h) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 26; or
(i) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 28; or
(j) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 30; or
(k) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 32; or
(l) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 34; or
(m) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 36; or
(n) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 38; or
(o) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 40; or
(p) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 42; or
(q) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 44; or
(r) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 46; or
(s) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 48; or
(t) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 50; or
(u) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 52; or
(v) the light chain complementarity determining region 1 (CDR1L) described in the amino acid sequence of SEQ ID: 6, the light chain complementarity determining region 2 (CDR2L) described in the amino acid sequence of SEQ ID: 7, the light chain complementarity determining region 3 (CDR3L) described in the amino acid sequence of SEQ ID: 54; or
(w) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 56;
the heavy chain of the first antigen-binding region includes the heavy chain complementarity determining region 1 (CDR1H) described in the amino acid sequence of SEQ ID: 2, and the heavy chain complementarity determining region 2 (CDR2H) described in the amino acid sequence of SEQ ID: 3, the heavy chain complementarity determining region 3 (CDR3H) described in the amino acid sequence of SEQ ID: 4.

Preferably, the CDR region of the light chain of the first antigen-binding region is selected from the CDR in (a)-(k), (m)-(u) or (w) above; further preferably selected from the CDR of (a)-(c), (h), (k) or (u), most preferably selected from the CDR in (a).

In some embodiments of the present application, the heavy chain variable region of the first antigen binding region comprises the amino acid sequence of SEQ ID NO: 1, SEQ ID NO: 67, SEQ ID NO: 70, SEQ ID NO: 73, SEQ ID NO : 75, SEQ ID NO: 78, SEQ ID NO: 80 or SEQ ID NO: 81; or comprises the heavy chain variable region of the first antigen binding region which is obtained by adding, deleting, or replacing one or more amino acids in SEQ ID NO: 1 and has the same function as SEQ ID NO: 1; preferably comprises the amino acid sequence of SEQ ID NO: 1, SEQ ID NO: 67, SEQ ID NO: 70, SEQ ID NO: 73, SEQ ID NO : 75, SEQ ID NO: 78, SEQ ID NO: 80 or SEQ ID NO: 81, and more preferably comprises the amino acid sequence of SEQ ID NO: 67 or SEQ ID NO: 81.

In some embodiments of the present application, the light chain variable region of the first antigen binding region comprises the amino acid sequence selected from those shown in SEQ ID NO: 11, SEQ ID NO: 13, SEQ ID NO: 15, SEQ ID NO: 17,SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51. SEQ ID NO: 53, SEQ ID NO: 55, SEQ ID NO: 66, SEQ ID NO: 71, SEQ ID NO: 72, SEQ ID NO: 74, SEQ ID NO: 76, SEQ ID NO: 77 or SEQ ID NO:79. Preferably, the light chain variable domain VL of the first antigen binding region comprises the amino acid sequence selected from those shown in SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 55, SEQ ID NO : 66, SEQ ID NO: 71, SEQ ID NO: 72, SEQ ID NO: 74, SEQ ID NO: 76, SEQ ID NO: 77 or SEQ ID NO: 79; More preferably, the light chain variable domain VL of the first antigen binding region comprises the amino acid sequence selected from those shown in SEQ ID NO: 11, SEQ ID NO: 13, SEQ ID NO: 15, SEQ ID NO: 25, SEQ ID NO: 31, SEQ ID NO: 51, SEQ ID NO: 66, SEQ ID NO: 72, SEQ ID NO: 79 or SEQ ID NO: 76; most preferably, the light chain variable domain VL of the first antigen binding region is the amino acid sequence shown in SEQ ID NO: 11, SEQ ID NO: 66, SEQ ID NO: 72, SEQ ID NO: 79 or SEQ ID NO: 76.

In some embodiments of the present application, the BsAb comprises the heavy chain constant region of an IgG, preferably, it comprises a heavy chain constant region of IgG1, IgG4 or IgG2; more preferably, it comprises a heavy chain constant region of IgG4; and most preferably, it comprises a heavy chain constant region as shown in SEQ ID NO:91.

In some embodiments of the present application, the second antigen-binding region that specifically binds to PD-1 includes a heavy chain variable region and a light chain variable region, and the CDR1H, CDR2H and CDR3H of the heavy chain variable region VH are CDR1H, CDR2H and CDR3H of heavy chain variable domains in SEQ ID NO: 83, or the CDR1H, CDR2H and CDR3H of the heavy chain variable region VH are CDR1H, CDR2H and CDR3H of the sequence which is obtained by adding, deleting or replacing one or more amino acids in SEQ ID NO: 83 and has the same function as SEQ ID NO: 83; the CDR1L, CDR2L and CDR3L of the light chain variable region VL are CDR1L, CDR2L and CDR3L of the heavy chain variable domains in SEQ ID NO: 87, or the CDR1L, CDR2L and CDR3L of the heavy chain variable region VL are CDR1L, CDR2L and CDR3L of the sequence which is obtained by adding, deleting or replacing one or more amino acids in SEQ ID NO: 87 and has the same function as SEQ ID NO: 87.

In some embodiments of the present application, the second antigen-binding region that specifically binds to PD-1 includes a heavy chain variable region and a light chain variable region,
the heavy chain variable region comprises CDR1H as shown in SEQ ID NO:84, CDR2H as shown in SEQ ID NO:85 and CDR3H as shown in SEQ ID NO:86; and
the light chain variable region includes CDR1L as shown in SEQ ID NO:88, CDR2L as shown in SEQ ID NO:89 and CDR3L as shown in SEQ ID NO:90.

In some embodiments of the present application, the heavy chain variable region comprises the sequence as shown in SEQ ID NO:83 and the light chain variable region comprises the sequence as shown in SEQ ID NO:87.

In some embodiments of the present application, the first antigen binding region or the second antigen binding region is in scFv form, preferably, the first antigen binding region is in scFv form.

In some embodiments of the present application, the first antigen-binding region and the second antigen-binding region are connected through a Linker,
preferably, the Linker includes (G4S)n, n is an integer greater than 1,
more preferably, the Linker is composed of (G4S)n, n is an integer from 2 to 10,
most preferably, the Linker is composed of (G4S)n, n is 2, 3 or 4, for example, the Linker is GGGGSGGGGSGGGGSGGGGS (SEQ ID NO: 65).

In some embodiments of the present application, the scFv includes a heavy chain variable region and a light chain variable region, and the heavy chain variable region and the light chain variable region are connected by a Linker,
preferably, the Linker includes (G4S)n, n is an integer greater than 1,
more preferably, the Linker is composed of (G4S)n, n is an integer from 2 to 10,
most preferably, the Linker consists of (G4S)n, and n is 2, 3 or 4.

In some embodiments of the present application, the BsAb consists of 4 peptide chains, 2 identical first chains and 2 identical second chains,
the first chain sequentially includes VH of the PD-1 binding region, CH of the PD-1 binding region, Linker, the VH of the ALK-1 binding region, Linker and the VL of the ALK-1 binding region in sequence from the N-terminal to the C-terminal, and
the second chain contains the VL and CL of the PD-1 binding region in sequence from the N-terminal to the C-terminal.

In some embodiments of the present application, the specific sequence of the BsAb is as follows:
the VH sequence of the PD-1 binding region is shown in SEQ ID NO: 83,
the CH sequence of the PD-1 binding region is shown in SEQ ID NO: 91,
the VL sequence of the PD-1 binding region is shown in SEQ ID NO: 87,
the CL sequence of the PD-1 binding region is shown in SEQ ID NO: 92,
the Linker sequence is shown in SEQ ID NO: 65,
the VL sequence of the ALK-1 binding region is as shown in SEQ ID NO: 66, SEQ ID NO: 71, SEQ ID NO: 72, SEQ ID NO: 74, SEQ ID NO: 76, SEQ ID NO: 77 or SEQ ID NO :79, and
the VH sequence of the ALK-1 binding region is as shown in SEQ ID NO: 67, SEQ ID NO: 1, SEQ ID NO: 70, SEQ ID NO: 73, SEQ ID NO: 75, SEQ ID NO: 78, SEQ ID NO :80 or SEQ ID NO:81.

Preferably, the sequence of the first chain is shown in SEQ ID NO: 93; the sequence of the second chain is shown in SEQ ID NO: 94.

In some embodiments of the present application, the BsAb consists of 4 peptide chains, 2 identical first chains and 2 identical second chains,
the first chain sequentially includes the VH of the ALK-1 binding region, the CH of the ALK-1 binding region, the Linker and the PD-1 binding region from the N-terminal to the C-terminal, and
the second chain contains the VL and CL of the ALK-1 binding region in sequence from the N-terminal to the C-terminal.

In some embodiments of the present application, the bispecific antibody consists of 4 peptide chains, 2 identical first chains and 2 identical second chains,
the first chain sequentially includes the VH and CH of the ALK-1 binding region from the N-terminus to the C-terminus, and
the second chain includes the PD-1 binding region, Linker, VL and CL of the ALK-1 binding region in sequence from the N-terminus to the C-terminus.

In some embodiments of the present application, the BsAb consists of 2 identical peptide chains, which sequentially include PD-1 binding region, IgG4-Fc, Linker, ALK- 1 binding region VH, Linker and ALK-1 binding region VL. Herein, IgG4-Fc means the Fc region of IgG4, preferably means the Fc region of human IgG4.

In some embodiments of the present application, the bispecific antibody consists of 4 peptide chains, 2 identical first chains and 2 identical second chains,
the first chain sequentially includes the VH of the ALK-1 binding region, the CH of the ALK-1 binding region, the Linker, the VH of the PD-1 binding region, the Linker, the VL of the PD-1 binding region, and
the second chain contains the VL and CL of the ALK-1 binding region in sequence from the N-terminus to the C-terminus.

According to the fourth aspect of the present application, the present application provides a polynucleotide, which can encode monoclonal antibody or antigen-binding portion thereof that binds to ALK-1 as described in the first aspect, the bispecific antibody or fragment thereof as described in the second or third aspect. The BsAb fragments referred to herein refer to the full length of the polypeptide chain constituting the BsAb or its fragments.

According to the fifth aspect of the present application, the present application provides an expression vector, which can express monoclonal antibody or antigen-binding portion thereof that binds to ALK-1 as described in the first aspect, the bispecific antibody or fragment thereof as described in the second or third aspect.

According to the sixth aspect of the present application, the present application provides an engineered cell, which contains the vector described in the fourth aspect.

According to the seventh aspect of the present application, the present application provides a pharmaceutical composition comprising monoclonal antibody or antigen-binding portion thereof that binds to ALK-1 as described in the first aspect, or the bispecific antibody or fragment thereof as described in the second or third aspect, or the polynucleotide as described in the fourth aspect, or the vector as described in the fifth aspect or the cell as described in the sixth aspect, and a pharmaceutically acceptable carrier.

In the eighth aspect, the present application provides the use of monoclonal antibody or antigen-binding portion thereof that binds to ALK-1 as described in the first aspect, the bispecific antibody or fragment thereof as described in the second or third aspect, the polynucleotides as described in the fourth aspect, the vector as described in the fifth aspect, the cells as described in the sixth aspect, or the pharmaceutical compositions as described in the seventh aspect in the preparation of drugs for inhibiting angiogenesis..

In some embodiments, the disease about tumor angiogenesis is a solid tumor or a hematological tumor.

In some embodiments, the disease about tumor angiogenesis is selected from the group consisting of esophageal cancer (eg, esophageal adenocarcinoma and esophageal squamous cell carcinoma), brain tumors, lung cancer (eg, small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, intestinal cancer liver metastasis, kidney cancer, urothelial cancer, non-Hodgkin lymphoma, central nervous system tumors (such as glioma, glioblastoma multiforme, glioma, or sarcoma), prostate cancer, or thyroid cancer.

In a ninth aspect, the present application provides a method for inhibiting angiogenesis, comprising administering an effective dose of the ALK-1-binding monoclonal antibody or antigen-binding portion thereof as described in the first aspect. , the bispecific antibody or fragment thereof as described in the second or third aspect, the polynucleotide as described in the fourth aspect, the vector as described in the fifth aspect or the cell as described in the sixth aspect or the pharmaceutical composition as described in seven aspects to a subject in need thereof, for example, inhibiting angiogenesis in tumors or some eye diseases, eye diseases mentioned such as retinal detachment, vitreoretinopathy, retinopathy of prematurity, glaucoma, synovitis, and proliferative diabetes Retinopathy, branch retinal vein occlusion, etc.

In a tenth aspect, the present application provides a method for treating tumors, comprising administering an effective dose of the ALK-1-binding monoclonal antibody or antigen-binding portion thereof as described in the first aspect, the BsAb or fragment thereof as described in the second or third aspect, the polynucleotide as described in the fourth aspect, the vector as described in the fifth aspect or the cell as described in the sixth aspect or the pharmaceutical composition as described in the seventh aspect to a subject in need thereof,the tumor mentioned include solid tumors and non-solid tumors, such as advanced or refractory hepatocellular carcinoma (HCC), colorectal cancer (RCC), non-small cell lung cancer (NSCLC), triple negative Breast cancer, gastric cancer (GC), gastroesophageal junction (GEJ) adenocarcinoma, cholangiocarcinoma, urothelial cancer (UC), esophageal square cell carcinoma (ESCC), brain tumor, lung cancer, breast cancer, ovarian cancer, fallopian tube cancer , glioblastoma, colorectal adenocarcinoma, intestinal adenocarcinoma, pituitary tumor, pituitary adenoma, pituitary macroadenoma, gestational trophoblastic tumor, choriocarcinoma, placental site trophoblastic tumor, epithelioid trophoblastic tumor, renal cell carcinoma, lung adenocarcinoma and gliosarcoma.

In an eleventh aspect, the present application provides a composition for inhibiting angiogenesis, which comprises the ALK-1-binding monoclonal antibody or antigen-binding portion thereof as described in the first aspect, the bispecific antibody or fragment thereof as described in the second or third aspect, the polynucleotide as described in the fourth aspect, the vector as described in the fifth aspect or the cell and a pharmaceutically acceptable carrier as described in the sixth aspect.

In a twelfth aspect, the present application provides a composition for treating tumors, which includes the ALK-1-binding monoclonal antibody or antigen-binding portion thereof as described in the first aspect, the bispecific antibody or fragment thereof as described in the second or third aspect, the polynucleotide as described in the fourth aspect, the vector as described in the fifth aspect or the cell and a pharmaceutically acceptable carrier as described in the sixth aspect.

In the thirteenth aspect, the present application provides a pharmaceutical composition, including the BsAb (such as BEV813 bsAb) of the second aspect of the present application and an anti-PD-1 antibody (such as pembrolizumab, nivolumab, toripalimab, sintilimab, camrelizumab, tislelizumab, penpilimab, serpalizumab, slulimumab etc.) or anti-PD-L1 antibodies (such as durvalumab, atezolizumab, envolizumab, sugelimumab, etc.), or cytotoxic and non-cytotoxic small molecules medicine; the pharmaceutical composition can be used to treat solid tumors, including but not limited to hepatocellular carcinoma, lung cancer, colorectal cancer, gastric cancer, breast cancer, esophageal squamous cell carcinoma, urothelial cancer, nausea pleural mesothelioma, etc.

In the fourteenth aspect, the present application provides a pharmaceutical composition, including the ALK-1 monoclonal antibody as described in the first aspect of the present application and an anti-PD-1 antibody (such as pembrolizumab, nivolumab, toripalimab, sintilimab, camrelizumab, tislelizumab, penpilimab, serpalizumab, slulimumab etc.) or anti-PD-L1 antibodies (such as durvalumab, atezolizumab, envolizumab, sugalizumab, etc.) or anti-VEGF antibody (such as bevacizumab, etc.), or cytotoxic and non-cytotoxic small molecule drugs; the pharmaceutical composition can be used to treat solid tumors, including but not limited to hepatocellular carcinoma, lung cancer, colorectal cancer, gastric cancer, breast cancer, esophageal squamous cell carcinoma, urothelial cancer, nausea pleural mesothelioma, etc.

### Description of the Drawings

Figure 1 shows the schematic diagram of the anti-ALK-1 antibody of the present application.
Figure 2 shows the schematic diagram of the BEV813 bsAb of the present application.
Figure 3 shows the comparison of cell binding activities of ALK1 mAbs, BEV813 bsAb binding to CHO-K1-hALK1 cell.
Figure 4 shows the binding to ALK1 epitope map of BEV813 bsAb competing with BMP9.
Figure 5 shows a comparison of the cellular functional activities of BEV813 bsAb and bevacizumab.
Figure 6 shows a comparison of the cellular functional activities of BEV813 and ALK1 mAb.
Figure 7 shows a comparison of the effects of BEV813 and bevacizumab on blocking HUVEC cell proliferation.
Figure 8 shows a comparison of the effects of BEV813 bsAb, ALK1 mAb and bevacizumab on blocking microtubule formation in HUVEC cells.
Figure 9 shows a comparison of the efficacy and safety of BEV813 bsAb and other antibodies in the CDX mouse model (MDA-MB-231) .
Figure 10 shows a comparison of the efficacy and safety of BEV813 bsAb and other antibodies in the CDX mouse model (KYSE450).
Figure 11 shows a comparison of the efficacy and safety of BEV813 bsAb and other antibodies in the CDX mouse model (HCC827).
Figure 12 shows a comparison of the efficacy and safety of BEV813 bsAb and other antibodies in the PDTX mouse model of intestinal cancer liver metastasis.
Figure 13 shows the purity of BEV813 bsAb mutants using SDS-PAGE.
Figure 14 shows the result of the different BEV813 bsAb mutants bands digested by Ides enzyme digestion using SDS-PAGE.
Figure 15 shows HPLC results.
Figure 16 shows the mass spectrum of the F(ab')2 of BEV813.
Figure 17 shows the mass spectrum of the sFc domain of BEV813 digested by PNGaseF.
Figure 18 shows the mass spectrum of the Fc domain of BEV813 digested by PNGaseF .
Figure 19 shows the mass spectrum of the sFc domain of BEV813-9 digested by PNGaseF.
Figure 20 shows the mass spectrum of F(ab')2 of BEV813-9.
Figure 21 shows the test results of isomers of BEV813 and BEV813-9 detected by nrCGE.
Figure 22 shows the activity of BEV813 bsAb and its mutants binding to CHO-K1-hALK-1 cells.
Figure 23 shows the result of BEV813 bsAb and its mutants competing with BMP9 for binding to the CHO-K1-hALK1 cell binding epitope.
Figure 24 shows the structural diagram of Nivo813 bispecific antibody.
Figure 25 shows the binding affinity of Nivo813 bsAb to PD-1 and ALK-1 simultaneously.
Figure 26 shows the comparison of binding activities of Nivo813 bsAb, ALK-1mAb (#18) to CHO-K1-hALK1 cell surface receptors.
Figure 27 shows comparison of the activities of Nivo813 bsAb, nivolumab binding to the H-PD-1 NFAT Reportor Jurkat cell surface receptors.
Figure 28 shows the results of Nivo813 bsAb blocking PD-1/PD-L1 interaction induced transduction of TCR signaling pathway and NFAT-mediated luciferase expression.
Figure 29 shows the results of Nivo813 bsAb blocking BMP9 induced phosphorylation of Smad1.
Figure 30 shows the effect of Nivo813 bsAb on inhibiting HUVEC microtubule formation.
Figure 31 shows a comparison of the effects of Nivolumab, ALK-1 mAb (#18) and Nivo813 bsAb in mouse tumor models.

### DETAILED DESCRIPTION

### Definitions

Unless otherwise defined, all technical and scientific terms used herein have the same meaning as commonly understood by one of ordinary skilled in the art.

Although the numerical ranges and approximate values of parameters shown in the broad scope of the present application are approximate, the numerical values shown in the specific examples are set forth as accurately as possible. Any numerical values, however, are inherently bound to contain certain errors resulting from the standard deviation found in their respective measurements. Additionally, all ranges disclosed herein are to be understood to encompass any and all subranges subsumed therein. For example, a range stated as "1 to 10" shall be deemed to include any and all subranges between the minimum value of 1 and the maximum value of 10, inclusive; that is, all subranges beginning with a minimum value of 1 or greater , such as 1 to 6.1, and a subrange ending with a maximum value of 10 or less, such as 5.5 to 10. Additionally, any reference referred to as "incorporated herein" shall be understood to be incorporated in its entirety.

It should also be noted that, as used in this specification, the singular includes the plural form of its referent unless expressly and unequivocally limited to one referent. The term "or" may be used interchangeably with the term "and/or" unless the context clearly indicates otherwise.
As used herein, the term "antibody" encompasses full-length antibodies (eg, IgG1 or IgG4 antibodies), its various functional fragments (for example, may only contain the antigen-binding portion, such as Fab, F(ab')₂ or scFv fragment) and modified antibodies (such as humanization, glycosylation, etc.). In some applications, it may be useful to make modifications to remove undesired glycosylation sites, or the absence of fucose moieties on the oligosaccharide chain, for example to enhance antibody-dependent cellular cytotoxicity (ADCC) functionality of antibodies. In other applications, galactosylation modifications can be performed to alter complement-dependent cytotoxicity (CDC).

The term "CDR region" or "CDR" as used herein refers to the complementarity determining regions of the heavy and light chains of an immunoglobulin. CDRs can be defined using various numbering schemes, such as Kabat (Wu et al., (1970) J Exp Med 132:211-50; Kabat et al., "Sequences of Proteins of Immunological Interest", 5th ed., Public Health Service, National Institutes of Health, Bethesda, Md., 1991), Chothia (Chothia et al., (1987) , J Mol Biol 196:901-17), IMGT (Lefranc et al. (2003) DeV Comp Immunol 27:55-77) and AbM (Martin and Thornton, (1996) J Bmol Biol 263:800-15). Correspondences between various numbering systems and variable region numbering are described (Lefranc et al., (2003), DeV Comp Immunol 27:55-77; Honegger and Pluckthun, (2001), J MolBiol 309:657-70; International Immunogenetics (IMGT) database; Web resource, http://www.imgt.org). Available programs (such as abYsis of UCL Business PLC) can be used to characterize CDRs. Unless otherwise expressly stated in the specification, as used herein, the terms "CDR", "CDR1H", "CDR2H", "CDR3H", "CDR1L", "CDR2L" and "CDR3L" include CDR defined by any method as described above (Kabat, Chothia, IMGT or AbM) defined CDR. The term CDR or CDRs is used herein to indicate one, or several or even all of these regions, as the case may be, which region contains the majority of the amino acid residues responsible for binding to the antigen or the epitope it recognizes. In the examples of the present application, the CDR areas are defined by the IMGT system.

The term "Fc region" or "Fc portion" as used herein is a term well known to those skilled in the art.

As used herein, the term "Fab region" refers to a region composed of the VH and CH1 domains of the heavy chain of an immunoglobulin ("Fab heavy chain") or the VL and CL domains of the light chain ("Fab light chain"), or both By.

As used herein, the term "scFv" or "single chain antibody fragment" means a single chain consisting of an antibody heavy chain variable region and an antibody light chain variable region linearly linked together by a linker (e.g., a short peptide of 10-25 amino acids), which exhibits specific binding to antigen.

The term "peptide linker" as used in the present application refers to a peptide used to combine different antigen binding sites and/or antibody fragments that ultimately comprise different antigen binding sites (e.g., single chain Fv, full length antibody, VH domain and/or VL domain, Fab, F(ab')2 and Fc region), preferably having the amino acid sequence originated by a synthesis technology. The peptide linker may comprise one or more of the amino acid sequences listed in the Examples, as well as other optionally selected amino acids.

As used herein, the term "binding" or " binding specifically" refers to an antibody binding to an antigenic epitope measured by in vitro assay (ELISA). It can be also achieved by using a molecular interaction instrument (Octet, Fortebio) to detect the binding affinity to antigen or FcyRIII. Binding affinity is expressed by the terms ka or kon (binding rate constant of the antibody in the antibody/antigen compound), kd or koff or kdis (dissociation constant) and KD (equilibrium dissociation constant, kd/ka or koff/kon or kdis/kon).

As used herein, a "therapeutically effective amount" or "effective amount" refers to a dose sufficient to demonstrate benefit to the subject to which it is administered. The actual amount administered, as well as the rate and time course of administration, will depend on the individual condition and severity of the person being treated. The prescription of treatment (e.g. the determination of the dosage, etc.) is ultimately the responsibility of general practitioners and other medical practitioners, and decisions are made relying on them. Usually, factors such as the disease being treated, the individual situation of the patient, the site of delivery, the method of administration, and other factors known to the doctors are taken into account.

The term "subject" as used herein refers to mammals, such as humans, but can also be other animals, such as wild animals (such as herons, storks, cranes, etc.), domestic animals (such as ducks, geese, etc.) or experimental animals (such as Orangutans, monkeys, rats, mice, rabbits, guinea pigs, prairie dogs, ground squirrels, etc.).

The compositions of the present application may be administered by a variety of methods known in the art. The skilled artisan will understand that the route and/or mode of administration will vary depending upon the desired results. In order to administer a compound of the present application by a particular route of administration, it may be necessary to cover the compound with a material that avoids its inactivation, or to co-administer the compound with such material. For example, the compound can be administered to the subject in a suitable carrier, such as liposomes or diluents. Pharmaceutically acceptable diluents include saline solutions and aqueous buffers. Pharmaceutical carriers include sterile aqueous solutions or dispersions and sterile powders for the temporary preparation of sterile injectable solutions or dispersions. The use of such media and agents for pharmaceutically active substances is known in the art.

The compositions of the present application may also contain adjuvants such as preservatives, wetting agents, emulsifiers and dispersants. The presence of microorganisms can be avoided both by the sterilization procedures described above and by the inclusion of various antibacterial and antifungal agents such as parabens, chlorobutanol, phenol, sorbic acid, etc. It may also be desirable to include isotonic agents in the composition, such as sugar, sodium chloride, and the like. In addition, prolonged absorption of the injectable pharmaceutical form may be brought about by the inclusion of agents such as aluminum monostearate and gelatin.

Adalimumab (trade name: Humira) is an antibody developed by AbbVie. It can bind tumor necrosis factor-α (TNFα) and is a TNF-inhibiting biological drug. Ipilimumab (trade name: Yervoy) is an antibody developed by Bristol-Myers Squibb (BMS) that can effectively block a molecule called cytotoxic T cell antigen-4 (CTLA-4).

Bevacizumab (trade name: Avastin) is a recombinant humanized anti-VEGF monoclonal antibody approved by the FDA on February 26, 2004, it was the first drug approved for marketing in the United States to inhibit tumor Angiogenesis. It can bind to human vascular endothelial growth factor (VEGF) and block its biological activity confirmed by in vivo and in vitro detection systems.

Nivolumab (trade name: Opdivo) is a genetically engineered human IgG4 monoclonal antibody that targets the human cell surface receptor named Programmed Cell Death-1 (PD-1, PCD-1), which has negative immunoregulatory functions.

Bispecific Antibodies, also known as bifunctional antibodies, are antibodies with the ability to bind at least two different antigens or two different epitopes of the same antigen. They can be produced through immune sorting and purification. In addition, it can also be obtained through genetical engineering. The method of genetic engineering has certain advantages due to their flexibility in terms of optimization of binding sites, format and yield. Currently, more than 45 kinds of format have been proven (Dafne Müller, Kontermann R E. 2010, BioDrugs, 24(2): 89-98). A variety of bispecific antibodies that have been developed so far are in the form of IgG-scFv, that is, the Morrison pattern (Coloma MJ, Morrison SL. 1997, Nat Biotechnol. 15: 159-163). Due to its form similar to the naturally occurring IgG form, it has advantages in antibody engineering, expression and purification, and has been proven to be one of the ideal forms of bispecific antibodies (Miller BR, Demarest SJ, et al., 2010, Protein Eng Des Sel; 23: 549-57; Fitzgerald J, Lugovskoy A. 2011. MAbs; 3: 299-309).

Human IgG1-LALA refers to the artificial mutation of two amino acid positions in the Fc segment of natural human IgG1 antibodies, namely L234A/L235A (LALA) (Lund J, Winter G, Jones PT, Pound JD, Tanaka T, Walker MR et al. Human Fc gamma RI and Fc gamma RII interact with distinct but overlapping sites on human IgG. J Immunol. 1991; 147: 2657 -2662.). These mutations can reduce the binding to the IgG Fc receptors FcγRI, FcγRII and FcγRIII, thereby reducing the effects of ATCC, ADCP, CDC.

### Specific implementation methods

### 1. Screening of anti-ALK-1 monoclonal antibodies

### 1.1 Construction, expression and purification of anti-ALK-1 monoclonal antibodies and mutants

After cloning the target sequence by PCR, the PCR product was purified according to the instructions of the DNA gel recovery kit (Axygen, AP-GX-250), and constructed into the pcDNA 3.4 vector (manufactured by Biointron). Then this plasmid was transformed into Top10 bacterial competent cells for the production of recombinant plasmid. The cells were subsequently plated on an agar plate containing ampicillin and cultured at 37°C overnight. The recombinant plamids were purified from the single white colonies and sequenced.

The transient expression method was adopted. The plasmid was transferred into Expi293F cells (Gibco, A14528). The transfection method was as follows (take a 30mL system as an example): The Expi293F cells were passaged according to the required transfection volume, and the cell density was adjusted to 1.5×10⁶ cells/ml before transfection; the cells to be transfected were counted, and the cell density was adjusted to 3×10⁶ cells/ml with pre-warmed Expi293F cell culture medium; 60ug of the plasmid was diluted with 1ml of culture medium and mixed well as solution 1; 15ul of transfection reagent was diluted with 1ml of culture medium, and mixed well as solution 2. Solution 2 was added to Solution 1 and mixed well. And after incubating at 37°C for 15 minutes, the mixed transfection solution was added dropwise to the cell solution while shaking. drop by drop into the cell solution, while shaking, then the cells were placed on a shaker for culture. After one week of expression, the supernatant was collected and centrifuged at 8000 rpm for 5 minutes. The protein in the cell supernatant was purified using a Protein A affinity chromatography column (self-made by Biointron), and the protein concentration was detected using a NanoDrop instrument (Thermo-NanoDrop^{™} One, item number ND-ONE-W).

The heavy chain constant region of the anti-ALK-1 monoclonal antibody uses the sequence of IgG1-LALA.

The present application uses anti-ALK-1 antibody (hereinafter referred to as BM) as a reference (benchmark), and its sequence information is as follows:
(1) BM
   Heavy chain variable region (VH) 
QVQLQESGPGLVKPSQTLSLTCTVSGGSISSGEYYWNWIRQHPGKGLEWI
GYIYYSGSTYYNPSLKSRVTISVDTSKNQFSLKLSSVTAADTAVYYCARESVAGFD
YWGQGTLVTVSS (SEQ ID NO: 1)
   3 CDRs of VH:
      CDR1H: GGSISSGEYY (SEQ ID NO: 2)
      CDR2H: IYYSGST (SEQ ID NO: 3)
      CDR3H: ARESVAGFDY (SEQ ID NO: 4)
      Light chain variable region (VL) 
EIVLTQSPGTLSLSPGERATLSCRASQSVSSSYLAWYQQKPGQAPRLLIYGT
SSRATGIPDRFSGSGSGTDFTLTISRLEPEDFAVYYCQQYGSSPITFGQGTRLEIK (SEQ ID
NO: 5)
      3 CDRs of VL:
         CDR1L: QSVSSSY (SEQ ID NO: 6)
         CDR2L: GTS (SEQ ID NO: 7)
         CDR3L: QQYGSSPIT (SEQ ID NO: 8)
      Heavy chain constant region (CH) 
ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKT
HTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESN
GQPENNYKTTPPVLDSDGSFFLYSKLTVDKSRWQQGNVFSCSVMHEALHNH
YTQKSLSLSPG (SEQ ID NO: 9)
      Light chain constant region (CL)



(SEQ ID NO: 10)

Based on the BM, the epitope is predicted by the deep learning model and the affinity change is predicted by the residue mutation module for the NMR (nuclear magnetic resonance) crystal structure respectively. Then, the final antigen mutation sequence is determined according to the scores. The residues with higher occurrence frequencies are classified into epitopes. Subsequently, site-saturation mutations are carried out on these antigen residues, and the change in the affinity of the site-saturation mutations is analyzed. After screening, the single-point mutation sequence of the anti-ALK-1 antibody for further experimental detection is finally determined. The numbers of each mutant and the corresponding sequence information are as follows:
(2) #18
   Compared with the anti-ALK-1 antibody BM, the anti-ALK-1 antibody #18, except for the point mutations of Q91A and S95W in the light chain variable region (VL) (the mutations at positions 91 and 95 based on the sequence of SEQ ID NO: 5, and the description of other mutants are similar), and the rest are consistent with the antibody sequence of BM.
   The light chain sequence of antibody #18 is as follows:
      Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQAYGSWPITFGQGTRLEIK (SEQ ID NO:
11)
      3 CDRs of VL:
         CDR1L: QSVSSSY (SEQ ID NO: 6)
         CDR2L: GTS (SEQ ID NO: 7)
         CDR3L: QAYGSWPIT (SEQ ID NO: 12)

      (3) #3
         Compared with the anti-ALK-1 antibody BM, the anti-ALK-1 antibody #3 shares the same sequence except for the point mutations of Q91L and S95F in the light chain variable region (VL).
         The light chain sequence of antibody #3 is as follows:
            Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQLYGSFPITFGQGTRLEIK (SEQ ID NO: 13)
            3 CDRs of VL:
               CDR1L: QSVSSSY (SEQ ID NO: 6)
               CDR2L: GTS (SEQ ID NO: 7)
               CDR3L: QLYGSFPIT (SEQ ID NO: 14)
      (4) #1
         Compared with the anti-ALK-1 antibody BM, the anti-ALK-1 antibody #1 shares the same sequence except for the point mutations of Q91G and S95F in the light chain variable region (VL).
         The light chain sequence of antibody #1 is as follows:
            Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQGYGSFPITFGQGTRLEIK (SEQ ID NO: 15)
            3 CDRs of VL:
               CDR1L: QSVSSSY (SEQ ID NO: 6)
               CDR2L: GTS (SEQ ID NO: 7)
               CDR3L: QGYGSFPIT (SEQ ID NO: 16)
      (5) #2
         Compared with the anti-ALK-1 antibody BM, the anti-ALK-1 antibody #2 shares the same sequence except for the point mutations of Q91F and S95F in the light chain variable region (VL).
      The light chain sequence of antibody #2 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQFYGSFPITFGQGTRLEIK (SEQ ID NO: 17)
         3 CDRs of VL:
            CDR1L: QSVSSSY (SEQ ID NO: 6)
            CDR2L: GTS (SEQ ID NO: 7)
            CDR3L: QFYGSFPIT (SEQ ID NO: 18)
      (6) #6
         Compared with the anti-ALK-1 antibody BM, the anti-ALK-1 antibody #6 shares the same sequence except for the point mutations of Q91P and S95V in the light chain variable region (VL).
         The light chain sequence of antibody # 6 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQPYGSVPITFGQGTRLEIK (SEQ ID NO: 19)
         3 CDRs of VL:
            CDR1L: QSVSSSY (SEQ ID NO: 6)
            CDR2L: GTS (SEQ ID NO: 7)
            CDR3L: QPYGSVPIT (SEQ ID NO: 20)
      (7) #7
         Compared with the anti-ALK-1 antibody BM, the anti-ALK-1 antibody #7 shares the same sequence except for the point mutations of Q91A and S95V in the light chain variable region (VL).
         The light chain sequence of antibody #7 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQAYGSVPITFGQGTRLEIK (SEQ ID NO: 21)
         3 CDRs of VL:
            CDR1L: QSVSSSY (SEQ ID NO: 6)
            CDR2L: GTS (SEQ ID NO: 7)
            CDR3L: QAYGSVPIT (SEQ ID NO: 22)
      (8) #8
         Compared with the anti-ALK-1 antibody BM, the anti-ALK-1 antibody #8 shares the same sequence except for the point mutations of Q91M and S95I in the light chain variable region (VL).
         The light chain sequence of antibody #8 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQMYGSIPITFGQGTRLEIK (SEQ ID NO: 23)
         3 CDRs of VL:
            CDR1L: QSVSSSY (SEQ ID NO: 6)
            CDR2L: GTS (SEQ ID NO: 7)
            CDR3L: QMYGSIPIT (SEQ ID NO: 24)
      (9) #9
         Compared with the anti-ALK-1 antibody BM, the anti-ALK-1 antibody #9 shares the same sequence except for the point mutations of Q91I and S95W in the light chain variable region (VL).
         The light chain sequence of antibody #9 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQIYGSWPITFGQGTRLEIK (SEQ ID NO: 25)
         3 CDRs of VL:
            CDR1L: QSVSSSY (SEQ ID NO: 6)
            CDR2L: GTS (SEQ ID NO: 7)
            CDR3L: QIYGSWPIT (SEQ ID NO: 26)
      (10) #12
         Compared with the anti-ALK-1 antibody BM, the anti-ALK-1 antibody #12 shares the same sequence except for the point mutations of Q91I and S95I in the light chain variable region (VL). The light chain sequence of antibody #12 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQIYGSIPITFGQGTRLEIK (SEQ ID NO: 27)
         3 CDRs of VL:
            CDR1L: QSVSSSY (SEQ ID NO: 6)
            CDR2L: GTS (SEQ ID NO: 7)
            CDR3L: QIYGSIPIT (SEQ ID NO: 28)
      (11) #13
         Compared with the anti-ALK-1 antibody BM, the anti-ALK-1 antibody #13 shares the same sequence except for the point mutations of Q91P and S95I in the light chain variable region (VL). The light chain sequence of antibody #13 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQPYGSIPITFGQGTRLEIK (SEQ ID NO: 29)
         3 CDRs of VL:
            CDR1L: QSVSSSY (SEQ ID NO: 6)
            CDR2L: GTS (SEQ ID NO: 7)
            CDR3L: QPYGSIPIT (SEQ ID NO: 30)
      (12) #14
         Compared with the anti-ALK-1 antibody BM, the anti-ALK-1 antibody #14 shares the same sequence except for the point mutations of Q91M and S95W in the light chain variable region (VL). The light chain sequence of antibody #14 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQMYGSWPITFGQGTRLEIK (SEQ ID NO:
31)
         3 CDRs of VL:
            CDR1L: QSVSSSY (SEQ ID NO: 6)
            CDR2L: GTS (SEQ ID NO: 7)
            CDR3L: QMYGSWPIT (SEQ ID NO: 32)
      (13) #15
         Compared with the anti-ALK-1 antibody BM, the anti-ALK-1 antibody #15 shares the same sequence except for the point mutations of Q91W and S95V in the light chain variable region (VL).
         The light chain sequence of antibody #15 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQWYGSVPITFGQGTRLEIK (SEQ ID NO:
33)
         3 CDRs of VL:
            CDR1L: QSVSSSY (SEQ ID NO: 6)
            CDR2L: GTS (SEQ ID NO: 7)
            CDR3L: QWYGSVPIT (SEQ ID NO: 34)
      (14) #16
         Compared with the anti-ALK-1 antibody BM, the anti-ALK-1 antibody #16 shares the same sequence except for the point mutations of Q91V and S95I in the light chain variable region (VL).
         The light chain sequence of antibody #16 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQVYGSIPITFGQGTRLEIK (SEQ ID NO: 35)
         3 CDRs of VL:
            CDR1L: QSVSSSY (SEQ ID NO: 6)
            CDR2L: GTS (SEQ ID NO: 7)
            CDR3L: QVYGSIPIT (SEQ ID NO: 36)
      (15) #17
         Compared with the anti-ALK-1 antibody BM, the anti-ALK-1 antibody #17 shares the same sequence except for the point mutations of Q91G and S95I in the light chain variable region (VL).
         The light chain sequence of antibody #17 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQGYGSIPITFGQGTRLEIK (SEQ ID NO: 37)
         3 CDRs of VL:
            CDR1L: QSVSSSY (SEQ ID NO: 6)
            CDR2L: GTS (SEQ ID NO: 7)
            CDR3L: QGYGSIPIT (SEQ ID NO: 38)
      (16) #19
         Compared with the anti-ALK-1 antibody BM, the anti-ALK-1 antibody #19 shares the same sequence except for the point mutations of Q91F and S95W in the light chain variable region (VL).
         The light chain sequence of antibody #19 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQFYGSWPITFGQGTRLEIK (SEQ ID NO: 39)
         3 CDRs of VL:
            CDR1L: QSVSSSY (SEQ ID NO: 6)
            CDR2L: GTS (SEQ ID NO: 7)
            CDR3L: QFYGSWPIT (SEQ ID NO:40)
      (17) #23
         Compared with the anti-ALK-1 antibody BM, the anti-ALK-1 antibody #23 shares the same sequence except for the point mutations of Q91F and S95V in the light chain variable region (VL).
         The light chain sequence of antibody #23 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQFYGSVPITFGQGTRLEIK (SEQ ID NO: 41)
         3 CDRs of VL:
            CDR1L: QSVSSSY (SEQ ID NO: 6)
            CDR2L: GTS (SEQ ID NO: 7)
            CDR3L: QFYGSVPIT (SEQ ID NO: 42)
      (18) #24 Compared with the anti-ALK-1 antibody BM, the anti-ALK-1 antibody #24 shares the same sequence except for the point mutations of Q91F and S95I in the light chain variable region (VL). The light chain sequence of antibody #24 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQFYGSIPITFGQGTRLEIK (SEQ ID NO: 43)
         3 CDRs of VL:
            CDR1L: QSVSSSY (SEQ ID NO: 6)
            CDR2L: GTS (SEQ ID NO: 7)
            CDR3L: QFYGSIPIT (SEQ ID NO: 44)
      (19) #25
         Compared with the anti-ALK-1 antibody BM, the anti-ALK-1 antibody #25 shares the same sequence except for the point mutations of Q91V and S95W in the light chain variable region (VL).
         The light chain sequence of antibody #25 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQVYGSWPITFGQGTRLEIK (SEQ ID NO:
45)
         3 CDRs of VL:
            CDR1L: QSVSSSY (SEQ ID NO: 6)
            CDR2L: GTS (SEQ ID NO: 7)
            CDR3L: QVYGSWPIT (SEQ ID NO: 46)
      (20) #26
         Compared with the anti-ALK-1 antibody BM, the anti-ALK-1 antibody #26 shares the same sequence except for the point mutations of Q91V and S95V in the light chain variable region (VL).
         The light chain sequence of antibody #26 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQVYGSVPITFGQGTRLEIK (SEQ ID NO: 47)
         3 CDRs of VL:
            CDR1L: QSVSSSY (SEQ ID NO: 6)
            CDR2L: GTS (SEQ ID NO: 7)
            CDR3L: QVYGSVPIT (SEQ ID NO: 48)
      (21) #27
         Compared with the anti-ALK-1 antibody BM, the anti-ALK-1 antibody #27 shares the same sequence except for the point mutations of Q91G and S95W in the light chain variable region (VL).
         The light chain sequence of antibody #27 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQGYGSWPITFGQGTRLEIK (SEQ ID NO:
49)
         3 CDRs of VL:
            CDR1L: QSVSSSY (SEQ ID NO: 6)
            CDR2L: GTS (SEQ ID NO: 7)
            CDR3L: QGYGSWPIT (SEQ ID NO: 50)
      (22) #28
         Compared with the anti-ALK-1 antibody BM, the anti-ALK-1 antibody #28 shares the same sequence except for the point mutations of Q91I and S95V in the light chain variable region (VL).
         The light chain sequence of antibody #28 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQIYGSVPITFGQGTRLEIK (SEQ ID NO: 51)
         3 CDRs of VL:
            CDR1L: QSVSSSY (SEQ ID NO: 6)
            CDR2L: GTS (SEQ ID NO: 7)
            CDR3L: QIYGSVPIT (SEQ ID NO: 52)
      (23) #29
         Compared with the anti-ALK-1 antibody BM, the anti-ALK-1 antibody #29 shares the same sequence except for the point mutations of Q91P and S95W in the light chain variable region (VL).
         The light chain sequence of antibody #29 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQPYGSWPITFGQGTRLEIK (SEQ ID NO: 53)
         3 CDRs of VL:
            CDR1L: QSVSSSY (SEQ ID NO: 6)
            CDR2L: GTS (SEQ ID NO: 7)
            CDR3L: QPYGSWPIT (SEQ ID NO: 54)
      (24) #30
         Compared with the anti-ALK-1 antibody BM, the anti-ALK-1 antibody #30 shares the same sequence except for the point mutations of Q91G and S95V in the light chain variable region (VL).
         The light chain sequence of antibody #30 is as follows:
         Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQGYGSVPITFGQGTRLEIK (SEQ ID NO: 55)
         3 CDRs of VL:
            CDR1L: QSVSSSY (SEQ ID NO: 6)
            CDR2L: GTS (SEQ ID NO: 7)
            CDR3L: QGYGSVPIT (SEQ ID NO: 56)

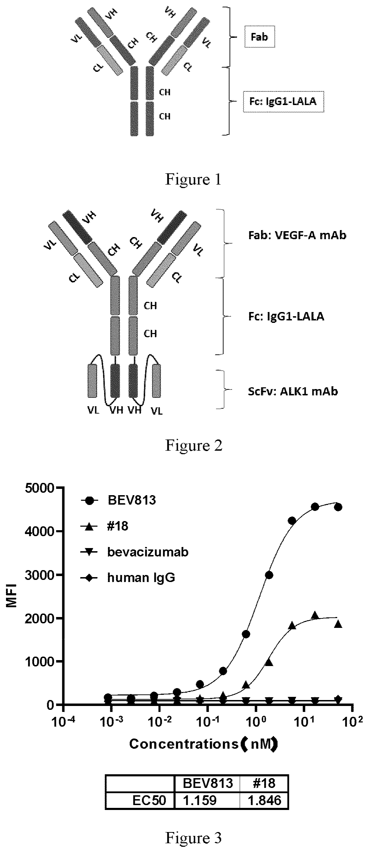

The structural schematic diagram corresponding to the above anti-ALK-1 monoclonal antibody and its mutant number is shown in Figure 1.

### 1.2 Determination of binding affinity of anti-ALK-1 monoclonal antibodies and their mutants

Gator (manufacturer: Gatorbio) affinity was determined according to existing methods (Estep, P et al., Solution-based measurement of high-throughput antibody-antigen affinity and epitope ranking, MAbs, 2013.5(2): p.270-8) conduct. Briefly, the sensor was equilibrated offline in the analysis buffer for 10 minutes, and then detected online for 60 seconds to establish a baseline. BM antibodies and each mutant were loaded online to the AHC sensor. The sensor was connected to an Anti-HFC probe (Probelife, 20- 5036). Then put the sensor into the prepared human ALK-1 antigen protein ("Human ALK-1/ACVRL1 Protein, His Tag", brand: Acro; product number: AL1-H5227; this protein contains the amino acid sequence 22-118 of the extracellular segment of human ALK-1 protein and has a polyhistidine tag at the C-terminus), then the sensor was transferred to PBS for dissociation. Kinetic analyzes were performed using a 1:1 binding model.

The affinity KD(M) results of BM and its mutants with human ALK-1 antigen protein are shown in Table 1 below:

**Table 1 Summary of binding Kinetics between different ALK-1 antibodies and ALK-1 antigen**

| Antigen | Antibody NO. | Mutations | Full R² | koff(1/s) | kon(1/Ms) | KD(M) |
|---|---|---|---|---|---|---|
| ALK-1/His | BM | no | 0.992 | 1.88E-03 | 4.41E+05 | 4.26E-09 |
| ALK-1/His | #3 | Q91L, S95F | 0.994 | 3.49E-04 | 4.16E+05 | 8.38E-10 |
| ALK-1/His | #1 | Q91G, S95F | 0.996 | 3.25E-04 | 3.76E+05 | 8.65E-10 |
| ALK-1/His | #2 | Q91F, S95F | 0.995 | 3.76E-04 | 3.70E+05 | 1.01E-09 |
| ALK-1/His | #6 | Q91P, S95V | 0.995 | 9.79E-04 | 3.98E+05 | 2.46E-09 |
| ALK-1/His | #7 | Q91A, S95V | 0.994 | 4.33E-04 | 3.63E+05 | 1.19E-09 |
| ALK-1/His | #8 | Q91M, S95I | 0.996 | 3.99E-04 | 3.78E+05 | 1.06E-09 |
| ALK-1/His | #9 | Q91I, S95W | 0.994 | 2.13E-04 | 3.16E+05 | 6.72E-10 |
| ALK-1/His | #12 | Q91I, S95I | 0.995 | 3.61E-04 | 3.89E+05 | 9.28E-10 |
| ALK-1/His | #13 | Q91P, S95I | 0.993 | 1.13E-03 | 4.63E+05 | 2.44E-09 |
| ALK-1/His | #14 | Q91M,S95W | 0.990 | 2.15E-04 | 4.00E+05 | 5.37E-10 |
| ALK-1/His | #15 | Q91W, S95V | 0.980 | 8.32E-03 | 4.59E+05 | 1.81E-08 |
| ALK-1/His | #16 | Q91V, S95I | 0.993 | 4.99E-04 | 3.18E+05 | 1.57E-09 |
| ALK-1/His | #17 | Q91G, S95I | 0.990 | 9.07E-04 | 2.85E+05 | 3.18E-09 |
| ALK-1/His | #18 | Q91A, S95W | 0.992 | 1.00E-04 | 3.22E+05 | 3.13E-10 |
| ALK-1/His | #19 | Q91F, S95W | 0.992 | 2.31E-04 | 2.81E+05 | 8.22E-10 |
| ALK-1/His | #23 | Q91F, S95V | 0.993 | 7.87E-04 | 3.87E+05 | 2.03E-09 |
| ALK-1/His | #24 | Q91F, S95I | 0.994 | 1.13E-03 | 3.78E+05 | 2.98E-09 |
| ALK-1/His | #25 | Q91V, S95W | 0.994 | 3.38E-04 | 3.56E+05 | 9.48E-10 |
| ALK-1/His | #26 | Q91V, S95V | 0.991 | 2.47E-04 | 3.30E+05 | 7.48E-10 |
| ALK-1/His | #27 | Q91G, S95W | 0.994 | 2.68E-04 | 3.40E+05 | 7.87E-10 |
| ALK-1/His | #28 | Q91I, S95V | 0.994 | 2.07E-04 | 3.27E+05 | 6.34E-10 |
| ALK-1/His | #29 | Q91P, S95W | 0.992 | 2.40E-03 | 3.40E+05 | 7.05E-09 |
| ALK-1/His | #30 | Q91G, S95V | 0.992 | 4.16E-04 | 3.37E+05 | 1.23E-09 |

Compared with the reference antibody BM, except for the #15 and #29 mutants, the binding affinities (KD values) of the other mutants were improved.

### 1.3 Blocking of Smad1 phosphorylation of ALK1 downstream induced by BMP9

As a ligand of ALK1, BMP9 molecules can bind to the ALK1 protein on the surface of HUVEC cells, activate the ALK1 receptor, and mediate the phosphorylation of the downstream protein Smad1. Therefore, we designed experiments to detect the blocking effect of 10 antibody molecules on BMP9-induced Smad1 phosphorylation.

HUVEC cells were seeded on 96-well plates. 2×10⁴ cells were placed in each well in ECM medium (Sciencell, 1001) containing 5% FBS and ECG, and incubate overnight in the incubator at 37°C, 5% CO₂. Remove the culture medium from the cell plate and wash twice with 200 µl PBS. Add 100 µl of ECM without FBS and ECG, and starve cells for 4 hours. Remove the medium, add 100uL of BM and its mutants which were serially diluted by ECM medium, treat for 1.5 hours, add BMP9 to the medium at a final concentration of 0.5ng/mL and incubate the cells for 45 minutes. The medium was removed, and Smad1 phosphorylation levels in cells were determined using an ELISA kit (Invitrogen, 85-86182-11). After three independent repeated experiments, the results are shown in Table 2 below. The blocking effect of the ALK1 monoclonal antibody mutant with increased affinity is significantly better than that of the wild-type BM antibody reference.

**Table 2 Summary of KD values of binding affinity of ALK-1 antibody to ALK1 antigen and IC50 values of its cellular functional activity**

| Antibody NO. | Mutations | KD (M) (N=2) | IC50 (nM) (N=3) |
|---|---|---|---|
| BM | no | 4.38E-09 | 17.3 |
| #3 | Q91L, S95F | 1.06E-09 | 5.3 |
| #1 | Q91G, S95F | 1.03E-09 | 5.8 |
| #9 | Q91I, S95W | 1.15E-09 | 11.6 |
| #12 | Q91I, S95I | 8.97E-10 | 11.2 |
| #14 | Q91M, S95W | 5.27E-10 | 11.1 |
| #18 | Q91A, S95W | 3.54E-10 | 8.2 |
| #19 | Q91F, S95W | 9.46E-10 | 5.2 |
| #25 | Q91V, S95W | 8.68E-10 | 9.1 |
| #26 | Q91V, S95V | 1.03E-09 | 12.4 |
| #27 | Q91G, S95W | 1.13E-09 | 6.3 |
| #28 | Q91I, S95V | 9.52E-10 | 6.9 |

### 1.4 Preliminary druggability of antibodies:

According to the KD value and the IC50 of blocking Smad1 phosphorylation above, six mutants #3, #1, #9, #14, #18, and #28 were selected for preliminary druggability testing. The developability of the molecule is evaluated by characterizing the druggability-related physical and chemical properties of the candidate mutants (including integrity, hydrophobicity, non-specific adsorption, colloidal stability, and thermal stability).

Purity (CE): Use sodium dodecyl sulfate capillary electrophoresis method (CE-SDS method) to examine the integrity of the candidate mutants. CE detection was performed using a PA800 electrophoresis instrument, and 100µg of the mutant sample was taken, after sample processing, the non/reducing purity test sample is obtained. A PDA detector is used, and the detection window width is 200µm. The judgment criterion is that the principal component of the candidate mutant is greater than 90%.

Thermal stability (DSF): Differential scanning fluorescence method (DSF method) was used to examine the thermal stability of candidate mutants. The detection was performed using a fluorescence quantitative PCR instrument. The mutant samples are stained with the fluorescent chromogen Sypro Orange and added to a 96-well plate for measurement. The sample volume is 10 µL, and 3 duplicate wells are tested each time. The judgment criterion is that the candidate mutant has a developability if its temperature Tm1 value is greater than 60°C.

Hydrophobicity (HIC): Hydrophobic interaction chromatography (HIC) was used to examine the hydrophobicity of candidate mutants. Hydrophobicity detection was performed using an Ultimate 3000 chromatograph and Thermo ProPac HIC-10 chromatographic column. The mobile phase is ammonium sulfate buffer, the flow rate is 1mL/min; the collection time is 30min, the injection volume is 10µL, and the column temperature is 25°C, detection wavelength is 280nm, injector temperature is 10°C. Adalimumab is used as the positive control and Ipilimumab is used as the negative control. The judgment standard is that the shorter the retention time of the mutant sample, the weaker the hydrophobicity. The hydrophobicity of the candidate molecule should be weaker than the negative control substance.

Non-specific adsorption (CIC): Cross-interaction chromatography (CIC method) was used to examine the non-specific adsorption of candidate mutants. Non-specific adsorption detection was performed using Ultimate 3000 and human serum IgG coupled Hitrap-NHS chromatographic column. The mobile phase is PBS, the flow rate is 0.1mL/min, the collection time is 20min, the injection volume is 5µL, the column temperature is 25°C, the detection wavelength is 280nm, and the sample is injected. The device temperature is 10°C. Adalimumab is used as the positive control and Ipilimumab is used as the negative control. The judgment standard is that the shorter the retention time of the mutant sample, the weaker the non-specific adsorption. The non-specific adsorption of the candidate mutant should be weaker than the negative control substance.

Colloidal stability (SMAC): The colloidal stability of candidate mutants were investigated using standup monolayer adsorption chromatography (SMAC method). A ZENIX chromatographic column was used with PBS as the mobile phase, a flow rate of 0.35mL/min, a sampling time of 20min, an injection volume of 10µL, a column temperature of 25°C, a detection wavelength of 214nm. Adalimumab is used as the positive control and Ipilimumab is used as the negative control. The judgment standard is that the shorter the retention time of the mutant sample, the better the colloidal stability. The colloidal stability of the candidate mutant should be weaker than the negative control substance.

The druggability results are shown in Table 3.

**Table 3 Summary of druggability results of ALK-1 antibody mutants**

| Antibody mutants NO. | nrCE-SDS (%) | | | rCE-SDS (%) | | | DSF | HIC (min) | CIC (min) | SMAC (min) |
|---|---|---|---|---|---|---|---|---|---|---|
| | Mono mer | Fragment with Low molecular weight | Fragme nt with high | Light chain | Heavy chain | Frag ment | Tm1 (°C) | | | |
| | | | molecu lar weight | | | | | | | |
| #3 | 99.4 | 0.6 | 0.0 | 20.6 | 79.0 | 0.4 | 69.9 | 16.0 8 | 9.54 | 8.11 |
| #1 | 99.1 | 0.9 | 0.0 | 19.3 | 80.4 | 0.3 | 70.2 | 16.2 9 | 9.77 | 8.65 |
| #9 | 99.2 | 0.8 | 0.0 | 20.1 | 79.6 | 0.3 | 70.3 | 15.9 5 | 9.47 | 8.05 |
| #14 | 99.1 | 0.9 | 0.0 | 20.1 | 79.7 | 0.2 | 70.5 | 16.1 0 | 9.51 | 8.08 |
| #18 | 99.3 | 0.7 | 0.0 | 20.3 | 78.9 | 0.8 | 70.2 | 16.4 2 | 9.59 | 8.28 |
| #28 | 99.1 | 0.9 | 0.0 | 18.9 | 80.1 | 1.0 | 70.0 | 15.6 3 | 9.54 | 8.09 |
| Adalimu mab (Positive control) | NA | NA | NA | NA | NA | NA | NA | 13.8 6 | 9.66 | 7.98 |
| Ipilimuma b (Negative control) | NA | NA | NA | NA | NA | NA | NA | 20.3 9 | 11.2 7 | 12.06 |

Among them, rCE-SDS refers to the reduced capillary electrophoresis; nrCE-SDS refers to the non-reduced capillary electrophoresis; Fragment with low molecular weight: protein peptides with a molecular weight smaller than the target protein. Fragment with high molecular weight: protein aggregates with a molecular weight greater than that of the target protein.

The above characterization results show:
(1) The CE purity of the six candidate mutants is better, indicating that the purity of the mutants is higher;
(2) The HIC retention times of the six candidate mutants are all earlier than the negative control, indicating that the mutants are less hydrophobic;
(3) The CIC retention times of the six candidate mutants are all earlier than the negative control, indicating that the mutants have better non-specific adsorption;
(4) The SMAC retention times of the six candidate mutants are all earlier than those of the negative control, indicating that the mutants have better colloidal stability;
(5) The Tm1 values of the six candidate mutants are all higher than 60°C, indicating that the mutants have better thermal stability.

Among the six candidate mutants, preliminary characterization results show that mutants #3, #1, #9, #14, #18, and #28 have better physical and chemical properties. The six candidate mutants have higher purity, weaker hydrophobicity, weaker non-specific adsorption, better colloidal stability, and better thermal stability.

### 2. Construction of anti-ALK-1-anti-VEGF bispecific antibody (BEV813 bsAb)

Based on the judgment of antibody affinity (KD value), cell biological function (IC50 value) and preliminary druggability analysis results, the #18 ALK-1 monoclonal antibody mutant sequence was selected as the basis for further improvement as the source of ScFv structure in the ALK1-VEGF bispecific antibody BEV813.

### 2.1 Components of BEV813 bsAb

BEV813 is a human IgG1 bispecific antibody with a typical "2+2" symmetric structure. It can be obtained by combining the heavy chain variable region (VH) and light chain variable region(VL) of the ALK-1 antibody (#18 ALK-1 monoclonal antibody) in the form of ScFv (Single-chain variable fragment) fused to the "C" end of the constant region of the VEGF-A monoclonal antibody (Bevacizumab) (as shown in Figure 2).

In order to enhance the stability of ScFv, Cys point mutation was introduced into the VH and VL of the ALK-1 binding region of mutant #18 to form disulfide bonds and enhance stability of ScFv structure.

As can be seen from Figure 2, BEV813 consists of 4 peptide chains, 2 identical first chains and 2 identical second chains. The first chain sequentially includes the VH of the VEGF binding region, the CH of the VEGF binding region, Linker, the VH of the ALK-1 binding region, Linker, and the VL of the ALK-1 binding region from the N-terminus to the C-terminus. The second chain sequentially includes VL and CL of the VEGF binding region from N-terminus to C-terminus. The four peptide chains can be connected through disulfide bonds.

The sequence information of BEV813 is as follows:
The VEGF binding region of BEV813 contains the following sequence:
Heavy chain variable region (VH) 


 (SEQ ID NO: 57)
3 CDRs of VH:
   CDR1H: GYTFTNYG (SEQ ID NO: 58)
   CDR2H: INTYTGEP (SEQ ID NO: 59)
   CDR3H: AKYPHYYGSSHWYFDV (SEQ ID NO: 60)
Light chain variable region (VL) 

 (SEQ ID NO: 61)
3 CDRs of VL:
   CDR1L: QDISNY (SEQ ID NO: 62)
   CDR2L: FTS (SEQ ID NO: 63)
   CDR3L: QQYSTVPWT (SEQ ID NO: 64)

The CH of the VEGF binding region, that is, the heavy chain constant region (CH) is shown in SEQ ID NO: 9. The CL of the VEGF binding region, that is, the light chain constant region (CL) is shown in SEQ ID NO: 10. The Linker sequence is shown in SEQ ID NO: 65, that is GGGGSGGGGSGGGGSGGGGS. Linker is located between the VEGF binding region and the ALK-1 binding region.

The ALK-1 binding region of BEV813 is in the form of scFv, which contains the following sequence:
Light chain variable region (VL) 
EIVLTQSPGTLSLSPGERATLSCRASQSVSSSYLAWYQQKPGQAPRLLIYGTSSRAT
GIPDRFSGSGSGTDFTLTISRLEPEDFAVYYCQAYGSWPITFGCGTRLEIK (SEQ ID NO:
66)
Heavy chain variable region (VH)

QVQLQESGPGLVKPSQTLSLTCTVSGGSISSGEYYWNWIRQHPGKCLEWIGYIYYS
GSTYYNPSLKSRVTISVDTSKNQFSLKLSSVTAADTAVYYCARESVAGFDYWGQGTLVT
VSS (SEQ ID NO: 67)

The Linker sequence is shown in SEQ ID NO: 65. Linker is located between VH and VL of scFv.
BEV813 first strand sequence: 
EVQLVESGGGLVQPGGSLRLSCAASGYTFTNYGMNWVRQAPGKGLEWVGWINTY
TGEPTYAADFKRRFTFSLDTSKSTAYLQMNSLRAEDTAVYYCAKYPHYYGSSHWYFDV
WGQGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKTH
TCPPCPAPEAAGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKG
QPREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGGGGGSGGGGSGG
GGSGGGGSQVQLQESGPGLVKPSQTLSLTCTVSGGSISSGEYYWNWIRQHPGKCLEWIG
YIYYSGSTYYNPSLKSRVTISVDTSKNQFSLKLSSVTAADTAVYYCARESVAGFDYWGQ
GTLVTVSSGGGGSGGGGSGGGGSGGGGSEIVLTQSPGTLSLSPGERATLSCRASQSVSSS
YLAWYQQKPGQAPRLLIYGTSSRATGIPDRFSGSGSGTDFTLTISRLEPEDFAVYYCQAY
GSWPITFGCGTRLEIK(SEQ ID NO: 68)
BEV813 second strand sequence:

DIQMTQSPSSLSASVGDRVTITCSASQDISNYLNWYQQKPGKAPKVLIYFTSSLHSG
VPSRFSGSGSGTDFTLTISSLQPEDFATYYCQQYSTVPWTFGQGTKVEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 69)

### 2.2 Preparation and purification of BEV813 bsAb

For the preparation process of BEV813 bsAb, please refer to the "Construction, expression and purification of anti-ALK-1 monoclonal antibodies and mutants" section.

### 2.3 in vitro assay of BEV813 bsAb

### 2.3.1 Affinity of BEV813 bsAb binding to ALK-1 protein and VEGFA protein

The affinity assay by ForteBio (Octet RED96e) was performed according to existing methods (Estep, P et al., Solution-based measurement of high-throughput antibody-antigen affinity and epitope ranking, MAbs, 2013.5(2): p.270-8). The sensor was equilibrated offline in the assay buffer for 10 minutes, then detected online for 60 seconds to establish a baseline. Dilute BEV813, bevacizumab, #18 mutant or BM to 5ug/mL using loading buffer (1×PBS, pH7.4, with 0.02% Tween-20 (10nM Na₂HPO₄.12H₂O, 2mM KH₂PO₄, 137mM NaCl, 2.7mM KCl, 0.02% Tween-20, pH7.4)); The AHC sensor (Fortebio, 18-5060) were coated by these antibodies for 120 seconds; Then dilute the antigen protein with loading buffer (same as above) (starting from 200nM, set up 7 concentration gradients); then the sensor conjugated with antibody was placed in a sample buffer with different concentration gradients of antigen for 60 seconds, and then transfer the sensor to dissociate in PBS for 300 s; then wash 3 times in regeneration buffer and neutralization buffer successively for 5 s in each solution each time. Finally, a 1:1 binding model was used for kinetic analysis.

**Table 1. Comparison of binding kinetic parameters of BEV813 bsAb, bevacizumab binding to VEGF antigen**

| Kinetic parameters of the binding of bispecific antibody BEV813, and the control antibody bevacizumab to VEGF165 | | | | |
|---|---|---|---|---|
| Sample ID | KD (M) | k*ₒₙ* (1/Ms) | k*_{off}*(1/s) | Full R^2 |
| bevacizumab | 2.911E-10 | 1.577E+05 | 4.590E-05 | 0.9966 |
| BEV813 | 7.244E-10 | 2.089E+05 | 1.513E-04 | 0.9942 |

**Table 2. Comparison of binding kinetic parameters of BEV813 bsAb, ALK1 mAbs binding to ALK1 antigen**

| Kinetic parameters of the binding of bispecific antibody BEV813, monoclonal antibody ALK-1 (#18mutant) and the control antibody BM to ALK-1-his | | | | |
|---|---|---|---|---|
| Sample ID | KD (M) | kon (1/Ms) | koff (1/s) | Full R^2 |
| BM | 1.386E-09 | 5.981E05 | 8.289E-04 | 0.9963 |
| #18 | 6.995E-10 | 4.759E05 | 3.329E-04 | 0.9914 |
| BEV813 | 6.886E-10 | 4.861E05 | 3.347E-04 | 0.9954 |

The results show that the affinity of BEV813 to VEGF-A antigen is slightly weaker than that of bevacizumab; As for ALK-1 antigen, the affinity of BEV813 is equivalent to that of ALK-1 monoclonal antibody (#18 mutant), but better than BM.

### 2.3.2 Binding activity of BEV813 bsAb to CHO-K1-hALK-1 cells

1x10⁵ cells/ml of CHO-K1-hALK-1 cells (provided by Stainwei Biotech Inc) were seeded in a 96-well plate, then add different antibodies with different concentrations: BEV813, ALK-1 mab (#18 mutant), bevacizumab (Biointron, B7424), human IgG (Beyotime, A7001). Incubate at 4°C for 1 hour. Then add VEGF 165-biotin (10µg/mL) (sino biological, 11066-HNAB-B) and APC-anti-human IgG mAb (Biolegend,410708) successively, incubate at 4°C for 1 hour. Collect the cells and analyze the binding of the test antibody to CHO-K1-hALK-1 cells by flow cytometry.

The results are shown in Figure 3. Compared with ALK-1 mAb (#18 mutant), BEV813 has higher binding affinity to CHO-K1-hALK1 cells.

### 2.3.3 BEV813 bsAb competes with ligand BMP9 for binding to CHO-K1-hALK-1 cells

1.0×10⁵ CHO-K1-hALK1 cells (provided by Stainwei Biotech Inc) were seeded in a 96-well plate, and then add gradient dilution of BMP9(Acro, GD2-H5211 and BEV813(initial concentration is 50nM). Incubate at 4°C for 1 hour. After washing three times with PBS solution containing 2% FBS, 100uL VEGF 165-biotin (sino biological, 11066-HNAB-B) is added and incubated at 4°C for 1 hour. After washing three times with PBS solution containing 2% FBS, 100uL of Streptavidin-APC (Biolegend, 405207) was added and incubated at 4°C for 1 hour. The cells were collected and then analyzed BEV813 competition with BMP9 for binding to CHO-K1-hALK1 cells by flow cytometry

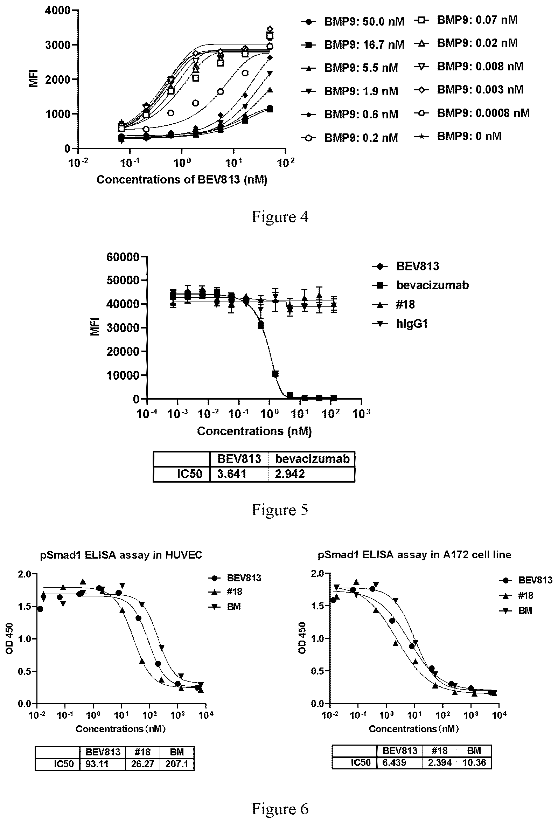

The results are shown in Figure 4. As the concentration of BMP9 increases, the binding affinity of BEV813 to CHO-K1-ALK1 cells decreases. This proves that the BEV813 competes with BMP9 for binding epitopes that bind to CHO-K1-hALK1 cells.

### 2.3.4. Blocking VEGF induced signaling activity of BEV813 bsAb

2.5×10⁴ H_VEGF Reporter 293 cells (Genomeditech(Shanghai) Co.LTD, GM-C09057) were seeded in a 96-well plate and cultured overnight. Different gradient dilution of antibodies were added: BEV813, ALK-1 mab (#18 mutant), bevacizumab (Biointron, B7424), human IgG (Beyotime, A7001), incubate at 4°C for 1 hour. Then add 1 nM VEGF165 protein (Sino, 11066-HNAH) and incubate at 37°C for 6 hours. After adding 100ul/well of cell lysis solution, measure the fluorescence signal using the luciferase kit.

The experimental results are shown in Figure 5. As the antibody concentration increases, BEV813 bsAb can block VEGF binding to H_VEGF Reporter 293 cells, and block the transmission of signals into the cells. Based on the IC50 value, the blocking effect of BEV813 bsAb is comparable to that of bevacizumab.

### 2.3.5 BEV813 bsAb blocks BMP9-induced phosphorylation of Smad1

As a ligand of ALK1, BMP9 molecules can activate the ALK1 receptor by binding to the ALK1 protein on the surface of HUVEC cells or A172 cells, thereby mediating the phosphorylation of the downstream protein Smad1. Therefore, we designed experiments to detect the blocking effect of BEV813 on BMP9-induced Smad1 phosphorylation.

2×10⁴ cells per well of HUVEC or A172 cells were seeded on 96-well plates. The cells were cultured in ECM medium (Sciencell, 1001) containing 5% FBS and ECG or DMEM medium (Gibco, 11995065) containing 10% FBS in the incubator with 37°C, 5% CO₂ overnight. Remove the culture medium from the plate and wash twice with 200 µl PBS. Add 100 µl of ECM without FBS and ECG or DMEM without FBS, and then starve the cells for 1 hour. Remove the culture medium from the plate, add 100uL of the test product with gradient dilution, and treat for 3 hours. Then add BMP9 at a final concentration of 0.15ng/mL or 0.3ng/mL to the culture medium, treat the cells for 45 minutes. The medium was removed and Smad1 phosphorylation levels were determined using an ELISA kit (Invitrogen, 85-86182-11).

The results are shown in Figure 6. The ALK1 monoclonal antibody (#18 mutant) molecule after affinity maturation has the strongest blocking effect, but the BEV813 also shows good blocking effect.

### 2.3.6 BEV813 bsAb inhibits HUVEC proliferation

HUVEC cells were seeded on 96-well plates, 5×10³ per well cells were cultured in M199 medium (Source Culture, 1001) containing 10% FBS. Different concentration gradients of the antibody containing 10ng/mL VEGF165 protein (Sino biological, 11066-HNAB-B) were added to the above cells incubated for 72 hours. The culture medium was removed and treated with Cell Titer Glo Kits detection kit (Promega, G755B), and the fluorescence value was read on a microplate reader.

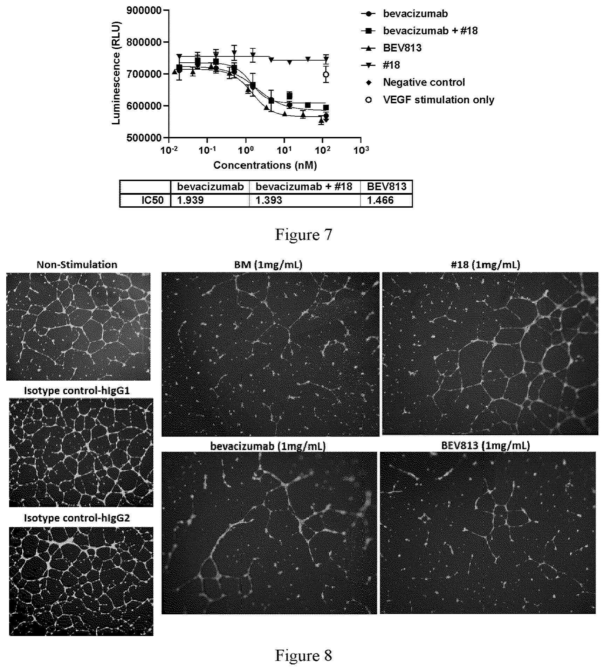

The blocking effect of HUVEC proliferation by BEV813 is shown in Figure 7. The results showed that the blocking effect of BEV813 on HUVEC proliferation was comparable to that of Bevacizumab.

### 2.3.7 BEV813 bsAb inhibits HUVEC microtubule formation

200 µL of Matrigel (Corning: 354234, Caifornia, USA) was added to the 24-well plate and solidified at 37°C for 30 minutes. HUVEC cells (Human Umbilical Vein Endothelial Cells) were incubated with 2 µg/ml calcein AM for 30 min and washed with PBS. 4×10⁴ HUVEC cells were added to a 24-well plate, and the cells were treated with 1 mg/ml of the antibody to be tested. After incubation at 37°C for 48 hours, the formation of microtubules was observed using a fluorescence microscope (Olympus CKX53, Tokyo, Japan). Among them, anti-HEL-Human IgG1 (hIgG1, Baiying Bio, Cat. No. B117901) and anti-HEL-Human IgG2 (hIgG2, Baiying Bio, Cat. No. B107803) as isotype control, non-stimulation group (Non-stimulation) as negative control; The effects of BM, #18 mutant, bevacizumab and BEV813 on the formation of microtubules in HUVEC cells were tested at the same concentration (1mg/ml).

The results are shown in Figure 8. Adding equal amounts of BEV813, ALK-1 monoclonal antibody (#18 mutant) and Bevacizumab monoclonal antibody to HUVEC cells can well inhibit the formation of microtubules. Moreover, the BEV813 showed a stronger blocking effect on inhibiting microtubule formation in HUVEC cells.

### 2.4 In vivo efficacy of BEV813 bsAb

### 2.4.1 Human immune system-reconstructed CDX mouse model

### (A) MDA-MB-231 breast cancer subcutaneous xenograft tumor model with PBMC-based humanized immune system

MDA-MB-231 (ATCC No. HTB-26) tumor cells were inoculated subcutaneously into tumor donor mice (NCG mice, female, provided by Jiangsu Jicui Yaokang Biotechnology Co., Ltd.). After the tumors grew, they were taken out and their volumes were between about 500~1000mm³. The tumors were cut into small pieces of approximately 2 mm×2 mm×2 mm, and inoculated subcutaneously on the right flank of the experimental mice with a trocar. Each mouse was inoculated with a piece of tumor tissue. Three days after tumor inoculation, 2 × 10⁶ cells/mouse of healthy adult PBMC (human peripheral blood mononuclear cells, Donor#: SC12291, provided by Shanghai Xuanfeng Biotechnology Co., Ltd.) were resuspended in PBS and inoculated into mice. On the day before grouping and the day at the end of the experiment, FACS was used to detect the proportion of human CD45-positive cells in the blood of mice. Animals were treated with Bevacizumab, ALK-1 mAb (#18 mutant) and BEV813 bsAb or saline (as the Vehicle group). The dose of Bevacizumab and ALK-1 mAb (#18 mutant) monoclonal antibodies was 5 mg/kg, and the dose of BEV813 bsAb was 6.7 mg/kg. Group dosing was initiated when the tumor size reached 53 mm³. Each antibody was administered intraperitoneally three times a week in a 100-uL volume each time. Each group consisted of 8 mice. Tumor volume and mouse body weight were measured twice weekly. After 3 weeks of administration, the animals were euthanized and the tumors were removed and weighed. One-Way ANOVA test was used to perform statistical analysis between groups on tumor volume and tumor weight, and p<0.05 was considered to have significant differences.

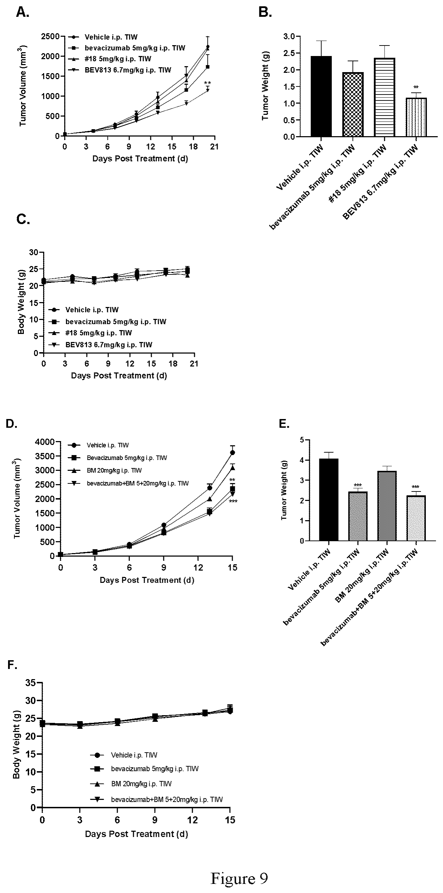

The experimental results are shown in Figure 9. 20 days after group administration, the tumor growth inhibition rate (TGI_{TV}%) of Bevacizumab(5 mg/kg), ALK-1 mAb (#18 mutant)(5 mg/kg), and BEV813 bsAb(6.7 mg/kg) were 22%, 0%, and 48% respectively (A in Figure 9); the tumor weight inhibition rate (TGI_{TW}) of tumor weight were 20%, 2%, and 51% respectively (B in Figure 9). ALK-1 mAb (#18 mutant)(cannot inhibit tumor growth at a dose of 5 mg/kg; Bevacizumab has a certain inhibitory effect on tumor growth at a dose of 5 mg/kg; while BEV813 bsAb with an equimolar ( 6.7 mg/kg) had a significant inhibitory effect on tumor growth, and the tumor volume and weight were significantly smaller than the control group (A and B in Figure 9,**p<0.01). During the treatment period, the body weight of mice in each group did not change significantly, indicating that the drug was safe (C in Figure 9).

In another experiment of the same model, 15 days after group administration, the tumor growth inhibition rate (TGI _{TV}%) of the tumor growth of the BM (#18, 20mg/kg), Bevacizumab (5mg/kg) and bevacizumab +BM (5+20mg/kg) group were 13%, 34% and 41% respectively (D in Figure 9); the tumor weight inhibition rate (TGI _{Tw}%) of tumor weight were 15%, 40% and 45% respectively (E in Figure 9). ALK1 mAb (BM) at a dose of 20 mg/kg has almost no inhibitory effect on tumors; while Bevacizumab at a dose of 5 mg/kg or in combination with BM both have a certain growth inhibitory effect on tumor. The tumor volume and the weight was significantly smaller than that of the control group (D and E in Figure 9, ***p<0.001). However, no statistical difference was seen between the bevacizumab group and the combination group. During the treatment period, there was no significant change in the body weight of mice in each group, indicating that the drug was safe (F in Figure 9).

By comparing A and D, as well as B and E in Figure 9, it can be concluded that BEV813 bsAb has better efficacy in inhibiting tumor growth than the combination of ALK1 mAb and bevacizumab, and BEV813bsAb has good safety profile.

### (B) PBMC humanized xenograft esophageal cancer KYSE450 model

KYSE450 tumor cells (JCRB ed. No. JCRB1430) washed with PBS and resuspended in a mixture with PBS:Matrigel (1:1 volume ratio) at a concentration of 1×10⁸/mL and subcutaneously inoculated into the right flank of tumor donor mice (NCG mice, male, provided by Jiangsu Jicui Yaokang Biotechnology Co., Ltd.). Four days after tumor inoculation, healthy adult PBMC (human peripheral blood mononuclear cells, Donor#: PAZ012T01, provided by Shanghai Heyousheng Biotechnology Co., Ltd.) were resuspended in PBS and inoculated into the mice at a dose of 2x10⁶ cells per mouse. One day before grouping and at the end of the experiment, the proportion of human CD45-positive cells in mouse blood was detected by FACS. Animals were treated with Bevacizumab, BEV813 bsAb, or saline. Bevacizumab was administered at a dose of 5 mg/kg, and BEV813 bsAb was administered at a dose of 6.7 mg/kg. When the mean tumor size reached 89 mm³, the drugs were administered in groups. Each antibody was intraperitoneally administered 3 times a week at a volume of 100 µL per administration. Each group consisted of 8 mice. Tumor volume and mouse body weight were measured twice weekly. After 4 weeks of drug administration, the animals were euthanized, and then the tumors were excised and weighed. One-Way ANOVA was applied to statistically analyze tumor volume and tumor weight between groups, with p < 0.05 considered statistically significant.

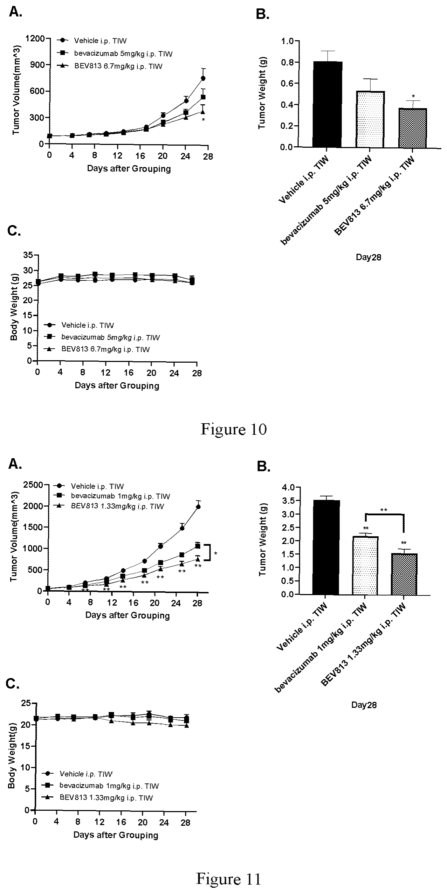

The results are shown in Figure 10. 28 days after group administration, the tumor growth inhibition rate (TGI_{TV}%) of the Bevacizumab (5mg/kg) group and the BEV813 bsAb (6.7mg/kg) were 28% and 52% respectively (A in Figure 10); tumor weight inhibition rate (TGI_{TW}%) were 34% and 53% respectively (B in Figure 10). Bevacizumab has a certain inhibitory effect on tumor growth at 5 mg/dose; while BEV813bsAb (6.7 mg/kg), with an equimolar has a significant inhibitory effect on tumor growth, the tumor volume and weight are significantly smaller than the control group (Figure A and B in 10 *p<0.05). During the treatment period, the body weight of mice in each group did not change significantly, indicating that the drug was safe (C in Figure 10).

### (C) human non-small cell lung tumor model of Subcutaneous transplantation of HCC827 cell line in CD34+ humanized mice

5×10⁶cells/mL of HCC827(Pengli Biomedical Technology (Shanghai) Co., Ltd.) were washed with PBS and resuspended in a mixture with PBS:Matrigel (volume ratio 1:1), inoculated into humanized mice (Pengli Biotech (Shanghai) Co., Ltd.) under the skin on the right side of the back near the armpit. Animals were treated with Bevacizumab, BEV813 bsAb, or saline. Bevacizumab was administered at a dose of 1 mg/kg, and BEV813 bsAb was administered at a dose of 1.33 mg/kg. When the mean tumor size reached 50-80 mm³, the drugs were administered in groups. Each antibody was administered intraperitoneally three times a week at a volume of 100uL. Each group consisted of 6 mice. Tumor volume and mouse body weight were measured twice weekly. Four weeks after dosing, animals were euthanized and tumors were removed and weighed. One-Way ANOVA was applied to statistically analyze tumor volume and tumor weight between groups, with p < 0.05 considered statistically significant.

The results are shown in Figure 11. 28 days after group administration, the tumor growth inhibition rate (TGI_{TV}%) of the Bevacizumab (1 mg/kg) group and the BEV813 bsAb (1.33 mg/kg) group were 46% and 61% respectively (A in Figure 11); tumor weight inhibition rate (TGI_{TW} %) were 38% and 56% respectively (B in Figure 11). Compared with the control group at the same period, the tumor volume decreased significantly from Day 7 to Day 28 (A, Figure 11, **p<0.01); tumor weight decreased significantly on Day 28 (B in Figure 11, **p<0.01). This shows that BEV813 bsAb (1.33 mg/kg) has a more significant inhibitory effect on tumor growth than Bevacizumab (1 mg/kg) with an equimolar (A and B in Figure 11, * p<0.05, **p<0.01). During the treatment period, the body weight of mice in each group did not change significantly, indicating that the drug was safe (C in Figure 11).

### 2.4.2 PDTX mouse model of intestinal cancer liver metastasis

Tumor tissue (2×2×2mm³) from patients with intestinal cancer liver metastasis was inoculated subcutaneously into the right flank of tumor donor mice (NCG mice, female, provided by Jiangsu Jicui Yaokang Biotechnology Co., Ltd.). Animals were treated with Bevacizumab, BEV813 bsAb, or saline. Bevacizumab was administered at a dose of 5 mg/kg, and BEV813 bsAb was administered at a dose of 6.7 mg/kg. When the mean tumor size reached 60-100 mm³, the drugs were administered in groups. Each antibody was administered intraperitoneally three times a week at a volume of 100 µL. Each group consisted of 6 mice. Tumor volume and body weight were measured twice weekly. Four weeks after dosing, animals were euthanized and tumors were removed and weighed.

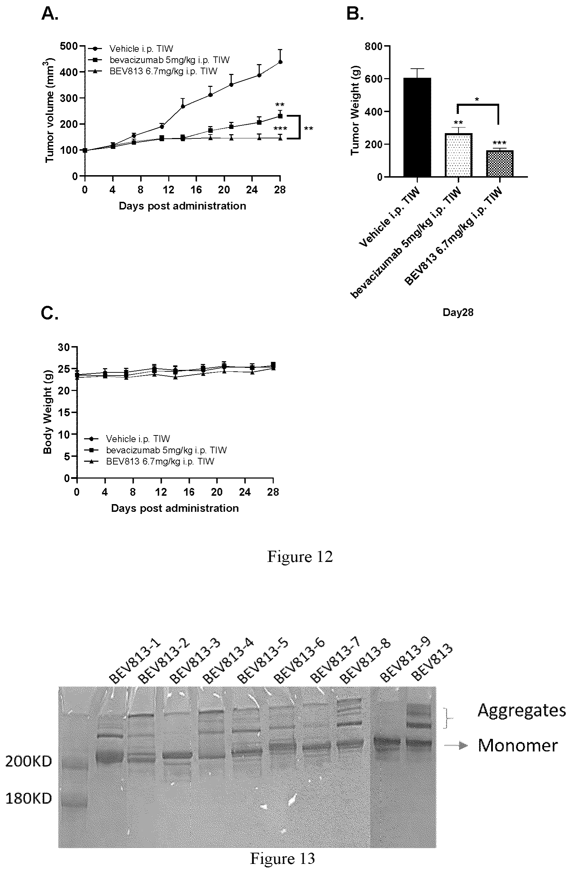

The results are shown in Figure 12. On days 28 after grouping, the tumor growth inhibition rate (TGI_{TV}%) of the Bevacizumab (5mg/kg) group and the BEV813 bsAb (6.7mg/kg) were 61.2% and 85.9% respectively (A in Figure 12); tumor weight inhibition rate (TGI_{TW}%) were 56% and 73% respectively (B in Figure 12). Both Bevacizumab and BEV813 had a significant inhibitory effect on tumor growth, and the tumor volume was significantly smaller than that of the control group (A, Figure 12, **p<0.01, ***p<0.001); tumor weights were also significantly smaller than those of the control group (B, Figure 12, **p<0.01, ***p<0.001). At the same time, equal molar amounts of BEV813 have better and more obvious tumor inhibitory effects than Bevacizumab, and there is a significant statistical difference (A and B in Figure 12, *p<0.05, **p<0.01). Besides, the drug has no obvious toxic side effects on animals and has good safety (C in Figure 12).

### 3. Construction of anti-ALK-1-anti-VEGF bispecific antibody (BEV813 bsAb) mutant

In order to solve the problem of the high proportion of isomers and their aggregates (polymers) of BEV813 bsAb during preparation, the ALK-1 sequence of BEV813 bsAb was mutated, and the sequence information of the obtained mutant and its mutated ALK-1 binding region is as follows (other regions are the same as BEV813 bsAb):
1.BEV813-1:
   The light chain variable region (VH) of the ALK-1 binding region: 
QVQLQESGPGLVKPSQTLSLTCTVSGGSISSGEYYWNWIRQHPGCGLEWIGYIYYS
GSTYYNPSLKSRVTISVDTSKNQFSLKLSSVTAADTAVYYCARESVAGFDYWGQGTLVT
VSS (SEQ ID NO:70)
   The light chain variable region (VL) of the ALK-1 binding region: SEQ ID NO: 66
2.BEV813-2:
   The light chain variable region (VH) of the ALK-1 binding region: SEQ ID NO: 67
   The light chain variable region (VL) of the ALK-1 binding region: 
EIVLTQSPGTLSLSPGERATLSCRASQSVSSSYLAWYQQKPGQAPRLLIYGTSSRATGIPD
RFSGSGSGTDFTLTISRLEPEDFAVYYCQAYGSWPITFCQGTRLEIK (SEQ ID NO:71)
3.BEV813-3:
   The light chain variable region (VH) of the ALK-1 binding region: SEQ ID NO: 67
   The light chain variable region (VL) of the ALK-1 binding region: 
EIVLTQSPGTLSLSPGERATLSCRASQSVSSSYLAWYQQKPGQAPRLLIYGTSSRATGIPD
RFSGSGSGTDFTLTISRLEPEDFAVYYCQAYGSWPITFGQCTRLEIK (SEQ ID NO:72)
4.BEV813-4:
   The light chain variable region (VH) of the ALK-1 binding region: 
QVQLQESGPGLVKPSQTLSLTCTVSGGSISSGEYYWNWIRQHPGKGCEWIGYIYYS
GSTYYNPSLKSRVTISVDTSKNQFSLKLSSVTAADTAVYYCARESVAGFDYWGQGTLVT
VSS (SEQ ID NO:73)
   The light chain variable region (VL) of the ALK-1 binding region: 
EIVLTQSPGTLSLSPGERATLSCRASQSVSSSYLAWYQQKPGQAPRLLIYGTSSRATGIPD
RFSGSGSGTDFTLTISRLEPEDFAVYYCQAYGSWPITCGQGTRLEIK (SEQ ID NO:74)
5.BEV813-5:
   The light chain variable region (VH) of the ALK-1 binding region: 
QVQLQESGPGLVKPSQTLSLTCTVSGGSISSGEYYWNWIRQHPGKGLEWIGYIYYS
GSTYYNPSLKSRVTISVDTSKNQFSLKLSSVTAADTAVYYCARESVAGFDYWGCGTLVT
VSS (SEQ ID NO:75)
   The light chain variable region (VL) of the ALK-1 binding region: 
EIVLTQSPGTLSLSPGERATLSCRASQSVSSSYLAWYQQKPGQCPRLLIYGTSSRATGIPD
RFSGSGSGTDFTLTISRLEPEDFAVYYCQAYGSWPITFGQGTRLEIK (SEQ ID NO:76)
6.BEV813-6:
   The light chain variable region (VH) of the ALK-1 binding region: SEQ ID NO: 75
   The light chain variable region (VL) of the ALK-1 binding region: 
EIVLTQSPGTLSLSPGERATLSCRASQSVSSSYLAWYQQKPGCAPRLLIYGTSSRATGIPD
RFSGSGSGTDFTLTISRLEPEDFAVYYCQAYGSWPITFGQGTRLEIK (SEQ ID NO:77)
7.BEV813-7:
   The light chain variable region (VH) of the ALK-1 binding region: 
QVQLQESGPGLVKPSQTLSLTCTVSGGSISSGEYYWNWIRQHPGKGLEWIGYIYYS
GSTYYNPSLKSRVTISVDTSKNQFSLKLSSVTAADTAVYYCARESVAGFCYWGQGTLVT
VSS (SEQ ID NO:78)
   The light chain variable region (VL) of the ALK-1 binding region: 
EIVLTQSPGTLSLSPGERATLSCRASQSVSSSYLAWYQQKPGQAPRCLIYGTSSRATGIPD
RFSGSGSGTDFTLTISRLEPEDFAVYYCQAYGSWPITFGQGTRLEIK (SEQ ID NO:79)
8.BEV813-8:
   The light chain variable region (VH) of the ALK-1 binding region: 
QVQLQESGPGLVKPSQTLSLTCTVSGGSISSGEYYWNWIRQHPGKGLEWIGYIYYS
GSTYYNPSLKSRVTISVDTSKNQFSLKLSSVTAADTAVYYCARESVAGCDYWGQGTLV
TVSS (SEQ ID NO:80)
   The light chain variable region (VL) of the ALK-1 binding region: SEQ ID NO: 79
9.BEV813-9:
   The light chain variable region (VH) of the ALK-1 binding region: 
QVQLQESGPGLVKPSQTLSLTCTVSGGSISSGEYYWNWIRQHPGKGLEWIGYIYYS
GSTYYNPSLKSRVTISVDTSKNQFSLKLSSVTAADTAVYYCARESVAGFDYCGQGTLVT
VSS (SEQ ID NO:81)
   The light chain variable region (VL) of the ALK-1 binding region: SEQ ID NO: 76
      BEV813-9 first chain: 
EVQLVESGGGLVQPGGSLRLSCAASGYTFTNYGMNWVRQAPGKGLEWVGWINTY
TGEPTYAADFKRRFTFSLDTSKSTAYLQMNSLRAEDTAVYYCAKYPHYYGSSHWYFDV
WGQGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKTH
TCPPCPAPEAAGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKG
QPREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGGGGGSGGGGSGG
GGSGGGGSQVQLQESGPGLVKPSQTLSLTCTVSGGSISSGEYYWNWIRQHPGKGLEWIG
YIYYSGSTYYNPSLKSRVTISVDTSKNQFSLKLSSVTAADTAVYYCARESVAGFDYCGQ
GTLVTVSSGGGGSGGGGSGGGGSGGGGSEIVLTQSPGTLSLSPGERATLSCRASQSVSSS
YLAWYQQKPGQCPRLLIYGTSSRATGIPDRFSGSGSGTDFTLTISRLEPEDFAVYYCQAY
GSWPITFGQGTRLEIK(SEQ ID NO:82)
      BEV813-9 second chain:

DIQMTQSPSSLSASVGDRVTITCSASQDISNYLNWYQQKPGKAPKVLIYFTSSLHSG
VPSRFSGSGSGTDFTLTISSLQPEDFATYYCQQYSTVPWTFGQGTKVEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO:69)

The above mutant numbers and their light and heavy chain sequence information are summarized in Table 6 below:

**Table 6. BEV813 bsAb mutant numbers and their light and heavy chain sequence information**

| BEV813 bsAb mutant NO. | ScFv VH sequence NO. | linker | ScFv VL sequence NO. |
|---|---|---|---|
| BEV813-1 | SEQ ID NO:70 | (G4S)4 | SEQ ID NO:66 |
| BEV813-2 | SEQ ID NO:67 | (G4S)4 | SEQ ID NO:71 |
| BEV813-3 | SEQ ID NO:67 | (G4S)4 | SEQ ID NO:72 |
| BEV813-4 | SEQ ID NO:73 | (G4S)4 | SEQ ID NO:74 |
| BEV813-5 | SEQ ID NO:75 | (G4S)4 | SEQ ID NO:76 |
| BEV813-6 | SEQ ID NO:75 | (G4S)4 | SEQ ID NO:77 |
| BEV813-7 | SEQ ID NO:78 | (G4S)4 | SEQ ID NO:79 |
| BEV813-8 | SEQ ID NO:80 | (G4S)4 | SEQ ID NO:79 |
| BEV813-9 | SEQ ID NO:81 | (G4S)4 | SEQ ID NO:76 |

### 3.1 Preparation and purification of BEV813 bsAb mutants

3.1.1 The preparation process of BEV813 bsAb mutants is the same as the construction, expression and purification of anti-ALK-1 monoclonal antibodies and mutants mentioned above.

3.1.2 Sodium dodecyl sulfate polyacrylamide gel electrophoresis (SDS-PAGE) to detect purity

Ides protease (Rhinogen, QIP-001-A) is an immunoglobulin-degrading enzyme that cleaves IgG only at a specific site below the hinge region to produce F(ab')₂ and single-chain Fc (sFc). The presence of the antibody after enzyme digestion can be effectively determined by SDS-PAGE or mass spectrometry.

Digest 2µg of BEV813 bsAb and its mutant samples with Ides enzyme (expressed in E. coli), then add non-reducing loading buffer. Incubate the samples in a metal bath (Mini Dry Bath, GL-120A) at 100°C for 10 minutes, followed by SDS-PAGE analysis (Tanon, EPS600).

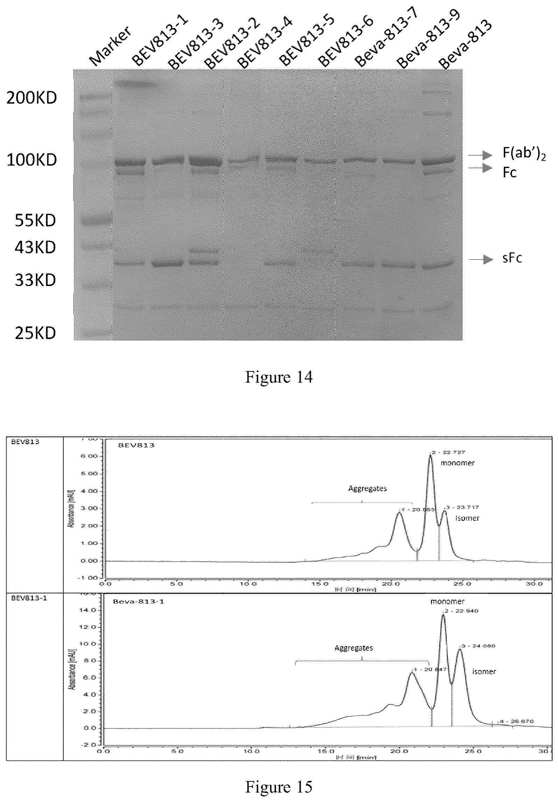

The results are shown in Figures 13 and 14. Figure 13 shows the purity of different mutants. BEV813-9 has no aggregates, BEV813-3 and BEV813-7 have a small amount of aggregates, and the rest have varying degrees of aggregates. Figure 14 shows the bands of different mutants digested by Ides. If isomers are present, the Fc band will be displayed. If no isomers are present, the sFc band will be visible and there will be no Fc band. After digestion by Ides, BEV813-9 has no isomers, while the rest have isomers.

The concept of isomers: The ScFv in one heavy chain is connected to the ScFv in the other heavy chain. Through Ides digestion, if the presence of the Fc band can be determined, it is proved that there is an isomer. The concept of aggregate (polymer): two or three antibody proteins are bound together by covalent or non-covalent means. Monomer: A single complete antibody protein, free of aggregates and isomers. sFc: Single chain Fc, a heavy chain antibody below the hinge region of the antibody, including ScFv; The domain corresponds to the heavy chain after the hinge region. ScFv: It is an antibody composed of an antibody heavy chain variable region and a light chain variable region connected by a short peptide (linker) of 15 to 20 amino acids. Fc: Two sFcs are linked together by disulfide bonds.

### 3.1.3 HPLC purity detection

The HPLC method was used to detect the purity of the sample. The instrument was DIONEX UltiMate 3000 from Thermofisher Scientific; the chromatographic column was BioMix SEC 300 from SePax (Sepax, BioMix SEC, 3um, 150mmX4.6mm), and the mobile phase was PBS containing 0.673M NaCl, the column temperature was 25°C, the flow rate was 0.4ml/min, and the injection volume was 5ug.

In liquid-phase HPLC, separation of samples using a BioMix chromatography column enables the detection of not only aggregates but also monomers and isoforms, thereby allowing relative quantification of aggregates, monomers, and isoforms in antibody proteins.

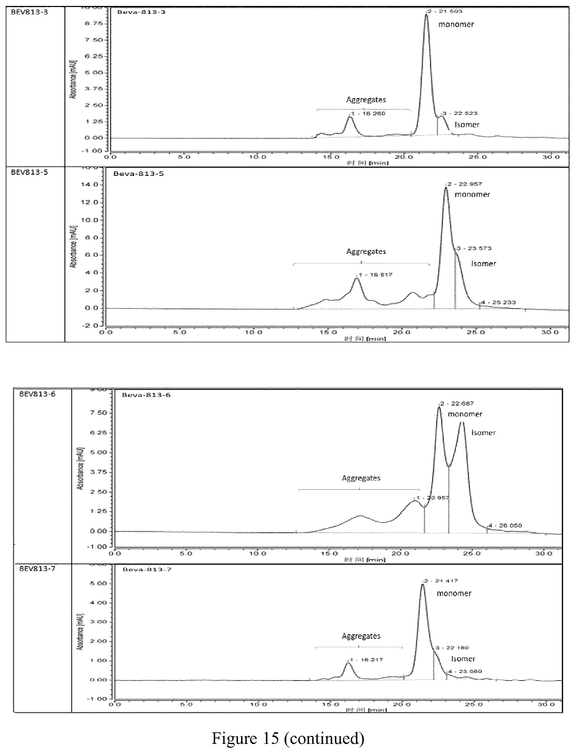

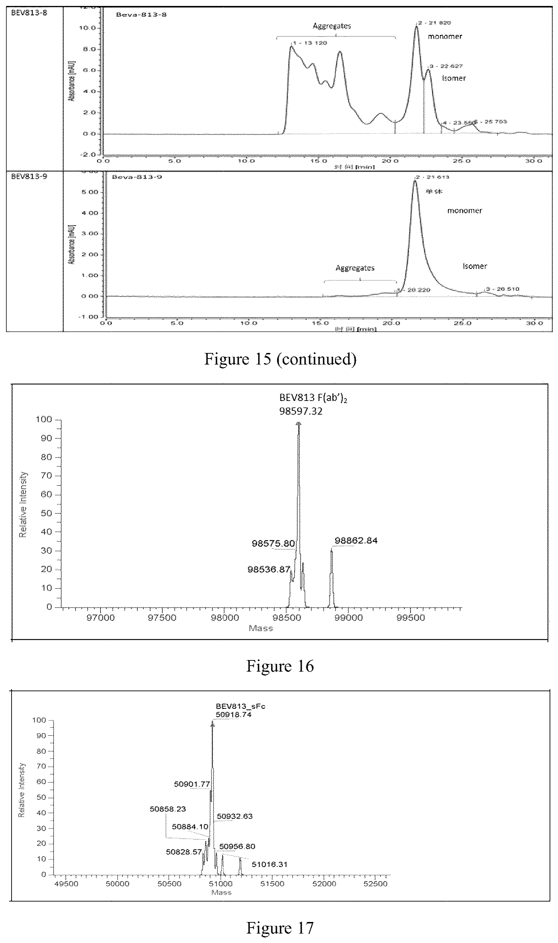

The results are shown in Figure 15. Only BEV813-9 exhibites mainly a single peak, and the rest have aggregates (before the main peak) and isomers (after the main peak). This result corresponds to the result of SDS-PAGE.

### 3.1.4 Structural characterization of mutant BEV813-9

### 3.1.4.1 Determination of molecular weight and isomer detection

Add the required amount of BEV813 or BEV813-9 antibody (0.5-10 mg/ml) to PBS buffer or other compatible buffer, then add IdeS protease to the IgG sample. Add 1 unit of IdeS protease to each 1µg of IgG for digestion. Incubate at 37°C for 30-60 minutes to obtain subunits. The liquid phase system was Thermo Fisher Vanquish UPLC, and the subunits were eluted in the column (MAbPac^{™} RP column, 4 µm, 3.0 × 100 mm (P/N 088644)) at a flow rate of 0.3ml/min, and mobile phase A is water containing 0.1% FA (Fomic acid), and mobile phase B is acetonitrile containing 0.1% FA. The gradient of mobile phase B was from 5% to 95% in 10 minutes. RP-UPLC was connected to a quadrupole electrostatic field orbital trap ultra-high resolution mass spectrometer (Thermo Orbitrap Exploris 240) for subunit molecular weight detection.

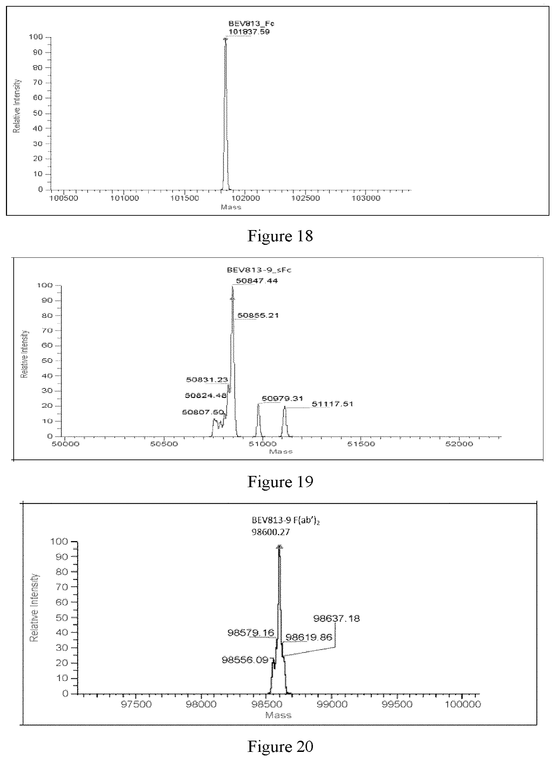

After deconvolution, the molecular weight of each subunit of the BsAb is listed in Table 7 below. The theoretical molecular weight is listed in the table, too. The errors between the actual measured molecular weight and the theoretical molecular weight are within the acceptable range. The main subunit of BEV813 is F(ab')₂, sFc and Fc. The presence of Fc indicates that there are isomers of this molecule (Figure 16-18). The subunit molecular weight of BEV813-9 is mainly F(ab')₂ and sFc, no Fc structure was detected, indicating that there are no isomers (Figure 19-20).

**Table 7 Molecular weight determination of BEV813 dual antibodies and BEV813-9 mutants**

| Antibody | Species Observed | Theoretical Mass(Da) | Observed Mass(Da) | Abundance |
|---|---|---|---|---|
| BEV813 | F(ab')₂ | 98601.14 | 98597.32 | Maj or |
| | sFc | 50919.30 | 50918.74 | Major |
| | Fc | 101838.60 | 101837.59 | Major |
| BEV813-9 | F(ab')₂ | 98601.14 | 98600.27 | Maj or |
| | sFc | 50847.19 | 50847.44 | Major |

Capillary electrophoresis (nrCGE (Non-reduced capillary gel electrophoresis)) was further used to detect the presence of antibody isomers. Inject 100µg of the sample (BEV813 dual antibodies and BEV813-9 mutants) into a 1.5mL microcentrifuge tube, and add the sample buffer (100 mM Tris-HCl, pH 9.0, 1% SDS) to a volume of 95µL, then add 2µL internal standard (10KD molecular weight marker), 5µL 250mM iodoacetamide (IAM) solution, cap the bottle, mix thoroughly, centrifuge at 300g for 1 min, seal with parafilm, then incubate in 70°C water for 10 min, and cool at room temperature for at least 3 minutes, transfer 100µL of sample to a 200µL microsample tube and spin to remove existing air bubbles. Place it into a universal bottle and cap the bottle. The PA800plus capillary electrophoresis instrument was used for detection. The antibodies were purified as follows before nrCGE detection. BEV813 and BEV-813-9 samples were captured by ProteinA in one step only from the cell culture; BEV813 monomer was captured by ProteinA and further purified by ion exchange (HiTrap Capto SP ImpRes, Cytiva: 17546851) as a control for nrCGE.

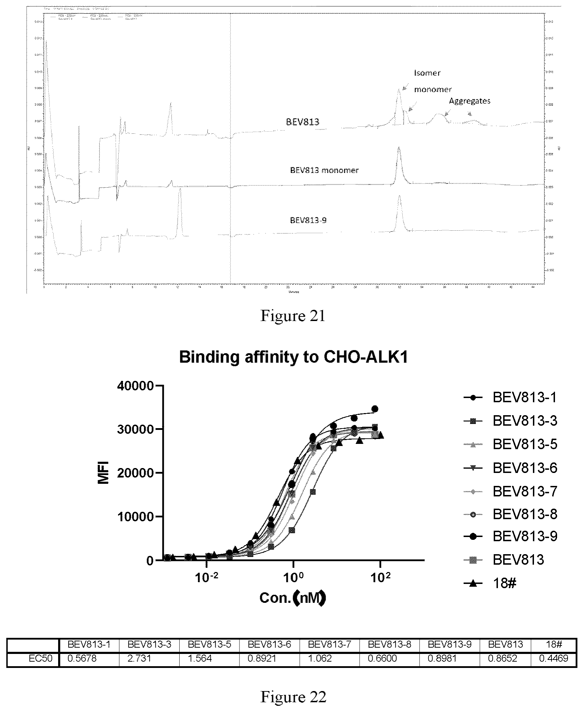

The results are shown in Figure 21. At the intact protein level, isoforms of BEV813 and BEV813-9 were detected by nrCGE. BEV813 showed a certain proportion of isomers, while BEV813-9 had no isomers.

### 3.2 In vitro activity of BEV813 bsAb mutants

### 3.2.1 Binding activity of BEV813 bsAb mutant to ALK-1 protein

The detection method is similar to the method mentioned above.

The affinity assay by ForteBio (Octet RED96e) was performed as below. The sensor was equilibrated offline in the assay buffer for 10 minutes, then detected online for 60 seconds to establish a baseline. Immobilize BM antibody and corresponding antibodies onto ProteinA sensors. Then transfer sensors to the human ALK-1 antigen solution. After that, transfer sensors to PBS containing 0.02% Tween-20 + 0.1% BSA in order to dissociate. Finally, a 1:1 binding model was used for kinetic analysis.

The results are shown in Table 8 below. The binding activity of BEV813-1, 5, 6, 8, and 9 mutants to ALK-1 antigen is better than that of BEV813.

**Table 8. Comparison of kinetic parameters of protein binding of BEV813 bsAb mutant to ALK-1 antigen**

| **Kinetic parameters of the binding of bispecific antibody BEV813, and the control antibody bevacizumab to VEGF165** | | | | |
|---|---|---|---|---|
| Sample ID | K*_{D}*(M) | K*ₒₙ* (1/Ms) | K*_{dis}* (1/s) | Full R^2 |
| BEV813-1 | 3.961E-09 | 4.560E05 | 1.806E-03 | 9.938E-01 |
| BEV 813-3 | 7.935E-09 | 3.011E05 | 2.389E-03 | 9.916E-01 |
| BEV 813-5 | 1.672E-09 | 4.185E05 | 6.995E-04 | 9.889E-01 |
| BEV 813-6 | 3.503E-09 | 4.039E05 | 1.415E-03 | 9.939E-01 |
| BEV 813-7 | 7.374E-09 | 4.058E05 | 2.992E-03 | 9.915E-01 |
| BEV 813-8 | 2.220E-09 | 5.243E05 | 1.164E-03 | 9.891E-01 |
| BEV 813-9 | 3.807E-09 | 6.445E05 | 2.454E-03 | 9.572E-01 |
| BEV 813 | 4.891E-09 | 4.470E05 | 2.186E-03 | 9.948E-01 |
| #18 | 1.342E-09 | 6.285E05 | 8.431E-04 | 9.935E-01 |

### 3.2.2 Binding activity of BEV813 bsAb to CHO-K1-hALK-1 cells

The detection method is consistent with the aforementioned method.

The results show in Figure 22 that the BEV813 and the mutants have high binding affinity to CHO-K1-hALK1 cells, especially BEV813-1.

### 3.2.3 BEV813 bsAb mutant blocks BMP9-induced phosphorylation of Smad1

The detection method is consistent with the aforementioned method in the patent.

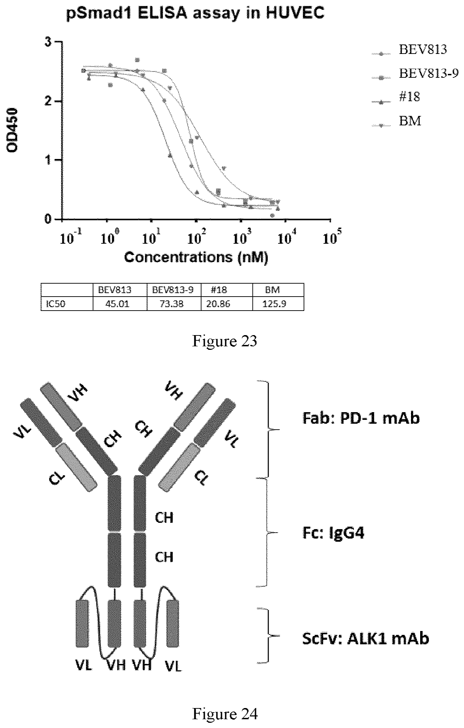

The results are shown in Figure 23. Both BEV813 and its mutant BEV813-9 have excellent blocking effect on the phosphorylation of Smad1 induced by BMP9, which is significantly better than the ALK1 monoclonal antibody BM.

### 4. Construction of anti-ALK-1/anti-PD-1 bispecific antibody (Nivo813 bsAb)

### 4.1 Format of Nivo813 bsAb

Nivo813 is a human IgG4 bispecific antibody with a typical "2+2" symmetric structure (as shown in Figure 24), which is made by combining the heavy chain variable region (VH) and the light chain variable region(VL)of the ALK-1 monoclonal antibody into the form of ScFv (Single-chain variable fragment)fused to the "C" end of the constant region of the PD-1 monoclonal antibody (Nivolumab).

The ALK-1 scFv antibody is connected to the C-terminus of the heavy chain of the PD-1 monoclonal antibody (Nivolumab) through a linker sequence (G20-linker: GGGGSGGGGSGGGGSGGGGS), thereby generating a new bispecific antibody Nivo813. In order to enhance the stability of ScFv, a point mutation to Cys was performed and a disulfide bond were introduced. At the same time, by maturing the affinity of ALK-1 mAb, the Fab segment of the antibody was modified to enhance its binding affinity without reducing the blocking function. The sequence information of Nivo813 is as follows:
PD-1 binding region of Nivo813 bsAb:
the heavy chain variable region (VH) of PD-1 binding region:



 (SEQ ID NO:83)

Three CDRs of the heavy chain variable region of the PD-1 binding region:
CDR1H: GITFSNSG (SEQ ID NO:84)
CDR2H: IWYDGSKR (SEQ ID NO:85)
CDR3H: ATNDDY (SEQ ID NO:86)
the light chain variable region (VL) of PD-1 binding region:


 (SEQ ID NO:87)

Three CDRs of the light chain variable region of the PD-1 binding region:
CDR1L: QSVSSY (SEQ ID NO:88)
CDR2L: DAS (SEQ ID NO:89)
CDR3L: QQSSNWPRT (SEQ ID NO:90)
(3) CH in the PD-1 binding region: 
ASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQ
EEMTKNQVSLTCLVKGFYPSDIAVEWESNGQPENNYKTTPPVLDSDGSFFLYSRLTVDK
SRWQEGNVFSCSVMHEALHNHYTQKSLSLSLGK (SEQ ID NO:91)
(4) CL of PD-1 binding region: 

 (SEQ ID
NO:92)
Linker sequence of PD-1 binding region: GGGGSGGGGSGGGGSGGGGS (SEQ ID NO: 65)
ALK-1 binding region of Nivo813 bsAb:
   (1) The light chain variable region (VL) of ALK-1 binding region: 
EIVLTQSPGTLSLSPGERATLSCRASQSVSSSYLAWYQQKPGQAPRLLIYGTSSRAT
GIPDRFSGSGSGTDFTLTISRLEPEDFAVYYCQAYGSWPITFGCGTRLEIK (SEQ ID NO:66)
   (2) the light chain variable region (VH) of ALK-1 binding region: 
QVQLQESGPGLVKPSQTLSLTCTVSGGSISSGEYYWNWIRQHPGKCLEWIGYIYYS
GSTYYNPSLKSRVTISVDTSKNQFSLKLSSVTAADTAVYYCARESVAGFDYWGQGTLVT
VSS (SEQ ID NO:67)
   (3) Linker sequence of ALK-1 binding region: GGGGSGGGGSGGGGSGGGGS (SEQ ID NO: 65)

The first chain of Nivo813:

QVQLVESGGGVVQPGRSLRLDCKASGITFSNSGMHWVRQAPGKGLEWVAVIWYD
GSKRYYADSVKGRFTISRDNSKNTLFLQMNSLRAEDTAVYYCATNDDYWGQGTLVTV
SSASTKGPSVFPLAPCSRSTSESTAALGCLVKDYFPEPVTVSWNSGALTSGVHTFPAVLQ
SSGLYSLSSVVTVPSSSLGTKTYTCNVDHKPSNTKVDKRVESKYGPPCPPCPAPEFLGGP
SVFLFPPKPKDTLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVMHEALHNHYTQKSLSLSLGKGGGGSGGGGSGGGGSGGGGSQVQLQ
ESGPGLVKPSQTLSLTCTVSGGSISSGEYYWNWIRQHPGKCLEWIGYIYYSGSTYYNPSL
KSRVTISVDTSKNQFSLKLSSVTAADTAVYYCARESVAGFDYWGQGTLVTVSSGGGGS
GGGGSGGGGSGGGGSEIVLTQSPGTLSLSPGERATLSCRASQSVSSSYLAWYQQKPGQA
PRLLIYGTSSRATGIPDRFSGSGSGTDFTLTISRLEPEDFAVYYCQAYGSWPITFGCGTRLE
IK(SEQ ID NO:93)

The second chain of Nivo813:

EIVLTQSPATLSLSPGERATLSCRASQSVSSYLAWYQQKPGQAPRLLIYDASNRATG
IPARFSGSGSGTDFTLTISSLEPEDFAVYYCQQSSNWPRTFGQGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO:94)

### 4.2 Preparation and purification of Nivo813 bsAb

For the preparation process of Nivo813 bsAb, please refer to the construction, expression and purification of anti-ALK-1 monoclonal antibodies and their mutants.

### 4.3 In vitro activity of Nivo813 bsAb

### 4.3.1 Binding activity of Nivo813 bsAb to ALK-1 protein and PD-1 protein

The detection method is consistent with that of BEV813 bsAb mentioned in the patent. Among them, Nivolumab as the control was purchased from Beijing Yiqiao Shenzhou Technology Co., Ltd., and product number is 68050-H001. The antigen PD-1 protein was purchased from Antibody system and product number is YHH02201; the antigen ALK-1 protein was purchased from ACROBiosystems and product number is AL1-H5227.

The results are shown in Tables 9 and 10 respectively. The binding activity of Nivo813 to PD-1 antigen is equivalent to that of Nivolumab; the binding activity to ALK-1 antigen is equivalent to that of the control antibody BM, but weaker than #18 mutant.

**Table 9. Comparison of binding kinetic parameters of Nivo813 bsAb, Nivolumab to PD-1 antigen protein**

| Sample ID | K*_{D}* (M) | K*ₒₙ* (1/Ms) | K*_{off} (*1/s) | Full R^2 |
|---|---|---|---|---|
| Nivolumab | 4.664E-09 | 1.400E06 | 6.530E-03 | 0.997 |
| Nivo813 | 4.521E-09 | 1.410E06 | 6.375E-03 | 0.995 |

**Table 10 Comparison of binding kinetic parameters of of Nivo813 bsAb, ALK-1 monoclonal antibody (BM, #18) to ALK-1 antigen protein**

| Kinetic parameters of the binding of bispecific antibody Nivo813, monoclonal antibody ALK-1 (#18) and the control antibody BM to ALK-1-his | | | | |
|---|---|---|---|---|
| Sample ID | K*_{D}* (M) | K*ₒₙ* (1/Ms) | K*_{off}* (1/s) | Full R^2 |
| BM | 1.386E-09 | 5.981E05 | 8.289E-04 | 0.996 |
| #18 | 6.995E-10 | 4.759E05 | 3.329E-04 | 0.991 |
| Nivo813 | 1.103E-09 | 5.954E05 | 6.569E-04 | 0.995 |

### 4.3.2 Binding activity of Nivo813 bsAb that simultaneously binds to PD-1 and ALK-1 antigens

Add 2 µg/mL ALK-1-his (ACROBiosystems, AL1-H5227) to the 96-well plate and incubate at 4°C overnight. The next day, the coated ALK-1-his antigen was washed off thoroughly with wash buffer, add 5% BSA (Shanghai Beyotime Biotechnology, ST023-200g) diluted with ELISA diluent buffer (Biolegend, 421203) and incubate at 37°C for 2 hours(300rpm). After washing away the blocking solution with washing solution, add different antibodies to be tested (primary antibody) (15nM, 3-fold dilution), and incubate at 37°C for 2 hours(300rpm). Human IgG1 antibody (Beijing Yiqiao, HG1K) was used as a negative control antibody. After washing away the primary antibody with washing buffer, add 5 µg/ml PD-1-mFc (Yiqiao Shenzhou, 10377-H05H), and incubate at 37°C for 1 hour(300rpm). After washing away the above protein with washing solution, add the secondary antibody Goat Anti-Mouse IgG-HRP (Yiqiao Shenzhou, SSA007) or Goat Anti-Human IgG-HRP (Yiqiao Shenzhou, SSA002) diluted 1:1000 incubate at 37°C for 1 hour(300rpm)). Then, 100 µl of the substrate 3,3',5,5'-Tetramethylbenzidine TMB (Liankebio, E0231) was added and the reaction was carried out for 15-30 minutes, and stop solution (Liankebio, E0300) was added to terminate the reaction. Finally, the absorbance value of OD450nm wavelength was read on a microplate reader (Molecular Devices, ID5-STD).

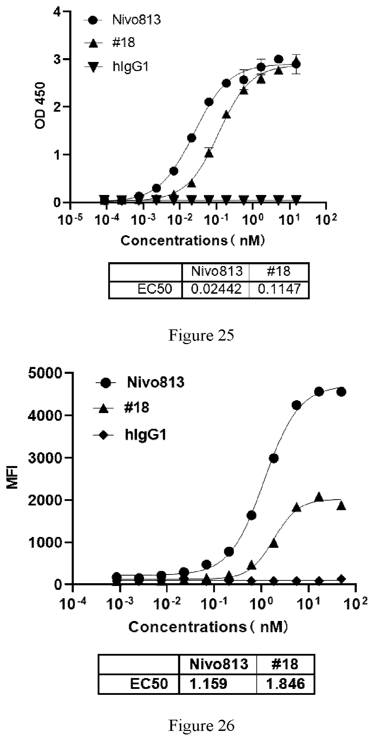

The results are shown in Figure 25. Nivo813 can binds to the antigens PD-1 and ALK-1 simultaneously with higher binding affinity than that of ALK-1 mAb (#18mutant); the EC50 value of Nivo813 bsAb is 5 times of that of #18 mutant.

### 4.3.3 Binding activity of Nivo813 bsAb to cell lines with high expression of ALK-1 or PD-1

1×10⁵ cells/ml of CHO-K1-ALK-1 cells (provided by Stainwei) were seeded in a 96-well plate, antibodies (primary antibody) to be tested (50 nM, 3-fold dilution) were added and incubated at 4°C for 1 hour. After the primary antibody is fully washed away in the FACS buffer, 10 µg/mL human PD-1-PE (ACRObiosystems, PD-1HP2F2, Fc) and 1:20 PE anti-human IgG Fc Antibody (secondary antibody) (Biolegend, 410708) are added respectively, incubate at 4 °C for 1 hour. After fully washing away the secondary antibodies with FACS buffer, collect the cells, and use flow cytometry to analyze the binding of the tested antibodies to CHO-K1-ALK-1 cells.

2×10⁶ H-PD-1 NFAT Reportor Jurkat cells (Genomeditech) cells/ml were seeded in a 96-well plate, and different antibodies (primary antibody) to be tested (50nM, 3-fold dilution) were added and incubated at 4°C for 1 hour. After fully washing away the primary antibody with FACS buffer, 10 µg/mL human ALK-1-his protein (ACRObiosystems, AL1-H 5228) was added and incubated at 4°C for 1 hour. After fully washing away the above proteins with FACS buffer, add 1:10 APC anti-His Tag Antibody (secondary antibody) (Biolegend, 362605) and 1:10 PE anti-human IgG Fc Antibody (secondary antibody) (Biolegend, 410708) respectively) and then incubate at 4°C for 1 hour. After fully washing away the secondary antibodies with FACS buffer, collect the cells, and use flow cytometry to analyze the binding of the tested antibodies to H-PD-1 NFAT Reportor Jurkat cells.

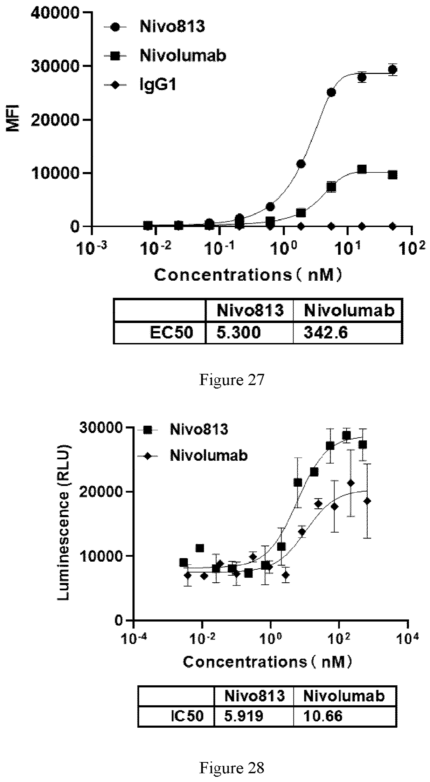

The binding activity of Nivo813 bsAb, ALK-1mAb (#18 mutant), and nivolumab to cell surface receptors was compared by two overexpression cell lines. The results are shown in Figures 26-27. Compared with ALK-1 mAb (#18 mutant), Nivo813 bsAb has higher binding activity to the ALK-1 receptor on CHO-K1-ALK-1 cell line (Figure 26). Similarly, compared with nivolumab, Nivo813 bsAb has higher binding activity to the PD-1 receptor on the H-PD-1 NFAT Reportor Jurkat cell line, and the EC50 value of Nivo813 bsAb is 65 times that of Nivolumab (Figure 27).

### 4.3.4 Nivo813 bsAb blocks the PD-1/PD-L1 interaction, causing the transduction of the TCR signaling pathway and NFAT-mediated luciferase expression

2.5×10⁴ Cells/well CHO-K1-PDL1 cells (Yoshiman Biotech) were seeded in 96-well plates and cultured overnight. The next day, 1×10⁵ Cells/well H-PD-1/NFAT reporter Jurkat cells (Jiman Biotech) containing the antibody to be tested (100ug/ml, 3-fold dilution) were added to the above-mentioned well plate, mixed thoroughly, and incubated at 37°C for 16 hours. The cell supernatant was taken to analyze the expression of luciferase.

The results are shown in Figure 28. Both Nivo813 bsAb and nivolumab can inhibit PD1/PDL1 interaction and restore downstream signal transduction and NFAT-mediated luciferase expression. Among them, Nivo813 bsAb has a higher inhibitory effect than nivolumab.

### 4.3.5 Nivo813 bsAb blocks BMP9-induced phosphorylation of Smad1

As a ligand of ALK1, BMP9 molecules can activate the ALK1 receptor by binding to the ALK1 protein on the surface of HUVEC cells or A172 cells, thereby mediating the phosphorylation of the downstream protein Smad1. Therefore, we designed experiments to detect the blocking effect of Nivo813 on BMP9-induced Smad1 phosphorylation.

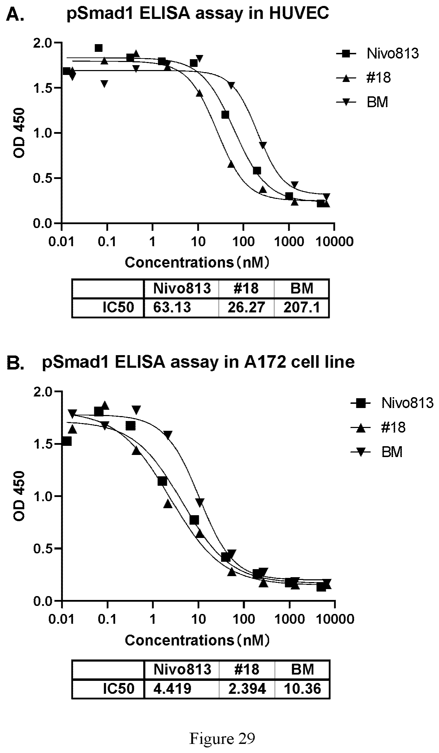

2×10⁴ per well of HUVEC (Allcells, H-001F-C) as shown in Figure 29. A and A172 cells (Kebai Biotech, CBP60575) as shown in Figure 29. B were seeded on a 96-well plate, and cultured in ECM medium containing 5% FBS and ECGS (Sciencell, 1001) or DMEM medium containing 10% FBS, 37°C, 5% CO₂ overnight. Remove the culture medium from the plate and wash twice with 200 µl PBS. Add 100 µl of ECM without FBS and ECGS or DMEM without FBS, and then starve the cells for 1 hour. Remove the culture medium and add 100uL of a certain concentration of gradient diluted tested antibody, treat for 3 hours, then add BMP9 at a final concentration of 0.15ng/mL or 0.3ng/mL, incubate for 45 minutes. Finally, the medium was removed and Smad1 phosphorylation levels were determined using an ELISA kit (Invitrogen, 85-86182-11).

The results are shown in Figure 29. The ALK1 monoclonal antibody (#18 mutant) has the strongest blocking effect, but the Nivo813 also shows good blocking effect.

### 4.3.6 Nivo813 bsAb inhibits HUVEC microtubule formation

200 µL of Matrigel (Corning: 354234, California, USA) was added to the 24-well plate and solidified at 37°C for 30 minutes. HUVEC cells were incubated with 2 µg/ml calcein AM for 30 min and washed with PBS. 4×10⁴ HUVEC cells were added to a 24-well plate, and the cells were treated with different concentration gradients of the antibody (1 mg/ml, 4-fold dilution). Human IgG4 antibody (Biointron, B107804) was used as the isotype control. After incubation at 37°C for 48 hours, the formation of microtubules was observed using a fluorescence microscope (Olympus CKX53, Tokyo, Japan).

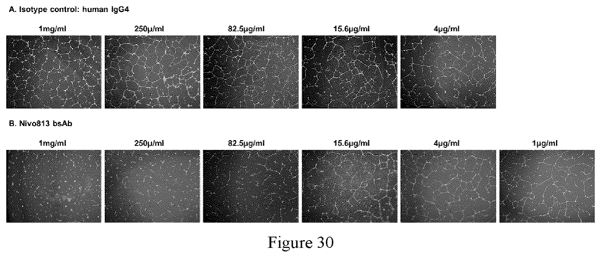

The results are shown in Figure 30. As the concentration of Nivo813 bsAb increases, HUVEC microtubule formation is inhibited in a dose-dependent manner.

### 4.4 In vivo efficacy testing of Nivo813 bsAb

### 4.4.1 Human immune system reconstruction CDX mouse model

MDA-MB-231 (ATCC No. HTB-26) human breast cancer cells were inoculated subcutaneously into tumor donor mice (NCG mice, female, provided by Jiangsu Jicui Yaokang Biotechnology Co., Ltd.). After the tumors grew, take out tumors with a volume of about 500~1000mm³ under sterile conditions, cut into approximately a small piece of 2 mm × 2 mm × 2 mm, and then inoculate subcutaneously on the right flank of the mouse with a trocar. Three days after tumor inoculation, healthy adult PBMC (human peripheral blood mononuclear cells, Donor#: SC12291, provided by Shanghai Xuanfeng Biotechnology Co., Ltd.) were resuspended in PBS and inoculated into mice with a dose of 2 × 10⁶ /mouse. One day before grouping and at the end of the experiment, FACS was used to detect the proportion of human CD45-positive cells in the blood of mice. Animals were treated with Bevacizumab, BEV813 bsAb, or saline. Bevacizumab was administered at a dose of 5 mg/kg, and BEV813 bsAb was administered at a dose of 6.7 mg/kg. Animals were treated with Nivolumab, ALK-1 mAb (#18 mutant) and Nivo813 bsAb, or saline (control vehicle). The dosage of Nivolumab, ALK-1 mAb (#18 mutant) monoclonal antibody, Nivo813 bsAb is 1 mg/kg, 5 mg/kg , 1.33 mg/kg, respectively. When the mean tumor size reached 53 mm³, the drugs were administered in groups. Each antibody was administered intraperitoneally three times a week in a volume of 100 mL each. Each group consisted of 8 mice. Tumor volume and mouse body weight were measured twice weekly. Three weeks after dosing, the animals were euthanized and the tumors were removed and weighed. One-Way ANOVA test was used to perform statistical analysis between groups on tumor volume and tumor weight, and p<0.05 was considered to have significant differences.

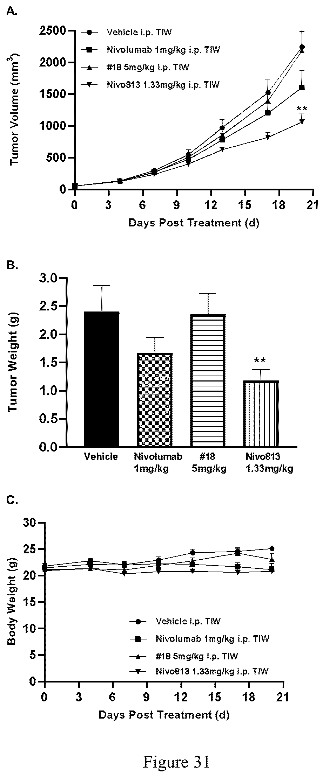

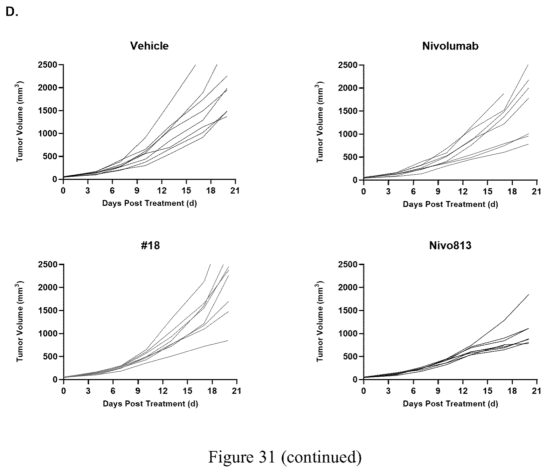

20 days after grouping, the tumor growth inhibition rate (TGI_{TV}%) of the Nivolumab (1mg/kg) group, ALK-1 mAb (#18 mutant) (5mg/kg) group and Nivo813 bsAb (1.33mg/kg) group were 30%, 0%, and 52% respectively (A in Figure 31); tumor weight inhibition rate (TGI_{TW}%) are 30%, 2%, and 51% respectively (B in Figure 31). ALK-1 mAb (#18 mutant) cannot inhibit tumor growth at a dose of 5 mg/kg; Nivolumab has a certain inhibitory effect on tumor growth at a low dose of 1 mg/kg, but compared with the control group, there was no significant difference (p>0.05); However, Nivo813 bsAb had a significant inhibitory effect on tumor growth in an equimolar with Nivolumab (1.33 mg/kg), and the tumor volume and weight were significantly smaller than that of the control group (Figure 31 A and B, **p=0.002; **p=0.008). In addition, in the group treated with NIvo813, 7 mice (total 8) showed good anti-tumor efficacy, while in the group treated with Nivolumab, Only3 (total 8) mice showed certain anti-tumor efficacy (D in Figure 31). During the treatment, Nivolumab, ALK-1 mAb (#18 mutant) and Nivo-813 bsAb had no acute adverse reactions after administration. The mice in each group ate and drank normally without obvious abnormality (C in Figure 31).

## Claims

1. A monoclonal antibody or an antigen-binding portion thereof that binds to ALK-1, wherein the antibody or an antigen-binding portion thereof has a heavy chain and a light chain;
the CDR1H, CDR2H and CDR3H of the heavy chain variable domain VH of the antibody or antigen-binding portion thereof are CDR1H, CDR2H and CDR3H of the heavy chain variable domain of SEQ ID NO: 1; or the CDR1H, CDR2H and CDR3H of the heavy chain variable domain VH of the antibody or antigen-binding part thereof are CDR1H, CDR2H and CDR3H of the sequence which is obtained by adding, deleting or replacing one or more amino acids in SEQ ID NO: 1 and has the same function as SEQ ID NO: 1;
the CDR1L, CDR2L and CDR3L of the light chain variable domain VL of the antibody or its antigen-binding portion thereof are obtained by amino acid mutation based on the CDR1L, CDR2L and CDR3L of the light chain variable domain of SEQ ID NO: 5, the mutation refers to mutation of amino acids at position 91 and position 95 of SEQ ID NO: 5.

2. The monoclonal antibody or antigen-binding portion thereof that binds to ALK-1 according to claim 1, the amino acids No. 91 and No. 95 are not a combination of tryptophan W and valine V, proline P and tryptophan W, leucine L and phenylalanine F.

3. The monoclonal antibody or antigen-binding portion thereof that binds to ALK-1 according to claim 1 or 2, the CDR1L, CDR2L and CDR3L of the light chain variable domain VL of the antibody or antigen-binding portion thereof are CDR1L, CDR2L and CDR3L of the light chain variable domain of SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53 or SEQ ID NO:55;
preferably, the CDR1L, CDR2L and CDR3L of the light chain variable domain VL of the antibody or antigen-binding portion thereof are CDR1L, CDR2L and CDR3L of the light chain variable domain of SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ **ID** NO:31, SEQ **ID** NO:35, SEQ ID NO:37, SEQ **ID** NO:39, SEQ ID NO:41, SEQ **ID** NO:43, SEQ ID NO:45, SEQ ID NO:47, SEQ **ID** NO:49, SEQ **ID** NO:51, SEQ ID NO:55;
more preferably, the CDR1L, CDR2L and CDR3L of the light chain variable domain VL of the antibody or antigen-binding portion thereof are CDR1L, CDR2L and CDR3L of the light chain variable domain of SEQ ID NO:11, SEQ ID NO:13, SEQ ID NO:15, SEQ ID NO:25, SEQ ID NO:31 or SEQ ID NO:51;
most preferably, the CDR1L, CDR2L and CDR3L of the light chain variable domain VL of the antibody or antigen-binding portion thereof are CDR1L, CDR2L and CDR3L of the light chain variable domain of SEQ ID NO:11.

4. The monoclonal antibody or antigen-binding portion thereof that binds to ALK-1 according to any one of claims 1-3, as defined according to the IMGT antibody numbering scheme for CDR regions, the light chain CDR is selected from:
(a) the light chain complementarity determining region 1 (CDR1L) described in the amino acid sequence of SEQ ID:6, the light chain complementarity determining region 2 (CDR2L) described in the amino acid sequence of SEQ ID:7, and the light chain complementarity determining region 3 (CDR3L) described in the amino acid sequence of SEQ ID:12; or
(b) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:14; or
(c) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:16; or
(d) he light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:18; or
(e) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:20; or
(f) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:22; or
(g) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:24; or
(h) the light chain complementarity determining region 1 (CDR1L) described in the amino acid sequence of SEQ ID: 6, the light chain complementarity determining region 2(CDR2L) described in the amino acid sequence of SEQ ID: 7, and the light chain complementarity determining region 3 (CDR3L) described in the amino acid sequence of SEQ ID:26; or
(i) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:28; or
(j) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:30; or
(k) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:32; or
(l) the light chain complementarity determining region 1 (CDR1L) described in the amino acid sequence of SEQ ID: 6, the light chain complementarity determining region (CDR2L) described in the amino acid sequence of SEQ ID: 7, and the light chain complementarity determining region 3 (CDR3L) described in the amino acid sequence of SEQ ID:34; or
(m) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:36; or
(n) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:38; or
(o) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:40; or
(p) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:42; or
(q) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:44; or
(r) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:46; or
(s) the light chain complementarity determining region 1 (CDR1L) described in the amino acid sequence of SEQ ID: 6, the light chain complementarity determining 2 (CDR2L) described in the amino acid sequence of SEQ ID: 7, and the light chain complementarity determining region 3 (CDR3L) described in the amino acid of SEQ ID:48; or
(t) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:50; or
(u) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:52; or
(v) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID:54; or
(w) the light chain complementarity determining region 1 (CDR1L) described in the amino acid sequence of SEQ ID:6, the light chain complementarity determining region 2 (CDR2L) described in the amino acid sequence of SEQ ID:7, and the light chain complementarity determining region 3 (CDR3L) described in the amino acid sequence of SEQ ID:56;
the heavy chain includes the heavy chain complementarity determining region 1 (CDR1H) described in the amino acid sequence of SEQ ID:2, the heavy chain complementarity determining region 2 (CDR2H) described in the amino acid sequence of SEQ ID:3, and the heavy chain complementarity determining region 3 (CDR3H) described in the amino acid sequence of SEQ ID:4;
preferably, the light chain CDR region is selected from the CDR of (a)-(k), (m)-(u) or (w) above;
further preferably, the light chain CDR region is selected from the CDR of (a)-(c), (h), (k) or (u); most preferably selected from the CDR in (a).

5. The monoclonal antibody or antigen-binding portion thereof that binds to ALK-1 according to any one of claims 1-4, the CDR1H, CDR2H and CDR3H of the heavy chain variable domain VH of the antibody or antigen-binding portion thereof are the CDR1H, CDR2H and CDR3H of the heavy chain variable domain of SEQ ID NO: 1; the CDR1L, CDR2L and CDR3L of the light chain variable domain VL of the antibody or antigen-binding portion thereof are the CDR1L, CDR2L and CDR3L of the light chain variable domains of SEQ ID NO: 11, SEQ ID NO: 13, SEQ ID NO: 15, SEQ ID NO: 25, SEQ ID NO: 31 or SEQ ID NO: 51;
preferably, the CDR1L, CDR2L and CDR3L of the light chain variable domain VL of the antibody or antigen-binding portion thereof are the CDR1L, CDR2L and CDR3L of the light chain variable domain of SEQ ID NO: 11.

6. The monoclonal antibody or antigen-binding portion thereof that binds to ALK-1 according to any one of claims 1-5, the heavy chain variable domain VH of the antibody or antigen-binding portion thereof comprises the amino acid sequence of SEQ ID NO:1, SEQ ID NO:67, SEQ ID NO:70, SEQ ID NO:73, SEQ ID NO:75, SEQ ID NO:78, SEQ ID NO:80 or SEQ ID NO:81; the light chain variable domain VL of the antibody or antigen-binding portion thereof comprises the amino acid sequence of SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:66, SEQ ID NO:71, SEQ ID NO:72, SEQ ID NO:74, SEQ ID NO:76, SEQ ID NO:77 or SEQ ID NO:79;
preferably, the light chain variable domain VL of the antibody or antigen-binding portion thereof comprises the amino acid sequence of SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:55, SEQ ID NO:66, SEQ ID NO:71, SEQ ID NO:72, SEQ ID NO:74, SEQ ID NO:76, SEQ ID NO:77 or SEQ ID NO:79;
more preferably, the light chain variable domain VL of the antibody or antigen-binding portion thereof comprises the amino acid sequence of SEQ ID NO:11, SEQ ID NO: 13, SEQ ID NO:15, SEQ ID NO:25, SEQ ID NO:31, SEQ ID NO:51, SEQ ID NO:72, SEQ ID NO:79 or SEQ ID NO:76; most preferably, the light chain variable domain VL of the antibody or antigen-binding portion thereof comprises the amino acid sequence of SEQ ID NO: 11.

7. The monoclonal antibody or antigen-binding portion thereof that binds to ALK-1 according to any one of claims 1-6, the antigen-binding fragment is a Fab fragment, an F(ab')₂ fragment or a single-chain antibody.

8. The monoclonal antibody or antigen-binding portion thereof that binds to ALK-1 according to any one of claims 1-7, the antibody or antigen-binding fragment is IgG, IgM, IgE, IgA or IgD, further preferably the antibody or antigen-binding fragment is IgG1, IgG2, IgG3 or IgG4, more preferably the antibody or antigen-binding fragment is human IgG1-LALA.

9. A bispecific antibody or antigen-binding fragment, comprises a first antigen-binding region (ALK-1 binding region) that specifically binds to ALK-1 and a second antigen-binding region that specifically binds to VEGF(VEGF binding region); the first antigen-binding region that specifically binds to ALK-1 comprises a heavy chain variable region (VH) and a light chain variable region (VL), and the second antigen-binding region that specifically binds to VEGF comprises a heavy chain variable region (VH) and a light chain variable region (VL) or a VEGF receptor fragment that specifically binds to VEGF;
the CDR1H, CDR2H and CDR3H of the heavy chain variable domain VH of the first antigen binding region are CDR1H, CDR2H and CDR3H of the heavy chain variable domain of SEQ ID NO: 1; or the CDR1H, CDR2H and CDR3H of the heavy chain variable domain VH of the first antigen binding region are CDR1H, CDR2H and CDR3H of the sequence which is obtained by adding, deleting or replacing one or more amino acids in SEQ ID NO:1 and has the same function as SEQ ID NO: 1;
the CDR1L, CDR2L and CDR3L of the light chain variable domain VL of the first antigen binding region are obtained by amino acid mutation based on the CDR1L, CDR2L and CDR3L of the light chain variable domain of SEQ ID NO: 5; the mutation refers to the mutation of amino acids at position 91 and position 95 of SEQ ID NO: 5.

10. The bispecific antibody or antigen-binding fragment according to claim 9, the mutation refers to the mutation of amino acids at position 91 and position 95 of SEQ ID NO: 5, and the amino acids No. 91 and No. 95 are not a combination of tryptophan W and valine V, proline P and tryptophan W, leucine L and phenylalanine F.

11. The bispecific antibody or antigen-binding fragment according to any one of claims 9 or 10, the CDR1L, CDR2L and CDR3L of the light chain variable domain VL of the first antigen binding region are CDR1L, CDR2L and CDR3L of the light chain variable domain of SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53 or SEQ ID NO:55;
preferably, the CDR1L, CDR2L and CDR3L of the light chain variable domain VL of the first antigen binding region are CDR1L, CDR2L and CDR3L of the light chain variable domain of SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:55;
more preferably, the CDR1L, CDR2L and CDR3L of the light chain variable domain VL of the first antigen binding region are CDR1L, CDR2L and CDR3L of the light chain variable domain of SEQ ID NO:11, SEQ ID NO:13, SEQ ID NO:15, SEQ ID NO:25, SEQ ID NO:31 or SEQ ID NO:51;
most preferably, the first CDR1L, CDR2L and CDR3L of the light chain variable domain VL of an antigen-binding region are CDR1L, CDR2L and CDR3L of the light chain variable domain of SEQ ID NO: 11.

12. The bispecific antibody or antigen-binding fragment according to any one of claims 9-11, the CDR1H, CDR2H and CDR3H of the heavy chain variable domain VH of the first antigen binding region are CDR1H, CDR2H and CDR3H of the heavy chain variable domain of SEQ ID NO: 1.

13. The bispecific antibody or antigen-binding fragment according to any one of claims 9-12, as defined according to the IMGT antibody numbering scheme for CDR regions, the light chain CDR of the first antigen-binding region is selected from the following combination:
(a) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 12; or
(b) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 14; or
(c) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 16; or
(d) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 18; or
(e) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 20; or
(f) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 22; or
(g) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 24; or
(h) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 26; or
(i) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 28; or
(j) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 30; or
(k) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 32; or
(l) The light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 34; or
(m) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 36; or
(n) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 38; or
(o) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 40; or
(p) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 42; or
(q) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 44; or
(r) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 46; or
(s) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 48; or
(t) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 50; or
(u) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 52; or
(v) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 54; or
(w) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 56;
the heavy chain variable domain of the first antigen binding region includes the heavy chain complementarity determining region 1 (CDR1H) described in the amino acid sequence of SEQ ID: 2, and the heavy chain complementarity determining region 2 (CDR2H) described in the amino acid sequence of SEQ ID: 3, the heavy chain complementarity determining region 3 (CDR3H) described in the amino acid sequence of SEQ ID: 4;
preferably, the light chain CDR region is selected from the CDR in (a)-(k), (m)-(u) or (w) described above; further preferably selected from CDR in (a)-(c) , (h), (k) or (u), most preferably selected from in (a).

14. The bispecific antibody or antigen-binding fragment according to any one of claims 9-13, the heavy chain variable region of the first antigen binding region comprises the amino acid sequence of SEQ ID NO: 1, SEQ ID NO: 67, SEQ ID NO: 70, SEQ ID NO: 73, SEQ ID NO: 75, SEQ ID NO: 78, SEQ ID NO: 80 or SEQ ID NO: 81; or comprises the heavy chain variable region of the first antigen binding region which is obtained by adding, deleting or replacing one or more amino acids in SEQ ID NO: 1 and has the same function as SEQ ID NO: 1;
preferably, the heavy chain variable region of the first antigen-binding region comprises the amino acid sequence of SEQ ID NO: 1, SEQ ID NO: 67 or SEQ ID NO: 81.

15. The bispecific antibody or antigen-binding fragment according to claim 14, the light chain variable region of the first antigen binding region comprises the amino acid sequence selected from those shown in SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:66, SEQ ID NO:71, SEQ ID NO:72, SEQ ID NO:74, SEQ ID NO:76, SEQ ID NO:77 or SEQ ID NO:79;
preferably, the light chain variable domain VL of the first antigen binding region comprises the amino acid sequence selected from those shown in SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:55, SEQ ID NO:66, SEQ ID NO:71, SEQ ID NO:72, SEQ ID NO:74, SEQ ID NO:76, SEQ ID NO:77or SEQ ID NO:79;
more preferably, the light chain variable domain VL of the first antigen binding region comprises the amino acid sequence selected from those shown in SEQ ID NO:11, SEQ ID NO:13, SEQ ID NO:15, SEQ ID NO:25, SEQ ID NO:31, SEQ ID NO:51, SEQ ID NO:66, SEQ ID NO:72, SEQ ID NO:79 or SEQ ID NO:76;
most preferably, the light chain variable domain VL of the first antigen binding region is the amino acid sequence shown in SEQ ID NO:11, SEQ ID NO:66, SEQ ID NO:72, SEQ ID NO:79 or SEQ ID NO:76.

16. The bispecific antibody according to any one of claims 9-15, the bispecific antibody comprises a heavy chain constant region of an IgG, preferably the bispecific antibody comprises a heavy chain constant region of an IgG1, IgG4 or IgG2; more preferably the bispecific antibody comprises a heavy chain constant region of an IgG1; most preferably, the bispecific antibody comprises a heavy chain constant region of SEQ ID NO:9.

17. The bispecific antibody or antigen-binding fragment according to any one of claims 9-16, the second antigen-binding region that specifically binds VEGF comprises a heavy chain variable region and a light chain variable region; the CDR1H, CDR2H and CDR3H of the heavy chain variable region VH are CDR1H, CDR2H and CDR3H of the heavy chain variable domain of SEQ ID NO: 57; or the CDR1H, CDR2H and CDR3H of the heavy chain variable region VH are CDR1H, CDR2H and CDR3H of the sequence which is obtained by adding, deleting or replacing one or more amino acids in SEQ ID NO: 57 and has the same function as SEQ ID NO: 57; the CDR1L, CDR2L and CDR3L of the light chain variable region VL are CDR1L, CDR2L and CDR3L of the light chain variable domain of SEQ ID NO:61, or the CDR1L, CDR2L and CDR3L of the heavy chain variable region VL are CDR1L, CDR2L and CDR3L of sequence which is obtained by adding, deleting or replacing one or more amino acids in SEQ ID NO: 61 and has the same function as SEQ ID NO: 61.

18. The bispecific antibody or antigen-binding fragment according to claim 17, the second antigen-binding region that specifically binds to VEGF comprises a heavy chain variable region and a light chain variable region,
the heavy chain variable region comprises CDR1H as shown in SEQ ID NO: 58, CDR2H as shown in SEQ ID NO: 59 and CDR3H as shown in SEQ ID NO: 60; and
the light chain variable region includes CDR1L as shown in SEQ ID NO:62, CDR2L as shown in SEQ ID NO:63 and CDR3L as shown in SEQ ID NO:64.

19. The bispecific antibody or antigen-binding fragment according to any one of claims 9-18, the heavy chain variable region comprises the sequence of SEQ ID NO: 57 and the light chain variable region comprises the sequence of SEQ ID NO: 61.

20. The bispecific antibody or antigen-binding fragment according to any one of claims 9-19, the first antigen binding region or the second antigen binding region is in scFv form; preferably, the first antigen binding region is in scFv form.

21. The bispecific antibody or antigen-binding fragment according to any one of claims 9-20, the first antigen-binding region and the second antigen-binding region are connected through a linker,
preferably, the linker includes (G4S)n, n is an integer greater than 1;
more preferably, the Linker is composed of (G4S)n, n is an integer from 2 to 10;
most preferably, the linker is composed of (G4S)n, and n is 2, 3 or 4.

22. The bispecific antibody or antigen-binding fragment according to any one of claims 9-21, the scFv includes a heavy chain variable region and a light chain variable region, and the heavy chain variable region and the light chain variable region are connected by a linker,
preferably, the linker includes (G4S)n, n is an integer greater than 1;
more preferably, the linker is composed of (G4S)n, n is an integer from 2 to 10;
most preferably, the linker consists of (G4S)n, where n is 2, 3 or 4.

23. The bispecific antibody or antigen-binding fragment according to any one of claims 9-22, the bispecific antibody consists of 4 chains, including two identical first chains and two identical second chains,
the first chain sequentially includes the VH of the VEGF binding region, the CH of the VEGF binding region, the Linker, the VH of the ALK-1 binding region, the Linker and the VL of the ALK-1 binding region from the N terminal to the C terminal; and
the second chain sequentially includes VL and CL of the VEGF binding region from N-terminal to C-terminal.

24. The bispecific antibody or antigen-binding fragment according to claim 23, the specific sequence contained in the bispecific antibody is as follows:
the VH sequence of the VEGF binding region is shown in SEQ ID NO: 57,
the CH sequence of the VEGF binding region is shown in SEQ ID NO: 9,
the VL sequence of the VEGF binding region is shown in SEQ ID NO: 61,
the CL sequence of the VEGF binding region is shown in SEQ ID NO: 10,
the Linker sequence is shown in SEQ ID NO: 65,
the VL sequence of the ALK-1 binding region is shown in SEQ ID NO: 66, SEQ ID NO: 71, SEQ ID NO: 72, SEQ ID NO: 74, SEQ ID NO: 76, SEQ ID NO: 77 or SEQ ID NO :79, and
the VH sequence of the ALK-1 binding region is shown in SEQ ID NO: 67, SEQ ID NO: 1, SEQ ID NO: 70, SEQ ID NO: 73, SEQ ID NO: 75, SEQ ID NO: 78, SEQ ID NO :80 or SEQ ID NO:81;
preferably, the sequence of the first chain is shown as SEQ ID NO: 68 or SEQ ID NO: 82; the sequence of the second strand is shown in SEQ ID NO: 69.

25. A bispecific antibody or antigen-binding fragment, comprises a first antigen-binding region (ALK-1 binding region) that specifically binds to ALK-1 and a second antigen-binding region that specifically binds to PD-1(PD-1 binding region); the first antigen-binding region that specifically binds to ALK-1 comprises a heavy chain variable region (VH) and a light chain variable region (VL), and the second antigen-binding region that specifically binds to PD-1 comprises a heavy chain variable region (VH) and a light chain variable region (VL) ;
the CDR1H, CDR2H and CDR3H of the heavy chain variable domain VH of the first antigen-binding region are CDR1H, CDR2H and CDR3H of the heavy chain variable domain of SEQ ID NO: 1; or the CDR1H, CDR2H and CDR3H of the heavy chain variable domain VH of the first antigen-binding region are CDR1H, CDR2H and CDR3H of the sequence which is obtained by adding, deleting or replacing one or more amino acids in SEQ ID NO: 1, and has the same functions as SEQ ID NO: 1;
the CDR1L, CDR2L and CDR3L of the light chain variable domain VL of the first antigen binding region are obtained by amino acid mutation based on the CDR1L, CDR2L and CDR3L of the light chain variable domain of SEQ ID NO: 5, the mutation refers to mutation of the amino acids at No. 91 and No. 95 of SEQ ID NO: 5; or CDR1L, CDR2L and CDR3L of the light chain variable domain VL of the first antigen binding region are CDR1L, CDR2L and CDR3L of the light chain variable domain of SEQ ID NO: 5.

26. The bispecific antibody or antigen-binding fragment according to claim 25, the mutation refers to mutation of the amino acids at No. 91 and No. 95 of SEQ ID NO: 5 and the amino acids at No. 91 and No. 95 are combination of tryptophan W and valine V, proline P and tryptophan W, leucine L and phenylalanine F.

27. The bispecific antibody or antigen-binding fragment according to any one of claims 25-26, the CDR1L, CDR2L and CDR3L of the light chain variable domain VL of the first antigen binding region are CDR1L, CDR2L and CDR3L of the variable domain of SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53 or SEQ ID NO:55;
preferably, the CDR1L, CDR2L and CDR3L of the light chain variable domain VL of the first antigen binding region are CDR1L, CDR2L and CDR3L of the variable domain of SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:55;
more preferably, the CDR1L, CDR2L and CDR3L of the light chain variable domain VL of the first antigen binding region are CDR1L, CDR2L and CDR3L of the variable domain of SEQ ID NO:11, SEQ ID NO:13, SEQ ID NO:15, SEQ ID NO:25, SEQ ID NO:31 or SEQ ID NO:51;
most preferably, the CDR1L, CDR2L and CDR3L of the light chain variable domain VL of the first antigen binding region are CDR1L, CDR2L and CDR3L of the light chain variable domain of SEQ ID NO: 11.

28. The bispecific antibody or antigen-binding fragment according to any one of claims 25-27, the CDR1H, CDR2H and CDR3H of the heavy chain variable domain VH of the first antigen binding region are CDR1H, CDR2H and CDR3H of the heavy chain variable domain of SEQ ID NO: 1.

29. The bispecific antibody or antigen-binding fragment according to any one of claims 25-28, as defined according to the IMGT antibody numbering scheme for CDR regions, the light chain CDR of the first antigen-binding region is selected from the following combination:
(a) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 12; or
(b) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 14; or
(c) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 16; or
(d) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 18; or
(e) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 20; or
(f) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 22; or
(g) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 24; or
(h) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 26; or
(i) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 28; or
(j) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 30; or
(k) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 32; or
(l) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 34; or
(m) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 36; or
(n) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 38; or
(o) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 40; or
(p) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 42; or
(q) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 44; or
(r) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 46; or
(s) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 48; or
(t) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 50; or
(u) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 52; or
(v) the light chain complementarity determining region 1 (CDR1L) described in the amino acid sequence of SEQ ID: 6, the light chain complementarity determining region 2 (CDR2L) described in the amino acid sequence of SEQ ID: 7, the light chain complementarity determining region 3 (CDR3L) described in the amino acid sequence of SEQ ID: 54; or
(w) the light chain complementarity determining region 1 (CDR1L) described in the amino acid sequence of SEQ ID: 6, the light chain complementarity determining region 2 (CDR2L) described in the amino acid sequence of SEQ ID: 7, and the light chain complementarity determining region 3 (CDR3L) described in the amino acid sequence of SEQ ID: 56;
the heavy chain of the first antigen-binding region includes the heavy chain complementarity determining region 1 (CDR1H) described in the amino acid sequence of SEQ ID: 2, and the heavy chain complementarity determining region 2 (CDR2H) described in the amino acid sequence of SEQ ID: 3, the heavy chain complementarity determining region 3 (CDR3H) described in the amino acid sequence of SEQ ID: 4;
preferably, the CDR region of the light chain of the first antigen-binding region is selected from the CDR in (a)-(k), (m)-(u) or (w) above; further preferably selected from the CDR in (a)-(c) , (h), (k) or (u); most preferably selected from the CDR in (a).

30. The bispecific antibody or antigen-binding fragment according to any one of claims 25-29, the heavy chain variable region of the first antigen binding region comprises the amino acid sequence of SEQ ID NO:1, SEQ ID NO:67, SEQ ID NO:70, SEQ ID NO:73, SEQ ID NO:75, SEQ ID NO:78, SEQ ID NO:80 or SEQ ID NO:81; or comprises the heavy chain variable region of the first antigen binding region which is obtained by adding, deleting, or replacing one or more amino acids in SEQ ID NO: 1 and has the same function as SEQ ID NO:1;
preferably comprises the amino acid sequence of SEQ ID NO: 1, SEQ ID NO:67, SEQ ID NO:70, SEQ ID NO:73, SEQ ID NO:75, SEQ ID NO:78, SEQ ID NO:80 or SEQ ID NO:81; and more preferably comprises the amino acid sequence of SEQ ID NO: 67 or SEQ ID NO: 81.

31. The bispecific antibody or antigen-binding fragment according to claim 30, the light chain variable region of the first antigen binding region comprises the amino acid sequence selected from those shown in SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:66, SEQ ID NO:71, SEQ ID NO:72, SEQ ID NO:74, SEQ ID NO:76, SEQ ID NO:77 or SEQ ID NO:79;
preferably, the light chain variable domain VL of the first antigen binding region comprises the amino acid sequence selected from those shown in SEQ ID NO: 11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:55, SEQ ID NO:66, SEQ ID NO:71, SEQ ID NO:72, SEQ ID NO:74, SEQ ID NO:76, SEQ ID NO:77 or SEQ ID NO:79;
more preferably, the light chain variable domain VL of the first antigen binding region comprises the amino acid sequence selected from those shown in SEQ ID NO:11, SEQ ID NO:13, SEQ ID NO:15, SEQ ID NO:25, SEQ ID NO:31, SEQ ID NO:51, SEQ ID NO:66, SEQ ID NO:72, SEQ ID NO:79 or SEQ ID NO:76;
most preferably, the light chain variable domain VL of the first antigen binding region is the amino acid sequence shown in SEQ ID NO:11, SEQ ID NO:66, SEQ ID NO:72, SEQ ID NO:79 or SEQ ID NO:76.

32. The bispecific antibody or antigen-binding fragment according to any one of claims 25-31, the bispecific antibody comprises the heavy chain constant region of an IgG; preferably, the bispecific antibody comprises a heavy chain constant region of IgG1, IgG4 or IgG2; more preferably, the bispecific antibody comprises a heavy chain constant region of IgG4; most preferably, the bispecific antibody comprises a heavy chain constant region as shown in SEQ ID NO:91.

33. The bispecific antibody or antigen-binding fragment according to any one of claims 25-32, the second antigen-binding region that specifically binds to PD-1 comprises a heavy chain variable region and a light chain variable region, and the CDR1H, CDR2H and CDR3H of the heavy chain variable region VH are CDR1H, CDR2H and CDR3H of heavy chain variable domains in SEQ ID NO: 83; or the CDR1H, CDR2H and CDR3H of the heavy chain variable region VH are CDR1H, CDR2H and CDR3H of the sequence which is obtained by adding, deleting or replacing one or more amino acids in SEQ ID NO: 83 and has the same function as SEQ ID NO: 83; the CDR1L, CDR2L and CDR3L of the light chain variable region VL are CDR1L, CDR2L and CDR3L of the heavy chain variable domains in SEQ ID NO: 87, or the CDR1L, CDR2L and CDR3L of the heavy chain variable region VL are CDR1L, CDR2L and CDR3L of the sequence which is obtained by adding, deleting or replacing one or more amino acids in SEQ ID NO: 87 and has the same function as SEQ ID NO: 87.

34. The bispecific antibody or antigen-binding fragment according to claim 33, the second antigen-binding region that specifically binds PD-1 comprises a heavy chain variable region and a light chain variable region,
the heavy chain variable region comprises CDR1H as shown in SEQ ID NO:84, CDR2H as shown in SEQ ID NO:85 and CDR3H as shown in SEQ ID NO:86; and
the light chain variable region includes CDR1L as shown in SEQ ID NO:88, CDR2L as shown in SEQ ID NO:89 and CDR3L as shown in SEQ ID NO:90.

35. The bispecific antibody or antigen-binding fragment according to any one of claims 25-34, the heavy chain variable region comprises the sequence as shown in SEQ ID NO:83 and the light chain variable region comprises the sequence as shown in SEQ ID NO:87.

36. The bispecific antibody or antigen-binding fragment according to any one of claims 25-35, the first antigen binding region or the second antigen binding region is in scFv form; preferably, the first antigen binding region is in scFv form.

37. The bispecific antibody or antigen-binding fragment according to any one of claims 25-36, the first antigen-binding region and the second antigen-binding region are connected through a linker,
preferably, the linker includes (G4S)n, n is an integer greater than 1;
more preferably, the linker is composed of (G4S)n, n is an integer from 2 to 10;
most preferably, the linker is composed of (G4S)n, n is 2, 3 or 4.

38. The bispecific antibody or antigen-binding fragment according to any one of claims 25-37, the scFv comprises a heavy chain variable region and a light chain variable region, and the heavy chain variable region and the light chain variable region are connected by a linker,
preferably, the linker includes (G4S)n, n is an integer greater than 1;
more preferably, the linker is composed of (G4S)n, n is an integer from 2 to 10;
most preferably, the linker is composed of (G4S)n, n is 2, 3 or 4.

39. The bispecific antibody or antigen-binding fragment according to any one of claims 25-38, the bispecific antibody consists of 4 peptide chains, 2 identical first chains and 2 identical second chains,
the first chain sequentially comprises VH of the PD-1 binding region, CH of the PD-1 binding region, linker, the VH of the ALK-1 binding region, linker and the VL of the ALK-1 binding region in sequence from the N-terminal to the C-terminal, and
the second chain comprises the VL and CL of the PD-1 binding region in sequence from the N-terminal to the C-terminal.

40. The bispecific antibody or antigen-binding fragment according to claim39, the specific sequence of the bispecific antibody is as follows:
the VH sequence of the PD-1 binding region is shown in SEQ ID NO: 83;
the CH sequence of the PD-1 binding region is shown in SEQ ID NO: 91;
the VL sequence of the PD-1 binding region is shown in SEQ ID NO: 87;
the CL sequence of the PD-1 binding region is shown in SEQ ID NO: 92;
the linker sequence is shown in SEQ ID NO: 65;
the VL sequence of the ALK-1 binding region is as shown in SEQ ID NO: 66, SEQ ID NO:71, SEQ ID NO:72, SEQ ID NO:74, SEQ ID NO:76, SEQ ID NO:77 or SEQ ID NO:79, and
the VH sequence of the ALK-1 binding region is as shown in SEQ ID NO: 67, SEQ ID NO: 1, SEQ ID NO:70, SEQ ID NO:73, SEQ ID NO:75, SEQ ID NO:78, SEQ ID NO:80 or SEQ ID NO:81;
preferably, the sequence of the first chain is shown in SEQ ID NO: 93; the sequence of the second chain is shown in SEQ ID NO: 94.

41. A polynucleotide encoding monoclonal antibody or antigen-binding portion thereof that binds to ALK-1 according to any one of claims 1 to 8, or the bispecific antibody or fragment thereof according to any one of claims 9 to 40.

42. An expression vector expressing which is capable of expressing monoclonal antibody or antigen-binding portion thereof that binds to ALK-1 according to any one of claims 1 to 8, or the bispecific antibody according to any one of claims 9 to 40.

43. An engineered cell containing the vector according to claim 42.

44. A pharmaceutical composition comprising monoclonal antibody or antigen-binding portion thereof that binds to ALK-1 according to any one of claims 1 to 8, or the bispecific antibody according to any one of claims 9 to 40, or the polynucleotide according to claim 41, or the vector according to claim 42, and a pharmaceutical acceptable carrier.

45. Use of the monoclonal antibody or antigen-binding portion thereof that binds to ALK-1 according to any one of claims 1 to 8, the bispecific antibody according to any one of claims 9 to 40, a polynucleotide according to claim 41, an vector according to claim 42, or a cell according to claim 43, and the pharmaceutically acceptable carrier or a pharmaceutical composition according to claim 44 in the preparation of a drug for inhibiting tumor angiogenesis.

46. Use of the monoclonal antibody or antigen-binding portion thereof that binds to ALK-1 according to any one of claims 1 to 8, the bispecific antibody according to any one of claims 9 to 40, a polynucleotide according to claim 41, an vector according to claim 42, or a cell according to claim 43, and the pharmaceutically acceptable carrier or a pharmaceutical composition according to claim 44 in the preparation of a drug for the treatment of a tumor;
the tumor is a solid tumor or a hematological tumor;
preferably, the tumor is selected from the group consisting of esophageal cancer (eg, esophageal adenocarcinoma and esophageal squamous cell carcinoma), brain tumors, lung cancer (eg, small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, intestinal cancer liver metastasis, kidney cancer, urothelial cancer, non-Hodgkin lymphoma, central nervous system tumors (such as glioma, glioblastoma multiforme, glioma, or sarcoma), prostate cancer, or thyroid cancer.

47. A pharmaceutical composition comprising the bispecific antibody according to any one of claims 9 to 24, and any of anti-PD-1 antibody, anti-PD-L1 antibody, cytotoxic or non-cytotoxic small molecule drugs.

48. Use of the composition according to claim 47 in the preparation of a drug for the treatment of a tumor, the tumor is solid tumor; preferably, the solid tumor is hepatocellular carcinoma, lung cancer, colorectal cancer, gastric cancer, breast cancer, esophageal squamous cell carcinoma, urothelial cancer, nausea pleural mesothelioma.

49. A pharmaceutical composition comprising the bispecific antibody according to any one of claims 1 to 8, and any of anti-PD-1 antibody, anti-PD-L1 antibody, cytotoxic or non-cytotoxic small molecule drugs.

50. Use of the composition according to claim 49 in the preparation of a drug for the treatment of a tumor, the tumor is solid tumor; preferably, the solid tumor is hepatocellular carcinoma, lung cancer, colorectal cancer, gastric cancer, breast cancer, esophageal squamous cell carcinoma, urothelial cancer, nausea pleural mesothelioma.
